# EUROPEAN PATENT SPECIFICATION

(11) **EP 0 675 904 B1**
(45) Date of publication and mention of the grant of the patent: **05.03.2003**
(21) Application number: 93923749.1
(22) Date of filing: 30.09.1993
(51) Int. Cl.: C12N 15/13, A61K 39/395, G01N 33/569

(54) **HUMAN NEUTRALIZING MONOCLONAL ANTIBODIES TO HUMAN IMMUNODEFICIENCY VIRUS**
HUMANER NEUTRALISIERENDER MONOKLONALER ANTIKÖRPER GEGEN DEN DIE IMMUNSCHWÄCHE BEIM MENSCHEN HERVORRINGENDEN VIRUS
ANTICORPS MONOCLONAUX NEUTRALISATEURS HUMAINS CONTRE LE VIRUS DE L'IMMUNODEFICIENCE HUMAINE

(30) Priority: 30.09.1992 US 954148
(43) Date of publication of application: 11.10.1995
(73) Proprietor: THE SCRIPPS RESEARCH INSTITUTE, La Jolla, CA 92037 (US)
(72) Inventor: BURTON, Dennis R., La Jolla, CA 92041 (US); BARBAS, Carlos F., San Diego, CA 92122 (US); LERNER, Richard A., La Jolla, CA 92037 (US)
(74) Representative: Fisher, Adrian John
(86) International application number: US9309328
(87) International publication number: WO94007922

(56) References cited:
- VACCINES 92: MOD. APPROACHES NEW VACCINES INCL. PREV. AIDS [ANNU. MEET.], 9TH (1992), 191-195. EDITOR(S): BROWN, FRED. PUBLISHER: COLD SPRING HARBOR LAB. PRESS, COLD SPRING HARBOR, N. Y., 1992, XP002006404 BUCHACHER A. ET AL.: "Human Monoclonal Antibodies against gp41 and gp120 as Potential Agent for Passive Immunization"
- PROCEEDINGS OF THE NATIONAL ACADEMY OF SCIENCES OF USA, vol. 89, no. 8, 15 April 1992, WASHINGTON US, pages 3175-3179, XP002006405 ZEBEDEE S.L. ET AL.: "Human combinatorial antibody libraries to hepatitis B"
- PROC NATL ACAD SCI U S A 89 (19). 1992. 9339-9343, XP002006406 BARBAS C F III ET AL: "RECOMBINANT HUMAN FAB FRAGMENTS NEUTRALIZE HUMAN TYPE 1 IMMUNODEFICIENCY VIRUS IN-VITRO."
- Proceedings of the National Academy of Sciences, USA, Vol. 88, issued November 1991, BURTON et al., "A Large Array of Human Monoclonal Antibodies to Type 1 Human Immunodeficiency Virus from Combinatorial Libraries of Asymptomatic Seropositive Inidividuals", pages 10134-10137, see entire document and especially Figure 1.
- Journal of Virology, Vol. 65, No. 1, issued january 1991, HO et al., "Conformational Epitope on gp120 Important in CD4 Binding and Human Immunodeficiency Virus Type 1 Neutralization Identified by a Human Monoclonal Antibody", pages 489-493, see entire document.
- Proceedings of the National Academy of Sciences, USA, Vol. 88, issued April 1991, GORNY et al., "Production of Site-Selected Neutralizing Human Monoclonal Antibodies against the Third Variable Domain of the Human Immunodeficiency Virus Type 1 Envelope Glycoprotein", pages 3238-3242, see entire document.
- Journal of Immunology, Vol. 146, No. 12, issued 15 June 1991, POSNER et al., "An IgG Human Monoclonal Antibody that Reacts with HIV-1/gp120, Inhibits Virus Binding to Cells, and Neutralizes Infection", pages 4325-4332, see entire document.
- Research Virology, Vol. 142, issued 1991, TILLEY et al., "A Human Monoclonal Antibody Against CD4-Binding Site of HIV1 gp120 Exhibits Potent, Broadly Neutralizing Activity", pages 247-259, see entire document.
- AIDS Research and Human Retroviruses, Vol. 8, No. 6, issued June 1992, KARWOWSKA et al., "Production of Human Monoclonal Antibodies Specific for Conformational and Linear Non-V3 Epitopes of gp120", pages 1099-1106, see entire document.

### SEQUENCE LISTING

(1) GENERAL INFORMATION:
   (i) APPLICANT:
      (A) NAME: The Scripps Research Institute
      (B) STREET: 10666 North Torrey Pines Road
      (C) CITY: La Jolla
      (D) STATE: CA
      (E) COUNTRY: USA
      (F) POSTAL CODE (ZIP): 92037
      (G) TELEPHONE: 619-554-2937
      (H) TELEFAX: 619-554-6312
   (ii) TITLE OF INVENTION: HUMAN NEUTRALIZING MONOCLONAL ANTIBODIES TO HUMAN IMMUNODEFICIENCY VIRUS
   (iii) NUMBER OF SEQUENCES: 151
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EP0)
   (v) CURRENT APPLICATION DATA:
      (A) APPLICATION NUMBER: PCT/US93/
      (B) FILING DATE: 30-SEP-1993
   (vi) PRIOR APPLICATION DATA:
      (A) APPLICATION NUMBER: US 07/954,148
      (B) FILING DATE: 30-SEP-1992
(2) INFORMATION FOR SEQ ID NO:1:
   (i) SEQUENCE CHARACTERISTICS:
      (A) LENGTH: 173 base pairs
      (B) TYPE: nucleic acid
      (C) STRANDEDNESS: single
      (D) TOPOLOGY: linear
   (ii) MOLECULE TYPE: DNA (genonic)
   (iii) HYPOTHETICAL: NO
   (iv) ANTI-SENSE: NO
   (xi) SEQUENCE DESCRIPTION: SEQ ID NO:1:
   GGCCGCAAAT TCTATTTCAA GGAGACAGTC ATAATGAAAT ACCTATTGCC TACGGCAGCC 60
   GCTGGATTGT TATTACTCGC TGCCCAACCA GCCATGGCCC AGGTGAAACT GCTCGAGATT 120
   TCTAGACTAG TTACCCGTAC GACGTTCCGG ACTACGGTTC TTAATAGAAT TCG 173
(2) INFORMATION FOR SEQ ID NO:2:
   (i) SEQUENCE CHARACTERISTICS:
      (A) LENGTH: 173 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:2:
   TCGACGAATT CTATTAAGAA CCGTAGTCCG GAACGTCGTA CGGGTAACTA GTCTAGAAAT 60
   CTCGAGCAGT TTCACCTGGG CCATGGCTGG TTGGGCAGCG AGTAATAACA ATCCAGCGGC 120
   TGCCGTAGGC AATAGGTATT TCATTATGAC TGTCTCCTTG AAATAGAATT TGC 173
(2) INFORMATION FOR SEQ ID NO:3:
   (i) SEQUENCE CHARACTERISTICS:
      (A) LENGTH: 131 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:3:
   TGAATTCTAA ACTAGTCGCC AAGGAGACAG TCATAATGAA ATACCTATTG CCTACGGCAG 60
   CCGCTGGATT GTTATTACTC GCTGCCCAAC CAGCCATGGC CGAGCTCGTC AGTTCTAGAG 120
   TTAAGCGGCC G 131
(2) INFORMATION FOR SEQ ID NO:4:
   (i) SEQUENCE CHARACTERISTICS:
      (A) LENGTH: 139 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:4:
   TCGACGGCCG CTTAACTCTA GAACTGACGA GCTCGGCCAT GGCTGGTTGG GCAGCGAGTA 60
   ATAACAATCC AGCGGCTGCC GTAGGCAATA GGTATTTCAT TATGACTGTC TCCTTGGCGA 120
   CTAGTTTAGA ATTCAAGCT 139
(2) INFORMATION FOR SEQ ID NO:5:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:5:
   Tyr Pro Tyr Asp Val Pro Asp Tyr Ala Ser
   1 5 10
(2) INFORMATION FOR SEQ ID NO:6:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (v) FRAGMENT TYPE: N-terminal
   (xi) SEQUENCE DESCRIPTION: SEQ ID NO:6;
(2) INFORMATION FOR SEQ ID NO:7:
   (i) SEQUENCE CHARACTERISTICS:
      (A) LENGTH: 23 amino acids
      (B) TYPE: amino acid
      (D) TOPOLOGY: linear
   (ii) MOLECULE TYPE: peptide
   (v) FRAGMENT TYPE: N-terminal
   (xi) SEQUENCE DESCRIPTION: SEQ ID NO:7:
   Met Lys Tyr Leu Leu Pro Thr Ala Ala Ala Gly Leu Leu Leu Leu Ala
   1 5 10 15
   Ala Gln Pro Ala Met Ala Glu
   20
(2) INFORMATION FOR SEQ ID NO:8:
   (i) SEQUENCE CHARACTERISTICS:
      (A) LENGTH: 198 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:8:
   TGTTGACAAT TAATCATCGG CTCGTATAAT GTGTGGAATT GTGAGCGGAT AACAATTTCA 60
   CACAGGAGGA AGGATCCATG AAATACCTAT TGCCTACGGC AGCCGCTGGA TTGTTATTAC 120
   TCGCTGCCCA ACCAGCCATG GCCGAGCTCG GTCGGTCGGT CCTCGAGGGT CGGTCGGTCT 180
   CTAGAGTTAA GCGGCCGC 198
(2) INFORMATION FOR SEQ ID NO:9:
   (i) SEQUENCE CHARACTERISTICS:
      (A) LENGTH: 198 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:9:
   GCGGCCGCTT AACTCTAGAG ACCGACCGAC CCTCGAGGAC CGACCGACCG AGCTCGGCCA 60
   TGGCTGGTTG GGCAGCGAGT AATAACAATC CAGCGGCTGC CGTAGGCAAT AGGTATTTCA 120
   TGGATCCTTC CTCCTGTGTG AAATTGTTAT CCGCTCACAA TTCCACACAT TATACGAGCC 180
   GATGATTAAT TGTCAACA 198
(2) INFORMATION FOR SEQ ID NO:10:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (v) FRAGMENT TYPE: N-terminal
   (xi) SEQUENCE DESCRIPTION: SEQ ID NO:10:
   Met Lys Thr Leu Leu Pro Thr Ala Ala Ala Gly Leu Leu Leu Leu Ala
   1 5 10 15
   Ala Gln Pro Ala Met Ala Glu Leu
   20
(2) INFORMATION FOR SEQ ID NO:11:
   (i) SEQUENCE CHARACTERISTICS:
      (A) LENGTH: 220 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:11:
   TGTTGACAAT TAATCATCGG CTCGTATAAT GTGTGGAATT GTGAGCGGAT AACAATTTCA 60
   CACAGGAGGA AGGATCCATG AAATACCTAT TGCCTACGGC AGCCGCTGGA TTGTTATTAC 120
   TCGCTGCCCA ACCAGCCATG GCCCAGGTGA AACTGCTCGA GGGTCGGTCG GTCTCTAGAC 180
   GGTCGGTCGG TCACTAGTCA TCATCATCAT CATTAAGCTA 220
(2) INFORMATION FOR SEQ ID NO:12:
   (i) SEQUENCE CHARACTERISTICS:
      (A) LENGTH: 220 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:12:
   TAGCTTAATG ATGATGATGA TGACTAGTGA CCGACCGACC GTCTAGAGAC CGACCGACCC 60
   TCGAGCAGTT TCACCTGGGC CATGGCTGGT TGGGCAGCGA GTAATAACAA TCCAGCGGCT 120
   GCCGTAGGCA ATAGGTATTT CATGGATCCT TCCTCCTGTG TGAAATTGTT ATCCGCTCAC 180
   AATTCCACAC ATTATACGAG CCGATGATTA ATTGTCAACA 220
(2) INFORMATION FOR SEQ ID NO:13:
   (i) SEQUENCE CHARACTERISTICS:
      (A) LENGTH: 28 amino acids
      (B) TYPE: amino acid
      (D) TOPOLOGY: linear
   (ii) MOLECULE TYPE: peptide
   (v) FRAGMENT TYPE: N-terminal
   (xi) SEQUENCE DESCRIPTION: SEQ ID NO:13:
   Met Lys Thr Leu Leu Pro Thr Ala Ala Ala Gly Leu Leu Leu Leu Ala
   1 5 10 15
   Ala Gln Pro Ala Met Ala Gln Val Lys Leu Leu Glu
   20 25
(2) INFORMATION FOR SEQ ID NO:14:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: C-terminal
   (xi) SEQUENCE DESCRIPTION: SEQ ID NO:14:
   Thr Ser His His His His His
   1 5
(2) INFORMATION FOR SEQ ID NO:15:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:15:
   GGCCGCAAAT TCTATTTCAA GGAGACAGTC AT 32
(2) INFORMATION FOR SEQ ID NO:16:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:16:
   AATGAAATAC CTATTGCCTA CGGCAGCCGC TGGATT 36
(2) INFORMATION FOR SEQ ID NO:17:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:17:
   GTTATTACTC GCTGCCCAAC CAGCCATGGC CC 32
(2) INFORMATION FOR SEQ ID NO:18:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:18:
   CAGTTTCACC TGGGCCATGG CTGGTTGGG 29
(2) INFORMATION FOR SEQ ID NO:19:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:19:
   CAGCGAGTAA TAACAATCCA GCGGCTGCCG TAGGCAATAG 40
(2) INFORMATION FOR SEQ ID NO:20:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:20:
   GTATTTCATT ATGACTGTCT CCTTGAAATA GAATTTGC 38
(2) INFORMATION FOR SEQ ID NO:21:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:21:
   AGGTGAAACT GCTCGAGATT TCTAGACTAG TTACCCGTAC 40
(2) INFORMATION FOR SEQ ID NO:22:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:22:
   CGGAACGTCG TACGGGTAAC TAGTCTAGAA ATCTCGAG 38
(2) INFORMATION FOR SEQ ID NO:23:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:23:
   GACGTTCCGG ACTACGGTTC TTAATAGAAT TCG 33
(2) INFORMATION FOR SEQ ID NO:24:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:24:
   TCGACGAATT CTATTAAGAA CCGTAGTC 28
(2) INFORMATION FOR SEQ ID NO:25:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:25:
   TGAATTCTAA ACTAGTCGCC AAGGAGACAG TCAT 34
(2) INFORMATION FOR SEQ ID NO:26:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:26:
   AATGAAATAC CTATTGCCTA CGGCAGCCGC TGGATT 36
(2) INFORMATION FOR SEQ ID NO:27:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:27:
   GTTATTACTC GCTGCCCAAC CAGCCATGGC C 31
(2) INFORMATION FOR SEQ ID NO:28:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:28:
   GAGCTCGTCA GTTCTAGAGT TAAGCGGCCG 30
(2) INFORMATION FOR SEQ ID NO:29:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:29:
   GTATTTCATT ATGACTGTCT CCTTGGCGAC TAGTTTAGAA TTCAAGCT 48
(2) INFORMATION FOR SEQ ID NO:30:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:30:
   CAGCGAGTAA TAACAATCCA GCGGCTGCCG TAGGCAATAG 40
(2) INFORMATION FOR SEQ ID NO:31:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:31:
   TGACGAGCTC GGCCATGGCT GGTTGGG 27
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:32:
   TCGACGGCCG CTTAACTCTA GAAC 24
(2) INFORMATION FOR SEQ ID NO:33:
   (i) SEQUENCE CHARACTERISTICS:
      (A) LENGTH: 666 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:33:
   CCATTCGTTT GTGAATATCA AGGCCAAGGC CAATCGTCTG ACCTGCCTCA ACCTCCTGTC   60
   AATGCTGGCG GCGGCTCTGG TGGTGGTTCT GGTGGCGGCT CTGAGGGTGG TGGCTCTGAG  120
   GGTGGCGGTT CTGAGGGTGG CGGCTCTGAG GGAGGCGGTT CCGGTGGTGG CTCTGGTTCC  180
   GGTGATTTTG ATTATGAAAA GATGGCAAAC GCTAATAAGG GGGCTATGAC CGAAAATGCC  240
   GATGAAAACG CGCTACAGTC TGACGCTAAA GGCAAACTTG ATTCTGTCGC TACTGATTAC  300
   GGTGCTGCTA TCGATGGTTT CATTGGTGAC GTTTCCGGCC TTGCTAATGG TAATGGTGCT  360
   ACTGGTGATT TTGCTGGCTC TAATTCCCAA ATGGCTCAAG TCGGTGACGG TGATAATTCA  420
   CCTTTAATGA ATAATTTCCG TCAATATTTA CCTTCCCTCC CTCAATCGGT TGAATGTCGC  480
   CCTTTTGTCT TTAGCGCTGG TAAACCATAT GAATTTTCTA TTGATTGTGA CAAAATAAAC  540
   TTATTCGGTG TCTTTGCGTT TCTTTTATAT GTTGCCACCT TTATGTATGT ATTTTCTACG  600
   TTTGCTAACA TACTGCGTAA TAAGGAGTCT TAATCATGCC AGTTCTTTTG GGTATTCCGT  660
   TATTAT                                                             666
(2) INFORMATION FOR SEQ ID NO:34:
   (i) SEQUENCE CHARACTERISTICS:
      (A) LENGTH: 211 amino acids
      (B) TYPE: amino acid
      (D) TOPOLOGY: unknown
   (ii) MOLECULE TYPE: protein
   (iii) HYPOTHETICAL: NO
   (v) FRAGMENT TYPE: internal
   (xi) SEQUENCE DESCRIPTION: SEQ ID NO:34:
   Pro Phe Val Cys Glu Tyr Gln Gly Gln Gly Gln Ser Ser Asp Leu Pro
   1               5                   10                  15
   Gln Pro Pro Val Asn Ala Gly Gly Gly Ser Gly Gly Gly Ser Gly Gly
               20                  25                  30
   Gly Ser Glu Gly Gly Gly Ser Glu Gly Gly Gly Ser Glu Gly Gly Gly
           35                  40                  45
   Ser Glu Gly Gly Gly Ser Gly Gly Gly Ser Gly Ser Gly Asp Phe Asp
       50                  55                  60
   Tyr Glu Lys Met Ala Asn Ala Asn Lys Gly Ala Met Thr Glu Asn Ala
   65                  70                  75                  80
   Asp Glu Asn Ala Leu Gln Ser Asp Ala Lys Gly Lys Leu Asp Ser Val
                   85                  90                  95
   Ala Thr Asp Tyr Gly Ala Ala Ile Asp Gly Phe Ile Gly Asp Val Ser
               100                 105                 110
   Gly Leu Ala Asn Gly Asn Gly Ala Thr Gly Asp Phe Ala Gly Ser Asn
           115                 120                 125
   Ser Gln Met Ala Gln Val Gly Asp Gly Asp Asn Ser Pro Leu Met Asn
       130                 135                 140
   Asn Phe Arg Gln Tyr Leu Pro Ser Leu Pro Gln Ser Val Glu Cys Arg
   145                 150                 155                 160
   Pro Phe Val Phe Ser Ala Gly Lys Pro Tyr Glu Phe Ser Ile Asp Cys
                   165                 170                 175
   Asp Lys Ile Asn Leu Phe Arg Gly Val Phe Ala Phe Leu Leu Tyr Val
               180                 185                 190
   Ala Thr Phe Met Tyr Val Phe Ser Thr Phe Ala Asn Ile Leu Arg Asn
           195                 200                 205
   Lys Glu Ser
       210
(2) INFORMATION FOR SEQ ID NO:35:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:35:
   GAGACGACTA GTGGTGGCGG TGGCTCTCCA TTCGTTTGTG AATATCAA 48
(2) INFORMATION FOR SEQ ID NO:36:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:36:
   TTACTAGCTA GCATAATAAC GGAATACCCA AAAGAACTGG 40
(2) INFORMATION FOR SEQ ID NO:37:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:37:
   TATGCTAGCT AGTAACACGA CAGGTTTCCC GACTGG 36
(2) INFORMATION FOR SEQ ID NO:38:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:38:
   ACCGAGCTCG AATTCGTAAT CATGGTC 27
(2) INFORMATION FOR SEQ ID NO:39:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:39:
   AGCTGTTGAA TTCGTGAAAT TGTTATCCGC T 31
(2) INFORMATION FOR SEQ ID NO:40:
   (i) SEQUENCE CHARACTERISTICS:
      (A) LENGTH: 708 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:40:
   GAGACGACTA GTGGTGGCGG TGGCTCTCCA TTCGTTTGTG AATATCAAGG CCAAGGCCAA 60
   TCGTCTGACC TGCCTCAACC TCCTGTCAAT GCTGGCGGCG GCTCTGGTGG TGGTTCTGGT 120
   GGCGGCTCTG AGGGTGGTGG CTCTGAGGGT GGCGGTTCTG AGGGTGGCGG CTCTGAGGGA 180
   GGCGGTTCCG GTGGTGGCTC TGGTTCCGGT GATTTTGATT ATGAAAAGAT GGCAAACGCT 240
   AATAAGGGGG CTATGACCGA AAATGCCGAT GAAAACGCGC TACAGTCTGA CGCTAAAGGC 300
   AAACTTGATT CTGTCGCTAC TGATTACGGT GCTGCTATCG ATGGTTTCAT TGGTGACGTT 360
   TCCGGCCTTG CTAATGGTAA TGGTGCTACT GGTGATTTTG CTGGCTCTAA TTCCCAAATG 420
   GCTCAAGTCG GTGACGGTGA TAATTCACCT TTAATGAATA ATTTCCGTCA ATATTTACCT 480
   TCCCTCCCTC AATCGGTTGA ATGTCGCCCT TTTGTCTTTA GCGCTGGTAA ACCATATGAA 540
   TTTTCTATTG ATTGTGACAA AATAAACTTA TTCCGTGGTG TCTTTGCGTT TCTTTTATAT 600
   GTTGCCACCT TTATGTATGT ATTTTCTACG TTTGCTAACA TACTGCGTAA TAAGGAGTCT 660
   TAATCATGCC AGTTCTTTTG GGTATTCCGT TATTATGCTA GCTAGTAA 708
(2) INFORMATION FOR SEQ ID NO:41:
   (i) SEQUENCE CHARACTERISTICS:
      (A) LENGTH: 201 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:41:
   TATGCTAGCT AGTAACACGA CAGGTTTCCC GACTGGAAAG CGGGCAGTGA GCGCAACGCA 60
   ATTAATGTGA GTTAGCTCAC TCATTAGGCA CCCCAGGCTT TACACTTTAT GCTTCCGGCT 120
   CGTATGTTGT GTGGAATTGT GAGCGGATAA CAATTTCACA CAGGAAACAG CTATGACCAT 180
   GATTACGAAT TCGAGCTCGG T 201
(2) INFORMATION FOR SEQ ID NO:42:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:42:
   CAGGTGCAGC TCGAGCAGTC TGGG 24
(2) INFORMATION FOR SEQ ID NO:43:
   (i) SEQUENCE CHARACTERISTICS:
      (A) LENGTH: 24 base pairs
      (B) TYPE: nucleic acid
      (C) STBANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:43:
   GAGGTGCAGC TCGAGGAGTC TGGG 24
(2) INFORMATION FOR SEQ ID NO:44:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:44:
   GCATGTACTA GTTTTGTCAC AAGATTTGGG 30
(2) INFORMATION FOR SEQ ID NO:45:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:45:
   GACATCGAGC TCACCCAGTC TCCA 24
(2) INFORMATION FOR SEQ ID NO:46:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:46:
   GAAATTGAGC TCACGCAGTC TCCA 24
(2) INFORMATION FOR SEQ ID NO:47:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:47:
   GCGCCGTCTA GAACTAACAC TCTCCCCTGT TGAAGCTCTT TGTGACGGGC AAG 53
(2) INFORMATION FOR SEQ ID NO:48:
   (i) SEQUENCE CHARACTERISTICS:
      (A) LENGTH: 12 amino acids
      (B) TYPE: amino acid
      (C) STRANDEDNESS: single
      (D) TOPOLOGY: circular
   (ii) MOLECULE TYPE: peptide
   (v) FRAGMENT TYPE: internal
   (xi) SEQUENCE DESCRIPTION: SEQ ID NO:48:
   Ser Ile Ser Gly Pro Gly Arg Ala Phe Tyr Thr Gly
   1               5                   10
(2) INFORMATION FOR SEQ ID NO:49:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:49:
   GTCGTTGACC AGGCAGCCCA G                                    21
(2) INFORMATION FOR SEQ ID NO:50:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:50:
   ATAGAAGTTG TTCAGCAGGC A                                    21
(2) INFORMATION FOR SEQ ID NO:51:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:51:
   ATTAACCCTC ACTAAAG 17
(2) INFORMATION FOR SEQ ID NO:52:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:52:
   GAATTCTAAA CTAGCTAGTT CG 22
(2) INFORMATION FOR SEQ ID NO:53:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:53:
   Leu Glu Glu Ser Gly Thr Glu Phe Lys Pro Pro Gly Ser Ser Val Lys
   1 5 10 15
   Val Ser Cys Lys Ala Ser Gly Gly Thr Phe Gly Asp Tyr Ala Ser Asn
   20 25 30
   Tyr Ala Ile Ser Trp Val Arg Gln Ala Pro Gly Gln Gly Leu Glu Tyr
   35 40 45
   Ile Gly Gly Ile Thr Pro Thr Ser Gly Ser Ala Asp Tyr Ala Gln Lys
   50 55 60
   Phe Gln Gly Arg Val Thr Ile Ser Ala Asp Arg Phe Thr Pro Ile Leu
   65 70 75 80
   Tyr Met Glu Leu Arg Ser Leu Arg Ile Glu Asp Thr Ala Ile Tyr Tyr
   85 90 95
   Cys Ala Arg Glu Arg Arg Glu Arg Gly Trp Asn Pro Arg Ala Leu Arg
   100 105 110
   Gly Ala Leu Asp Phe Trp Gly Gln Gly Thr Arg Val Phe Val Ser Pro
   115 120 125
(2) INFORMATION FOR SEQ ID NO:54:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:54:
   Leu Glu Glu Ser Gly Ala Ala Val Gln Lys Pro Gly Ser Ser Val Arg
   1               5                   10                  15
   Val Ser Cys Gln Ala Ser Gly Gly Thr Phe Asp Asn Phe Ala Ser Asn
               20                  25                  30
   Tyr Ala Val Ser Trp Val Arg Gln Ala Pro Gly Gln Gly Leu Glu Trp
           35                  40                  45
   Met Gly Gly Ile Thr Pro Thr Ser Gly Thr Ala Thr Tyr Ser Gln Lys
       50                  55                  60
   Phe Gln Gly Arg Val Thr Ile Ser Ala Ala Pro Leu Thr Pro Ile Ile
   65                  70                  75                  80
   Tyr Met Glu Leu Arg Ser Leu Arg Asp Asp Asp Thr Ala Val Tyr Tyr
                   85                  90                  95
   Cys Ala Arg Glu Arg Arg Glu Arg Gly Trp Asn Pro Arg Ala Leu Val
               100                 105                 110
   Gly Ala Leu Asp Val Trp Gly Gln Gly Thr Thr Val
           115                 120
(2) INFORMATION FOR SEQ ID NO:55:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:55:
   Leu Glu Glu Ser Gly Thr Glu Phe Lys Pro Pro Gly Ser Ser Val Lys
   1               5                   10                  15
   Val Ser Cys Lys Ala Ser Gly Gly Thr Phe Gly Asp Tyr Ala Ser Asn
               20                  25                  30
   Tyr Ala Ile Ser Trp Val Arg Gln Ala Pro Gly Gln Gly Leu Glu Tyr
           35                  40                  45
   Ile Gly Gly Ile Thr Pro Thr Ser Gly Ser Ala Asp Tyr Ala Gln Lys
       50                  55                  60
   Phe Gln Gly Arg Val Thr Ile Ser Ala Asp Arg Phe Thr Pro Ile Leu
   65                  70                  75                  80
   Tyr Met Glu Leu Arg Ser Leu Arg Ile Glu Asp Thr Ala Ile Tyr Tyr
   85 90 95
   Cys Ala Arg Glu Arg Arg Glu Arg Gly Trp Asn Pro Arg Ala Leu Arg
   100 105 110
   Gly Ala Leu Asp Phe Trp Gly Gln Gly Thr Arg Val Phe Val Ser Pro
   115 120 125
(2) INFORMATION FOR SEQ ID NO:56:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:56:
   Leu Glu Glu Ser Gly Ala Glu Val Lys Lys Pro Gly Ser Ser Val Lys
   1 5 10 15
   Val Ser Cys Lys Ala Ser Gly Gly Ile Phe Ser Asp Phe Ala Ser Asn
   20 25 30
   Tyr Ala Ile Ser Trp Val Arg Gln Ala Pro Gly Gln Gly Leu Glu Tyr
   35 40 45
   Met Gly Gly Ile Thr Pro Thr Ser Gly Ser Ala Asp Tyr Ala Gln Lys
   50 55 60
   Phe Gln Gly Arg Val Thr Ile Ser Ala Asp Ala Ala Thr Pro Arg Val
   65 70 75 80
   Tyr Met Glu Leu Arg Ile Leu Arg Ser Glu Asp Thr Ala Val Tyr Phe
   85 90 95
   Cys Ala Arg Glu Arg Arg Glu Arg Gly Trp Asn Pro Arg Ala Leu Arg
   100 105 110
   Gly Ala Leu Glu Val Trp Gly Gln Gly Thr Thr Val Ile Val Ser Pro
   115 120 125
(2) INFORMATION FOR SEQ ID NO:57:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:57:
   Leu Glu Glu Ser Gly Ala Ala Val Gln Lys Pro Gly Ser Ser Val Arg
   1               5                   10                  15
   Val Ser Cys Gln Ala Ser Gly Gly Thr Phe Asp Asn Phe Ala Ser Asn
               20                  25                  30
   Tyr Ala Val Ser Trp Val Arg Gln Ala Pro Gly Gln Gly Leu Glu Trp
           35                  40                  45
   Met Gly Gly Ile Thr Pro Thr Ser Gly Thr Ala Thr Tyr Ser Gln Lys
       50                  55                  60
   Phe Gln Gly Arg Val Thr Ile Ser Ala Ala Pro Leu Thr Pro Ile Ile
   65                  70                  75                  80
   Tyr Met Glu Leu Arg Ser Leu Arg Asp Asp Asp Thr Ala Val Tyr Tyr
                   85                  90                  95
   Cys Ala Arg Glu Arg Arg Glu Arg Gly Trp Asn Pro Arg Ala Leu Val
               100                 105                 110
   Gly Ala Leu Asp Val Trp Gly Gln Gly Thr Thr Val Ile Val Ser Ser
           115                 120                 125
(2) INFORMATION FOR SEQ ID NO:58:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:58:
   Leu Glu Gln Ser Gly Ala Glu Val Lys Lys Pro Gly Ser Ser Val Lys
   1               5                   10                  15
   Val Ser Cys Lys Thr Ser Gly Gly Thr Phe Ser Asp Tyr Ala Ser Asn
               20                  25                  30
   His Ala Ile Ser Trp Val Arg Gln Ala Pro Gly Gln Gly Leu Glu Tyr
           35                  40                  45
   Met Gly Gly Ile Thr Pro Thr Ser Gly Thr Ala Asp Tyr Ala Gln Lys
       50                  55                  60
   Phe Gln Ala Arg Val Thr Ile Ser Ala His Glu Phe Thr Pro Ile Val
   65                  70                  75                  80
   Tyr Met Glu Leu Arg Ser Leu Arg Ser Asp Gln His Ala Thr Tyr Tyr
                   85                  90                  95
   Cys Ala Thr Glu Arg Arg Glu Arg Gly Trp Asn Pro Arg Ala Leu Arg
               100                 105                 110
   Gly Ala Leu Asp Ile Trp Gly Gln Gly Thr Thr Val Ile Val Ser Ser
           115                 120                 125
(2) INFORMATION FOR SEQ ID NO:59:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:59:
(2) INFORMATION FOR SEQ ID NO:60:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:60:
   Leu Glu Gln Ser Gly Gly Gly Leu Val Lys Pro Gly Gly Ser Leu Arg
   1 5 10 15
   Leu Ser Cys Glu Gly Ser Gly Phe Thr Phe Thr Asn Ala Trp Met Thr
   20 25 30
   Trp Val Arg Gln Ser Pro Gly Lys Gly Leu Glu Trp Val Ala Ser Ile
   35 40 45
   Lys Ser Lys Phe Asp Gly Gly Ser Pro His Tyr Ala Ala Pro Val Glu
   50 55 60
   Gly Arg Phe Thr Ile Ser Arg Asn Asp Leu Glu Asp Lys Leu Phe Leu
   65 70 75 80
   Glu Met Ser Gly Leu Lys Ala Glu Asp Thr Gly Val Tyr Tyr Cys Ala
   85 90 95
   Thr Lys Tyr Pro Arg Tyr Phe Asp Met Met Ala Gly Val Arg Asn His
   100 105 110
   Phe Tyr Met Asp Val Trp Gly Thr Gly Thr Thr Val Ile Val Ser Ser
   115 120 125
(2) INFORMATION FOR SEQ ID NO:61:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:61:
   Leu Glu Glu Ser Gly Gly Gly Leu Val Lys Pro Gly Gly Ser Leu Arg
   1 5 10 15
   Leu Ser Cys Glu Gly Ser Gly Phe Thr Phe Thr Asn Ala Trp Met Thr
   20 25 30
   Trp Val Arg Gln Ser Pro Gly Lys Gly Leu Glu Trp Val Ala Ser Ile
   35 40 45
   Lys Ser Lys Phe Asp Gly Gly Ser Pro His Tyr Ala Ala Pro Val Glu
   50 55 60
   Gly Arg Phe Thr Ile Ser Arg Asn Asp Leu Glu Asp Lys Leu Phe Leu
   65 70 75 80
   Glu Met Ser Gly Leu Lys Ala Glu Asp Thr Gly Val Tyr Tyr Cys Ala
   85 90 95
   Thr Lys Tyr Pro Arg Tyr Ser Asp Met Met Ala Gly Val Arg Asn His
   100 105 110
   Leu Tyr Met Asp Val Trp Gly Lys Gly Thr Thr Val Ile Val Ser Ser
   115 120 125
(2) INFORMATION FOR SEQ ID NO:62:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:62:
   Leu Glu Glu Ser Gly Gly Arg Leu Val Lys Pro Gly Gly Ser Leu Arg
   1 5 10 15
   Leu Ser Cys Glu Ala Ser Gly Phe Thr Phe Thr Asn Ser Trp Met Thr
   20 25 30
   Trp Val Arg Gln Ser Pro Gly Lys Gly Leu Glu Trp Val Ala Ser Ile
   35 40 45
   Lys Arg Lys Phe Asp Gly Gly Ser Pro His Tyr Ala Ala Pro Val Glu
   50 55 60
   Gly Arg Phe Ser Ile Ser Arg Asn Asp Leu Glu Asp Lys Met Phe Leu
   65                  70                  75                  80
   Glu Met Ser Gly Leu Lys Ala Glu Asp Thr Gly Val Tyr Tyr Cys Ala
                   85                  90                  95
   Thr Lys Tyr Pro Arg Tyr Ser Asp Met Met Thr Gly Val Arg Asn His
               100                 105                 110
   Phe Tyr Met Asp Val Trp Gly Lys Gly Thr Thr Val Ile Val Ser Ser
           115                 120                 125
(2) INFORMATION FOR SEQ ID NO:63:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:63:
   Leu Glu Glu Ser Gly Gly Gly Leu Val Lys Pro Gly Gly Ser Leu Arg
   1               5                   10                  15
   Leu Ser Cys Glu Ser Ser Gly Phe Thr Phe Thr Asn Ala Trp Met Thr
               20                  25                  30
   Trp Val Arg Gln Ser Pro Gly Lys Gly Leu Glu Trp Val Ala Ser Ile
           35                  40                  45
   Lys Ser Lys Phe Asp Gly Gly Ser Pro His Tyr Ala Ala Pro Val Glu
       50                  55                  60
   Gly Arg Phe Thr Ile Ser Arg Asn Asp Leu Glu Asp Lys Leu Phe Leu
   65                  70                  75                  80
   Glu Met Ser Gly Leu Lys Ala Glu Asp Thr Gly Val Tyr Tyr Cys Ala
                   85                  90                  95
   Thr Lys Tyr Pro Arg Tyr Ser Asp Met Met Ala Gly Val Arg Asn His
               100                 105                 110
   Phe Tyr Met Asp Val Trp Gly Lys Gly Thr Thr Val Ile Val Ser Ser
           115                 120                 125
(2) INFORMATION FOR SEQ ID NO:64:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:64:
   Leu Glu Glu Ser Gly Gly Arg Leu Val Lys Pro Gly Gly Ser Leu Arg
   1               5                   10                  15
   Leu Ser Cys Glu Gly Ser Gly Phe Thr Phe Thr Asn Ala Trp Met Thr
               20                  25                  30
   Trp Val Arg Gln Ser Pro Gly Lys Gly Leu Glu Trp Val Ala Ser Ile
           35                  40                  45
   Lys Ser Lys Phe Asp Gly Gly Ser Pro His Tyr Ala Ala Pro Val Glu
       50                  55                  60
   Gly Arg Phe Ser Ile Ser Arg Asn Asp Leu Glu Asp Lys Met Phe Leu
   65                  70                  75                  80
   Glu Met Ser Gly Leu Lys Ala Glu Asp Thr Gly Val Tyr Tyr Cys Ala
                   85                  90                  95
   Thr Lys Tyr Pro Arg Tyr Ser Asp Met Met Thr Gly Val Arg Asn His
               100                 105                 110
   Phe Tyr Met Asp Val Trp Gly Lys Gly Thr Thr Val Ile Val Ser Ser
           115                 120                 125
(2) INFORMATION FOR SEQ ID NO:65:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:65:
   Leu Glu Glu Ser Gly Gly Gly Leu Val Lys Pro Gly Gly Ser Leu Arg
   1               5                   10                  15
   Leu Ser Cys Ala Gly Ser Gly Phe Thr Phe Thr Asn Ala Trp Met Thr
               20                  25                  30
   Trp Val Arg Gln Ser Pro Gly Lys Gly Leu Glu Trp Val Ala Ser Ile
           35                  40                  45
   Lys Ser Lys Phe Asp Gly Gly Ser Ser His Tyr Pro Gly Pro Val Glu
       50                  55                  60
   Gly Arg Phe Thr Ile Ser Arg Asn Tyr Ile Glu Asp Lys Leu Phe Leu
   65                  70                  75                  80
   Glu Met Ser Gly Leu Lys Ala Glu Asp Thr Gly Val Tyr Tyr Cys Ala
                   85                  90                  95
   Thr Lys Tyr Pro Arg Tyr Tyr Asp Met Met Arg Gly Val Arg Asn His
               100                 105                 110
   Tyr Tyr Met Asp Val Trp Gly Lys Gly Thr Thr Val Ile Val Ser Ser
           115                 120                 125
(2) INFORMATION FOR SEQ ID NO:66:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:66:
   Leu Glu Gln Ser Gly Ala Glu Val Lys Lys Pro Gly Ala Ser Val Lys
   1               5                   10                  15
   Val Ser Cys Gln Ala Ser Gly Tyr Arg Phe Ser Asn Phe Val Ile His
               20                  25                  30
   Trp Val Arg Gln Ala Pro Gly Gln Arg Phe Glu Trp Met Gly Trp Ile
           35                  40                  45
   Asn Pro Tyr Asn Gly Asn Lys Glu Phe Ser Ala Lys Phe Gln Asp Arg
       50                  55                  60
   Val Thr Phe Thr Ala Asp Thr Ser Ala Asn Thr Ala Tyr Met Glu Leu
   65                  70                  75                  80
   Arg Ser Leu Arg Ser Ala Asp Thr Ala Val Tyr Tyr Cys Ala Arg Val
                   85                  90                  95
   Gly Pro Tyr Ser Trp Asp Asp Ser Pro Gln Asp Asn Tyr Tyr Met Asp
               100                 105                 110
   Val Trp Gly Lys Gly Thr Thr Val Ile Val Ser Ser
           115                 120
(2) INFORMATION FOR SEQ ID NO:67:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:67:
   Leu Glu Gln Ser Gly Ala Glu Val Lys Lys Pro Gly Ala Ser Val Lys
   1               5                   10                  15
   Val Ser Cys Gln Ala Ser Gly Tyr Arg Phe Ser Asn Phe Val Ile His
               20                  25                  30
   Trp Val Arg Gln Ala Pro Gly Gln Arg Phe Glu Trp Met Gly Trp Ile
           35                  40                  45
   Asn Pro Tyr Asn Gly Asn Lys Glu Phe Ser Ala Lys Phe Gln Asp Arg
       50                  55                  60
   Val Thr Phe Thr Ala Asp Thr Asp Ala Asn Thr Ala Tyr Met Glu Leu
   65                  70                  75                  80
   Arg Ser Leu Arg Ser Ala Asp Thr Ala Ile Tyr Tyr Cys Ala Arg Val
                   85                  90                  95
   Gly Pro Tyr Thr Trp Asp Asp Ser Pro Gln Asp Asn Tyr Tyr Met Asp
               100                 105                 110
   Val Trp Gly Lys Gly Thr Lys Val Ile Val Ser Ser
           115                 120
(2) INFORMATION FOR SEQ ID NO:68:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:68:
   Leu Glu Gln Ser Gly Ala Glu Val Lys Lys Pro Gly Ala Ser Val Lys
   1               5                   10                  15
   Val Ser Cys Gln Ala Ser Gly Tyr Arg Phe Ser Asn Phe Val Ile His
               20                  25                  30
   Trp Val Arg Gln Ala Pro Gly Gln Arg Phe Glu Trp Met Gly Trp Ile
           35                  40                  45
   Asn Pro Tyr Asn Gly Asn Lys Glu Phe Ser Ala Lys Phe Gln Asp Arg
       50                  55                  60
   Val Thr Phe Thr Ala Asp Thr Asp Ala Asn Thr Ala Tyr Met Glu Leu
   65                  70                  75                  80
   Arg Ser Leu Arg Ser Thr Asp Thr Ala Ile Tyr Tyr Cys Ala Arg Val
                   85                  90                  95
   Gly Pro Tyr Thr Trp Asp Asp Ser Pro Gln Asp Asn Tyr Tyr Met Asp
               100                 105                 110
   Val Trp Gly Lys Gly Thr Lys Val Ile Val Ser Ser
           115                 120
(2) INFORMATION FOR SEQ ID NO:69:
   (i) SEQUENCE CHARACTERISTICS:
      (A) LENGTH: 130 amino acids
      (B) TYPE: amino acid
      (D) TOPOLOGY: linear
   (ii) MOLECULE TYPE: protein
   (xi) SEQUENCE DESCRIPTION: SEQ ID NO:69:
(2) INFORMATION FOR SEQ ID NO:70:
   (i) SEQUENCE CHARACTERISTICS:
      (A) LENGTH: 130 amino acids
      (B) TYPE: amino acid
      (D) TOPOLOGY: linear
   (ii) MOLECULE TYPE: protein
   (xi) SEQUENCE DESCRIPTION: SEQ ID NO:70:
   Leu Glu Glu Ser Gly Gly Gly Leu Val Lys Ala Gly Gly Ser Leu Arg
   1 5 10 15
   Leu Ser Cys Val Gly Ser Gly Phe Thr Phe Ser Ser Ala Trp Met Ala
   20 25 30
   Trp Val Gly Gln Ala Pro Gly Arg Gly Leu Glu Trp Val Gly Leu Ile
   35 40 45
   Lys Ser Lys Ala Asp Gly Glu Thr Thr Asp Tyr Ala Thr Pro Val Lys
   50 55 60
   Gly Arg Phe Ser Ile Ser Arg Asn Asn Leu Glu Asp Thr Val Tyr Leu
   65 70 75 80
   Gln Met Asp Ser Leu Arg Ala Asp Asp Thr Ala Val Tyr Tyr Cys Ala
   85 90 95
   Thr Gln Lys Pro Arg Tyr Phe Asp Leu Leu Ser Gly Gln Tyr Arg Arg
   100 105 110
   Val Ala Gly Ala Phe Asp Val Trp Gly His Gly Thr Thr Val Thr Val
   115 120 125
   Ser Pro
   130
(2) INFORMATION FOR SEQ ID NO:71:
   (i) SEQUENCE CHARACTERISTICS:
      (A) LENGTH: 130 amino acids
      (B) TYPE: amino acid
      (D) TOPOLOGY: linear
   (ii) MOLECULE TYPE: protein
   (xi) SEQUENCE DESCRIPTION: SEQ ID NO:71:
   Leu Glu Glu Ser Gly Gly Gly Leu Ile Lys Pro Gly Gly Ser Leu Arg
   1 5 10 15
   Leu Ser Cys Val Gly Ser Gly Phe Thr Phe Ser Ser Ala Trp Met Thr
   20 25 30
   Trp Val Arg Gln Ala Pro Gly Lys Gly Leu Glu Trp Ile Gly Leu Ile
   35 40 45
   Lys Ser Lys Ala Asp Gly Glu Thr Thr Asp Tyr Ala Thr Pro Val Lys
   50 55 60
   Gly Arg Phe Thr Ile Ser Arg Asn Asn Leu Glu Asn Thr Val Tyr Leu
   65 70 75 80
   Gln Met Asp Ser Leu Arg Ala Asp Asp Thr Ala Val Tyr Tyr Cys Ala
   85 90 95
   Thr Gln Lys Pro Ser Tyr Tyr Asn Leu Leu Ser Gly Gln Tyr Arg Arg
   100 105 110
   Val Ala Gly Ala Phe Asp Val Trp Gly His Gly Thr Thr Val Thr Val
   115 120 125
   Ser Pro
(2) INFORMATION FOR SEQ ID NO:72:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:72:
(2) INFORMATION FOR SEQ ID NO:73:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:73:
(2) INFORMATION FOR SEQ ID NO:74:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:74:
   Leu Glu Gln Ser Gly Glu Ala Val Val Gln Pro Gly Arg Ser Leu Arg
   1 5 10 15
   Leu Ser Cys Ala Ala Ser Gly Phe Ile Phe Arg Asn Tyr Ala Met His
   20 25 30
   Trp Val Arg Gln Ala Pro Gly Lys Gly Leu Glu Trp Val Ala Leu Ile
   35 40 45
   Lys Tyr Asp Gly Arg Asn Lys Tyr Tyr Ala Asp Ser Val Lys Gly Arg
   50 55 60
   Phe Thr Ile Ser Arg Asp Asn Ser Lys Asn Thr Leu Tyr Leu Gln Met
   65 70 75 80
   Asn Ser Leu Arg Ala Glu Asp Thr Ala Val Tyr Tyr Cys Ala Arg Asp
   85 90 95
   Ile Gly Leu Lys Gly Glu His Tyr Asp Ile Leu Thr Ala Tyr Gly Pro
   100 105 110
   Asp Tyr Trp Gly Gln Gly Thr Leu Val Thr Val Ser Ser
   115 120 125
(2) INFORMATION FOR SEQ ID NO:75:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:75:
   Leu Glu Glu Ser Gly Glu Ala Val Val Gln Pro Gly Thr Ser Leu Arg
   1               5                   10                  15
   Leu Ser Cys Ala Ala Ser Gly Phe Thr Phe Arg Asn Tyr Ala Met His
               20                  25                  30
   Trp Val Arg Gln Ala Pro Gly Lys Gly Leu Glu Trp Val Ala Leu Ile
           35                  40                  45
   Lys Tyr Asp Gly Arg Asn Lys Tyr Tyr Ala Asp Ser Val Lys Gly Arg
       50                  55                  60
   Phe Ser Ile Ser Arg Asp Asn Ser Lys Asn Thr Leu Tyr Leu Glu Met
   65                  70                  75                  80
   Asn Ser Leu Arg Ala Glu Asp Thr Ala Val Tyr Tyr Cys Ala Arg Asp
                   85                  90                  95
   Ile Gly Leu Lys Gly Glu His Tyr Asp Ile Leu Thr Ala Tyr Gly Pro
               100                 105                 110
   Asp Tyr Trp Gly Gln Gly Ala Leu Val Thr Val Ser Ser
           115                 120                 125
(2) INFORMATION FOR SEQ ID NO:76:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:76:
   Leu Glu Gln Ser Gly Glu Ala Val Val Gln Pro Gly Arg Ser Leu Arg
   1               5                   10                  15
   Leu Ser Cys Ala Ala Ser Gly Phe Thr Phe Arg Asn Tyr Ala Met His
               20                  25                  30
   Trp Val Arg Gln Ala Pro Gly Lys Gly Leu Glu Trp Val Ala Leu Ile
   35 40 45
   Lys Tyr Asp Gly Arg Asn Lys Tyr Tyr Ala Asp Ser Val Lys Gly Arg
   50 55 60
   Phe Thr Ile Ser Arg Asp Asn Ser Lys Asn Thr Leu Tyr Leu Gln Met
   65 70 75 80
   Asn Ser Leu Arg Ala Glu Asp Thr Ala Val Tyr Tyr Cys Ala Arg Asp
   85 90 95
   Ile Gly Leu Lys Ala Glu His Tyr Asp Ile Leu Thr Ala Tyr Gly Pro
   100 105 110
   Asp Tyr Trp Gly Gln Gly Thr Leu Val Thr Val Ser Ser
   115 120 125
(2) INFORMATION FOR SEQ ID NO:77:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:77:
   Leu Glu Gln Ser Gly Glu Ala Val Val Gln Pro Gly Arg Ser Leu Arg
   1 5 10 15
   Leu Ser Cys Ala Ala Ser Gly Phe Ile Phe Arg Asn Tyr Ala Met His
   20 25 30
   Trp Val Arg Gln Ala Pro Gly Lys Gly Leu Glu Trp Val Ala Leu Ile
   35 40 45
   Lys Tyr Asp Gly Arg Asn Lys Tyr Tyr Ala Asp Ser Val Lys Gly Arg
   50 55 60
   Phe Thr Ile Ser Arg Asp Asn Ser Lys Asn Thr Leu Tyr Leu Gln Met
   65 70 75 80
   Asn Ser Leu Arg Ala Glu Asp Thr Ala Val Tyr Tyr Cys Ala Arg Asp
   85 90 95
   Ile Gly Leu Lys Gly Glu His Tyr Asp Ile Leu Thr Ala Tyr Gly Pro
   100 105 110
   Asp Tyr Trp Gly Gln Gly Thr Leu Val Thr Val Ser Ser
   115 120 125
(2) INFORMATION FOR SEQ ID NO:78:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:78:
   Leu Glu Gln Ser Gly Gly Gly Val Val Lys Pro Gly Gly Ser Leu Arg
   1 5 10 15
   Leu Ser Cys Glu Gly Ser Gly Phe Thr Phe Pro Asn Ala Trp Met Thr
   20 25 30
   Trp Val Arg Gln Ser Pro Gly Lys Gly Leu Glu Trp Val Ala Ser Ile
   35 40 45
   Lys Ser Lys Phe Asp Gly Gly Ser Pro His Tyr Ala Ala Pro Val Glu
   50 55 60
   Gly Arg Phe Thr Ile Ser Arg Asn Asp Leu Glu Asp Lys Val Phe Leu
   65 70 75 80
   Gln Met Asn Gly Leu Lys Ala Glu Asp Thr Gly Val Tyr Tyr Cys Ala
   85 90 95
   Thr Arg Tyr Pro Arg Tyr Ser Glu Met Met Gly Gly Val Arg Lys His
   100 105 110
   Phe Tyr Met Asp Val Trp Gly Lys Gly Thr Thr Val Ser Val Ser Ser
   115 120 125
(2) INFORMATION FOR SEQ ID NO:79:
   (i) SEQUENCE CHARACTERISTICS:
      (A) LENGTH: 128 amino acids
      (B) TYPE: amino acid
      (D) TOPOLOGY: linear
   (ii) MOLECULE TYPE: protein
   (xi) SEQUENCE DESCRIPTION: SEQ ID NO:79:
   Leu Glu Glu Ser Gly Gly Gly Val Val Lys Pro Gly Gly Ser Leu Arg
   1               5                   10                  15
   Leu Ser Cys Glu Gly Ser Gly Phe Thr Phe Pro Asn Ala Trp Met Thr
               20                  25                  30
   Trp Val Arg Gln Ser Pro Gly Lys Gly Leu Glu Trp Val Ala Ser Ile
           35                  40                  45
   Lys Ser Lys Phe Asp Gly Gly Ser Pro His Tyr Ala Ala Pro Val Glu
       50                  55                  60
   Gly Arg Phe Thr Ile Ser Arg Asn Asp Leu Glu Asp Lys Val Phe Leu
   65                  70                  75                  80
   Gln Met Asn Gly Leu Lys Ala Glu Asp Thr Gly Val Tyr Tyr Cys Ala
                   85                  90                  95
   Thr Arg Tyr Pro Arg Tyr Ser Glu Met Met Gly Gly Val Arg Lys His
               100                 105                 110
   Phe Tyr Met Asp Val Trp Gly Lys Gly Thr Thr Val Ser Val Ser Ser
           115                 120                 125
(2) INFORMATION FOR SEQ ID NO:80:
   (i) SEQUENCE CHARACTERISTICS:
      (A) LENGTH: 122 amino acids
      (B) TYPE: amino acid
      (D) TOPOLOGY: linear
   (ii) MOLECULE TYPE: protein
   (xi) SEQUENCE DESCRIPTION: SEQ ID NO:80:
(2) INFORMATION FOR SEQ ID NO:81:
   (i) SEQUENCE CHARACTERISTICS:
      (A) LENGTH: 126 amino acids
      (B) TYPE: amino acid
      (D) TOPOLOGY: linear
   (ii) MOLECULE TYPE: protein
   (xi) SEQUENCE DESCRIPTION: SEQ ID NO:81:
(2) INFORMATION FOR SEQ ID NO:82:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:82:
(2) INFORMATION FOR SEQ ID NO:83:
   (i) SEQUENCE CHARACTERISTICS:
      (A) LENGTH: 106 amino acids
      (B) TYPE: amino acid
      (D) TOPOLOGY: linear
   (ii) MOLECULE TYPE: protein
   (xi) SEQUENCE DESCRIPTION: SEQ ID NO:83:
   Glu Leu Thr Gln Ser Pro Ser Ser Leu Ser Ala Ser Ile Gly Asp Arg
   1               5                   10                  15
   Val Thr Ile Thr Cys Arg Ala Ser Gln Gly Ile Asn Asn Tyr Leu Ala
               20                  25                  30
   Trp Tyr Gln Gln Arg Pro Gly Lys Val Pro Arg Leu Leu Ile Tyr Ala
           35                  40                  45
   Ala Ser Thr Leu Gln Ser Gly Val Pro Thr Arg Phe Ser Gly Ser Gly
       50                  55                  60
   Ser Gly Thr Asp Phe Thr Leu Thr Ile Ser Ser Leu Gln Pro Glu Asp
   65                  70                  75                  80
   Val Ala Thr Tyr Tyr Cys Gln Lys Tyr Asn Ser Val Pro Arg Thr Phe
                   85                  90                  95
   Gly Gly Gly Thr Lys Val Glu Ile Lys Arg
               100                 105
(2) INFORMATION FOR SEQ ID NO:84:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:84:
   Glu Leu Thr Gln Ser Pro Ser Ser Leu Ser Ala Ser Val Gly Asp Arg
   1               5                   10                  15
   Val Thr Ile Thr Cys Arg Ala Ser Gln Gly Ile Ser Asn Tyr Leu Ala
               20                  25                  30
   Trp Tyr Gln Gln Lys Pro Gly Lys Val Pro Lys Leu Leu Ile Tyr Ala
           35                  40                  45
   Ala Ser Thr Leu Gln Ser Gly Val Pro Ser Arg Phe Ser Gly Ser Gly
       50                  55                  60
   Ser Gly Thr Asp Phe Thr Leu Thr Ile Ser Ser Leu Gln Pro Glu Asp
   65                  70                  75                  80
   Val Ala Thr Tyr Tyr Cys Gln Lys Tyr Asn Ser Ala Pro Arg Thr Phe
                   85                  90                  95
   Gly Gln Gly Thr Lys Val Glu Ile Lys Arg Thr
               100                 105
(2) INFORMATION FOR SEQ ID NO:85:
   (i) SEQUENCE CHARACTERISTICS:
      (A) LENGTH: 106 amino acids
      (B) TYPE: amino acid
      (D) TOPOLOGY: linear
   (ii) MOLECULE TYPE: protein
   (xi) SEQUENCE DESCRIPTION: SEQ ID NO:85:
   Glu Leu Thr Gln Ser Pro Ser Ser Leu Ser Ala Ser Ile Gly Asp Arg
   1               5                   10                  15
   Val Thr Ile Thr Cys Arg Ala Ser Gln Gly Ile Asn Asn Tyr Leu Ala
               20                  25                  30
   Trp Tyr Gln Gln Arg Pro Gly Lys Ala Pro Asn Leu Leu Ile Tyr Ala
           35                  40                  45
   Ala Ser Thr Leu Gln Ser Gly Val Pro Pro Arg Phe Ser Gly Ser Gly
       50                  55                  60
   Ser Gly Thr Asp Phe Thr Leu Thr Ile Ser Ser Leu Gln Pro Glu Asp
   65                  70                  75                  80
   Val Ala Thr Tyr Tyr Cys Gln Lys Tyr Asn Ser Val Pro His Thr Phe
                   85                  90                  95
   Gly Gly Gly Thr Lys Val Glu Ile Lys Arg
               100                 105
(2) INFORMATION FOR SEQ ID NO:86:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:86:
   Glu Leu Thr Gln Ser Pro Gly Thr Leu Ser Leu Ser Pro Gly Glu Arg
   1               5                   10                  15
   Ala Thr Leu Ser Cys Arg Ala Ser Gln Ser Val Ile Ser Asn Tyr Leu
               20                  25                  30
   Ala Trp Tyr Gln Gln Lys Pro Gly Gln Ala Pro Arg Leu Leu Ile Tyr
           35                  40                  45
   Gly Val Ser Asn Arg Ala Thr Gly Ile Pro Asp Arg Phe Ser Gly Ser
       50                  55                  60
   Gly Ser Gly Thr Asp Phe Thr Leu Thr Ile Ser Arg Leu Glu Pro Glu
   65 70 75 80
   Asp Phe Ala Val Tyr Ser Cys Gln Gln Tyr Gly Thr Ser Pro Trp Thr
   85 90 95
   Phe Gly Gln Gly Thr Lys Val Glu Ile Lys Arg Thr
   100 105
(2) INFORMATION FOR SEQ ID NO:87:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:87:
   Glu Leu Thr Gln Ser Pro Gly Thr Leu Ser Leu Ser Pro Gly Glu Arg
   1 5 10 15
   Ala Thr Leu Ser Cys Arg Ala Ser Gln Ser Val Ser Asn Asn Tyr Leu
   20 25 30
   Ala Trp Tyr Gln Gln Arg Pro Gly Gln Ala Pro Arg Leu Leu Ile Tyr
   35 40 45
   Gly Ala Ser Asn Arg Ala Thr Gly Ile Pro Asp Arg Phe Ser Gly Ser
   50 55 60
   Gly Ser Gly Thr Ala Phe Thr Leu Thr Ile Ser Ser Leu Gln Pro Glu
   65 70 75 80
   Asp Val Ala Ile Tyr Tyr Cys Gln Gln Tyr His Ser Ser Pro Tyr Thr
   85 90 95
   Phe Gly Gln Gly Thr Lys Leu Glu Ile Lys Arg
   100 105
(2) INFORMATION FOR SEQ ID NO:88:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:88:
   Glu Leu Thr Gln Ser Pro Gly Thr Leu Ser Leu Ser Pro Gly Glu Arg
   1               5                   10                  15
   Ala Thr Leu Ser Cys Arg Ala Ser His Arg Val Asn Asn Asn Phe Leu
               20                  25                  30
   Ala Trp Tyr Gln Gln Lys Pro Gln Ala Pro Arg Leu Leu Ile Ser Gly
           35                  40                  45
   Ala Ser Thr Arg Ala Thr Gly Ile Pro Asp Arg Phe Ser Gly Ser Gly
       50                  55                  60
   Ser Gly Thr Asp Phe Thr Leu Thr Ile Ser Arg Leu Glu Pro Asp Asp
   65                  70                  75                  80
   Phe Ala Val Tyr Tyr Cys Gln Gln Tyr Gly Asp Ser Pro Leu Tyr Ser
                   85                  90                  95
   Phe Gly Gln Gly Thr Lys Leu Glu Ile Lys Arg Thr
               100                 105
(2) INFORMATION FOR SEQ ID NO:89:
   (i) SEQUENCE CHARACTERISTICS:
      (A) LENGTH: 105 amino acids
      (B) TYPE: amino acid
      (D) TOPOLOGY: linear
   (ii) MOLECULE TYPE: protein
   (xi) SEQUENCE DESCRIPTION: SEQ ID NO:89:
   Glu Leu Thr Gln Ser Pro Ala Ser Val Ser Ala Ser Val Gly Asp Thr
   1               5                   10                  15
   Val Thr Ile Thr Cys Arg Ala Ser Gln Asp Ile His Asn Trp Leu Ala
               20                  25                  30
   Trp Tyr Gln Gln Gln Pro Gly Lys Ala Pro Lys Leu Leu Ile Tyr Ala
           35                  40                  45
   Ala Ser Ser Leu Gln Ser Gly Val Pro Ser Arg Phe Ser Gly Arg Gly
       50                  55                  60
   Ser Gly Thr Asp Phe Thr Leu Thr Ile Ser Ser Leu Gln Pro Glu Asp
   65                  70                  75                  80
   Phe Ala Thr Tyr Tyr Cys Gln Gln Gly Asn Ser Phe Pro Lys Phe Gly
   85 90 95
   Pro Gly Thr Val Val Asp Ile Lys Arg
   100 105
(2) INFORMATION FOR SEQ ID NO:90:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:90:
   Glu Leu Thr Gln Ser Pro Gly Thr Leu Ser Leu Ser Pro Gly Glu Arg
   1 5 10 15
   Ala Thr Leu Ser Cys Arg Ala Ser Gln Ser Leu Ser Asn Asn Tyr Leu
   20 25 30
   Ala Trp Tyr Gln Gln Lys Pro Gly Gln Ala Pro Arg Leu Leu Ile Tyr
   35 40 45
   Gly Ser Ser Thr Arg Gly Thr Gly Ile Pro Asp Arg Phe Ser Gly Gly
   50 55 60
   Gly Ser Gly Thr Asp Phe Thr Leu Thr Ile Ser Arg Leu Glu Pro Glu
   65 70 75 80
   Asp Phe Ala Val Tyr Tyr Cys Gln His Tyr Gly Asn Ser Val Tyr Thr
   85 90 95
   Phe Gly Gln Gly Thr Lys Leu Glu Ile Lys Arg
   100 105
(2) INFORMATION FOR SEQ ID NO:9
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO:91:
(2) INFORMATION FOR SEQ ID NO:92:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO:92:
   Gln Ser Pro Ser His Leu Ser Leu Ser Pro Gly Glu Arg Ala Ile Leu
   1               5                   10                  15
   Ser Cys Arg Ala Ser Gln Arg Val Ser Ala Pro Tyr Leu Ala Trp Tyr
               20                  25                  30
   Gln Gln Arg Pro Gly Gln Ala Pro Arg Leu Val Ile Tyr Gly Ala Ser
           35                  40                  45
   Thr Arg Ala Thr Asp Ile Pro Asp Arg Phe Ser Gly Ser Gly Ser Gly
       50                  55                  60
   Thr Asp Phe Thr Leu Thr Ile Ser Arg Leu Glu Pro Glu Asp Phe Ala
   65                  70                  75                  80
   Ile Tyr Tyr Cys Gln Val Tyr Gly Gln Ser Pro Val Leu Phe Gly Gln
                   85                  90                  95
   Gly Thr Lys Leu Glu Met Lys Arg
   100
(2) INFORMATION FOR SEQ ID NO:93:
   (i) SEQUENCE CHARACTERISTICS:
      (A) LENGTH: 105 amino acids
      (B) TYPE: amino acid
      (D) TOPOLOGY: linear
   (ii) MOLECULE TYPE: protein
   (xi) SEQUENCE DESCRIPTION: SEQ ID NO:93:
   Gln Ser Pro Gly Thr Leu Ser Leu Ser Pro Gly Asp Arg Ala Thr Leu
   1 5 10 15
   Ser Cys Arg Ala Ser Gln Ser Leu Ser Ser Ser Phe Leu Ala Trp Tyr
   20 25 30
   Gln Gln Lys Pro Gly Gln Ala Pro Arg Leu Leu Ile Tyr Ser Ala Ser
   35 40 45
   Met Arg Ala Thr Gly Ile Pro Asp Arg Phe Arg Gly Ser Val Ser Gly
   50 55 60
   Thr Asp Phe Thr Leu Thr Ile Thr Arg Leu Glu Pro Glu Asp Phe Ala
   65 70 75 80
   Val Tyr Tyr Cys Gln Arg Phe Gly Thr Ser Pro Leu Tyr Thr Phe Gly
   85 90 95
   Gln Gly Thr Lys Leu Glu Met Lys Arg
   100 105
(2) INFORMATION FOR SEQ ID NO:94:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO:94:
   Gln Ser Pro Gly Thr Leu Ser Leu Ser Pro Gly Glu Arg Ala Thr Leu
   1 5 10 15
   Ser Cys Arg Ala Ser Gln Ser Phe Ser Ser Asn Phe Leu Ala Trp Tyr
   20 25 30
   Gln Gln Lys Pro Gly Gln Ala Pro Arg Leu Leu Ile Tyr Val His Pro
   35 40 45
   Asn Arg Ala Thr Gly Val Pro Asp Arg Phe Ser Gly Ser Gly Ser Gly
   50 55 60
   Thr Asp Phe Thr Leu Thr Ile Arg Arg Leu Glu Pro Glu Asp Phe Ala
   65 70 75 80
   Val Tyr Tyr Cys Gln Gln Tyr Gly Ala Ser Leu Val Ser Phe Gly Pro
   85 90 95
   Gly Thr Lys Val His Ile Lys Arg
   100
(2) INFORMATION FOR SEQ ID NO:95:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:95:
(2) INFORMATION FOR SEQ ID NO:96:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:96:
   Glu Leu Thr Gln Ser Pro Gly Thr Leu Ser Leu Thr Pro Gly Glu Arg
   1               5                   10                  15
   Ala Thr Leu Ser Cys Arg Thr Ser His Ser Ile Arg Ser Arg Arg Leu
               20                  25                  30
   Ala Trp Tyr Gln Val Lys Gly Gly Gln Ala Pro Arg Leu Leu Ile Tyr
           35                  40                  45
   Gly Val Ser Asn Arg Ala Gly Gly Ile Pro Asp Arg Phe Ser Gly Ser
       50                  55                  60
   Gly Ser Gly Thr Asp Phe Thr Leu Thr Ile Ser Arg Leu Glu Pro Glu
   65                  70                  75                  80
   Asp Phe Ala Val Tyr Tyr Cys Gln Gln Tyr Gly Ser Ser Arg Tyr Thr
                   85                  90                  95
   Phe Gly Gln Gly Thr Lys Leu Glu Ile Lys Arg Thr
               100                 105
(2) INFORMATION FOR SEQ ID NO:97:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:97:
   Glu Leu Thr Gln Ala Pro Gly Thr Leu Ser Leu Ser Pro Gly Glu Arg
   1               5                   10                  15
   Ala Thr Phe Ser Cys Arg Ser Ser His Ser Ile Arg Ser Arg Arg Val
               20                  25                  30
   Arg Trp Tyr Gln His Lys Pro Gly Gln Ala Pro Arg Leu Val Ile His
   35 40 45
   Gly Val Ser Asn Arg Ala Ser Gly Ile Ser Asp Arg Phe Ser Gly Ser
   50 55 60
   Gly Ser Gly Thr Asp Phe Thr Leu Thr Ile Thr Arg Val Glu Pro Glu
   65 70 75 80
   Asp Phe Ala Leu Tyr Tyr Cys Gln Val Tyr Gly Ala Ser Ser Tyr Thr
   85 90 95
   Phe Gly Gln Gly Thr Lys Leu Glu Arg Lys Arg
   100 105
(2) INFORMATION FOR SEQ ID NO:98:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:98:
   Glu Leu Thr Gln Ala Pro Gly Thr Leu Ser Leu Ser Pro Gly Asp Arg
   1 5 10 15
   Ala Thr Phe Ser Cys Arg Ser Ser His Asn Ile Arg Ser Arg Arg Val
   20 25 30
   Ala Trp Tyr Gln His Lys Pro Gly Gln Ala Pro Arg Leu Val Ile His
   35 40 45
   Gly Val Ser Asn Arg Ala Ser Gly Ile Ser Asp Arg Phe Ser Gly Ser
   50 55 60
   Gly Ser Gly Thr Asp Phe Thr Leu Thr Ile Thr Arg Leu Glu Pro Glu
   65 70 75 80
   Asp Phe Ala Leu Tyr Tyr Cys Gln Val Tyr Gly Ala Ser Ser Tyr Thr
   85 90 95
   Phe Gly Gln Gly Thr Lys Leu Asp Phe Lys Arg Thr
   100 105
(2) INFORMATION FOR SEQ ID NO:99:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:99:
(2) INFORMATION FOR SEQ ID NO:100:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO:100:
   Gln Ser Pro Gly Thr Leu Ser Leu Ser Pro Gly Glu Arg Ala Thr Leu
   1               5                   10                  15
   Ser Cys Arg Ala Ser Gln Ser Leu Ser Asn Asn Tyr Leu Ala Trp Tyr
               20                  25                  30
   Gln Gln Lys Pro Gly Gln Ala Pro Arg Leu Leu Ile Tyr Gly Ser Ser
           35                  40                  45
   Thr Arg Ala Thr Gly Ile Pro Asp Arg Phe Ser Gly Gly Gly Ser Gly
       50                  55                  60
   Thr Asp Phe Thr Leu Thr Ile Ser Arg Leu Glu Pro Glu Asp Phe Ala
   65                  70                  75                  80
   Val Tyr Tyr Cys Gln Gln Tyr Gly Asn Ser Val Tyr Thr Phe Gly Gln
                   85                  90                  95
   Gly Thr Lys Leu Glu Ile Lys Arg
               100
(2) INFORMATION FOR SEQ ID NO:101:
   (i) SEQUENCE CHARACTERISTICS:
      (A) LENGTH: 106 amino acids
      (B) TYPE: amino acid
      (D) TOPOLOGY: linear
   (ii) MOLECULE TYPE: protein
   (xi) SEQUENCE DESCRIPTION: SEQ ID NO:101:
   Glu Leu Thr Gln Ser Pro Ser Ser Leu Ser Ala Ser Val Gly Asp Arg
   1               5                   10                  15
   Val Thr Ile Thr Cys Arg Thr Ser Gln Gly Ile Ser Asn Tyr Leu Ala
               20                  25                  30
   Trp Tyr Gln Gln Lys Pro Gly Lys Val Pro Lys Leu Leu Ile Tyr Gly
           35                  40                  45
   Ala Ser Thr Leu Gln Ser Gly Gly Pro Ser Arg Phe Ser Gly Ser Gly
       50                  55                  60
   Ser Gly Thr Asp Phe Thr Leu Thr Ile Asn Ser Leu Gln Pro Glu Asp
   65                  70                  75                  80
   Val Ala Thr Tyr Ser Cys Gln Asn Tyr Asp Ser Ala Pro Trp Thr Phe
                   85                  90                  95
   Gly Gln Gly Thr Lys Val Asp Ile Lys Arg
               100                 105
(2) INFORMATION FOR SEQ ID NO:102:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:102:
(2) INFORMATION FOR SEQ ID NO:103:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:103:
   Glu Leu Thr Gln Ser Pro Ser Ser Leu Ser Ala Ser Val Gly Asp Arg
   1               5                   10                  15
   Val Thr Ile Thr Cys Arg Ala Ser Gln Asn Ile Asn Asn Tyr Leu Asn
               20                  25                  30
   Trp Tyr Gln Gln Lys Pro Gly Glu Ala Pro Lys Leu Leu Ile His Thr
           35                  40                  45
   Ala Phe Asn Leu Gln Ser Gly Val Pro Ser Arg Phe Ser Gly Thr Ala
       50                  55                  60
   Ser Gly Thr Glu Phe Thr Leu Thr Ile Arg Ser Leu Gln Pro Glu Asp
   65 70 75 80
   Phe Ala Thr Tyr Tyr Cys Gln Gln Ser Tyr Ser Thr Pro Tyr Thr Phe
   85 90 95
   Gly Gln Gly Thr Lys Val Glu Ile Lys Arg Thr
   100 105
(2) INFORMATION FOR SEQ ID NO:104:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:104:
   Glu Leu Thr Gln Ser Pro Ser Ser Leu Ser Ala Ser Val Gly Asp Arg
   1 5 10 15
   Val Thr Ile Thr Cys Arg Ala Ser Gln Ser Ile Ser Ser Tyr Leu Asn
   20 25 30
   Trp Tyr Gln Gln Lys Pro Gly Lys Ala Pro Lys Leu Leu Ile Tyr Ala
   35 40 45
   Ala Ser Ser Leu Gln Ser Gly Val Pro Ser Arg Phe Ser Gly Ser Gly
   50 55 60
   Ser Gly Thr Asp Phe Thr Leu Thr Ile Ser Ser Leu Gln Pro Glu Asp
   65 70 75 80
   Phe Ala Thr Tyr Tyr Cys Gln Gln Ser Tyr Ser Thr Pro Tyr Thr Phe
   85 90 95
   Gly Gln Gly Thr Lys Leu Glu Ile Lys Arg Thr
   100 105
(2) INFORMATION FOR SEQ ID NO:105:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:105:
   Glu Leu Thr Gln Ser Pro Ser Ser Leu Ser Ala Ser Val Gly Asp Arg
   1 5 10 15
   Val Thr Ile Thr Cys Arg Ala Ser Gln Ser Ile Ser Ser Tyr Leu Asn
   20 25 30
   Trp Tyr Gln Gln Lys Pro Gly Lys Ala Pro Lys Leu Leu Ile Tyr Ala
   35 40 45
   Ala Ser Ser Leu Gln Ser Gly Val Pro Ser Arg Phe Ser Gly Ser Gly
   50 55 60
   Ser Gly Thr Asp Phe Thr Leu Thr Ile Ser Ser Leu Gln Pro Glu Asp
   65 70 75 80
   Phe Ala Thr Tyr Tyr Cys Gln Gln Ser Tyr Ser Thr Pro Gln Thr Phe
   85 90 95
   Gly Gln Gly Thr Lys Leu Glu Ile Lys Arg Thr
   100 105
(2) INFORMATION FOR SEQ ID NO:106:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO:106:
   Gln Ser Pro Ser Ser Leu Ser Ala Ser Val Gly Asp Arg Val Thr Ile
   1 5 10 15
   Thr Cys Arg Ala Ser Gln Thr Ile Ser Ser Tyr Leu Asn Trp Tyr Gln
   20 25 30
   Gln Lys Pro Gly Lys Ala Pro Lys Leu Leu Ile Tyr Ala Ala Ser Ser
   35 40 45
   Leu Gln Ser Gly Val Pro Ser Arg Phe Ser Gly Gly Gly Ser Gly Thr
   50 55 60
   Asp Phe Thr Leu Thr Ile Ser Ser Leu Gln Pro Glu Asp Phe Ala Thr
   65 70 75 80
   Tyr Tyr Cys Gln Gln Ser Tyr Ser Thr Pro Tyr Thr Phe Gly Gln Gly
   85 90 95
   Thr Lys Leu Glu Ile Lys Arg Thr
   100
(2) INFORMATION FOR SEQ ID NO:107:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:107:
   Glu Leu Thr Gln Ser Pro Ser Ser Leu Ser Ala Ser Val Gly Asp Arg
   1 5 10 15
   Val Thr Ile Thr Cys Gln Ala Ser Gln Asp Ile Arg Asn Tyr Leu Asn
   20 25 30
   Trp Tyr Gln Gln Lys Pro Gly Lys Ala Pro Lys Leu Leu Ile Tyr Asp
   35 40 45
   Ala Ser Asn Ser Glu Thr Gly Val Pro Ser Arg Phe Ser Gly Ser Gly
   50 55 60
   Ser Gly Arg Asp Phe Thr Phe Thr Ile Ser Ser Leu Gln Pro Glu Asp
   65 70 75 80
   Val Ala Thr Tyr Tyr Cys Gln Gln His Gln Asn Val Pro Leu Thr Phe
   85 90 95
   Gly Gly Gly Thr Lys Val Glu Ile Lys Arg Thr
   100 105
(2) INFORMATION FOR SEQ ID NO:108:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:108:
   Glu Leu Thr Gln Ser Pro Ser Ser Leu Ser Ala Ser Val Gly Asp Arg
   1               5                   10                  15
   Val Thr Ile Thr Cys Gln Ala Ser Gln Asp Ile Ser Asn His Leu Asn
               20                  25                  30
   Trp Tyr Gln Gln Lys Pro Gly Lys Ala Pro Lys Leu Leu Ile Tyr Asp
           35                  40                  45
   Ala Ser Asn Leu Glu Thr Gly Val Pro Ser Arg Phe Ser Gly Ser Gly
       50                  55                  60
   Ser Gly Thr Asp Phe Thr Phe Thr Ile Ser Ser Leu Gln Pro Glu Asp
   65                  70                  75                  80
   Ile Ala Thr Tyr Tyr Cys Gln Gln Tyr Asp Asn Leu Pro Leu Thr Phe
                   85                  90                  95
   Gly Gly Gly Thr Lys Val Glu Ile Lys Arg Thr
               100                 105
(2) INFORMATION FOR SEQ ID NO:109:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:109:
   Glu Leu Thr Gln Ser Pro Ser Ser Leu Ser Ala Ser Val Gly Asp Arg
   1               5                   10                  15
   Ile Thr Ile Thr Cys Arg Ala Ser Gln Thr Ile Asn Asn Tyr Leu Asn
               20                  25                  30
   Trp Tyr Gln Gln Lys Pro Gly Lys Ala Pro Lys Leu Leu Ile Tyr Gly
           35                  40                  45
   Ala Ser Asn Leu Gln Ser Gly Val Pro Ser Arg Phe Ser Gly Ser Gly
       50                  55                  60
   Ser Gly Thr Asp Phe Thr Leu Thr Ile Ser Ser Leu Gln Pro Glu Asp
   65                  70                  75                  80
   Phe Ala Thr Tyr Phe Cys Gln Gln Ser Tyr Asn Thr Pro Pro Trp Thr
                   85                  90                  95
   Phe Gly Gln Gly Thr Lys Val Glu Ile Lys Arg Thr
   100 105
(2) INFORMATION FOR SEQ ID NO:110:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO:110:
(2) INFORMATION FOR SEQ ID NO:111:
   (i) SEQUENCE CHARACTERISTICS:
      (A) LENGTH: 93 amino acids
      (B) TYPE: amino acid
      (D) TOPOLOGY: linear
   (ii) MOLECULE TYPE: protein
   (xi) SEQUENCE DESCRIPTION: SEQ ID NO:111:
   Glu Arg Ala Thr Leu Ser Cys Arg Ala Ser Gln Arg Val Asn Ser Asn
   1 5 10 15
   Tyr Leu Ala Trp Tyr Gln Gln Lys Pro Gly Gln Thr Pro Arg Val Val
   20 25 30
   Ile Tyr Ser Thr Ser Arg Arg Ala Thr Gly Val Pro Asp Arg Phe Ser
   35 40 45
   Gly Ser Gly Ser Gly Thr Asp Phe Thr Leu Thr Ile Ser Arg Leu Glu
   50 55 60
   Pro Glu Asp Phe Ala Val Tyr Tyr Cys Gln Gln Phe Gly Asp Ala Gln
   65 70 75 80
   Tyr Thr Phe Gly Gln Gly Thr Lys Leu Glu Ile Lys Arg
   85 90
(2) INFORMATION FOR SEQ ID NO:112:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO:112:
   Thr Gln Ser Pro Ser Ser Val Ser Ala Ser Val Gly Asp Thr Val Thr
   1 5 10 15
   Phe Thr Cys Arg Ala Ser Gln Asp Ile Arg Asn Tyr Leu Asn Trp Tyr
   20 25 30
   His Gln Lys Pro Gly Lys Ala Pro Lys Leu Leu Ile Ser Asp Ala Ser
   35 40 45
   Asp Leu Glu Ile Gly Val Pro Ser Arg Phe Ser Gly Ser Gly Ser Ala
   50 55 60
   Thr Tyr Phe Ser Phe Thr Ile Ser Ser Leu Gln Pro Glu Asp Ile Gly
   65 70 75 80
   Thr Tyr Tyr Cys Gln Gln Tyr Ala Asp Leu Ile Thr Phe Gly Gly Gly
   85 90 95
   Thr Lys Val Glu Ile Lys Arg Thr
   100
(2) INFORMATION FOR SEQ ID NO:113:
   (i) SEQUENCE CHARACTERISTICS:
      (A) LENGTH: 96 amino acids
      (B) TYPE: amino acid
      (D) TOPOLOGY: linear
   (ii) MOLECULE TYPE: protein
   (xi) SEQUENCE DESCRIPTION: SEQ ID NO:113:
   Ser Pro Gly Glu Arg Ala Thr Leu Ser Cys Arg Ala Ser Gln Ser Val
   1               5                   10                  15
   Gly Thr Asn Leu Ala Trp Tyr Gln Gln Lys Pro Gly Gln Ala Pro Arg
               20                  25                  30
   Leu Leu Ile Phe Asp Ala Ser Thr Arg Asp Thr Tyr Ile Pro Asp Thr
           35                  40                  45
   Phe Ser Gly Ser Gly Ser Gly Thr Asp Phe Ala Leu Thr Ile Ser Ser
       50                  55                  60
   Leu Gln Ser Glu Asp Phe Gly Phe Tyr Tyr Cys Gln Gln Tyr Asp Asn
   65                  70                  75                  80
   Trp Pro Pro Thr Phe Gly Gln Gly Thr Lys Leu Glu Val Lys Arg Thr
                   85                  90                  95
(2) INFORMATION FOR SEQ ID NO:114:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:114:
   Glu Leu Thr Gln Ser Pro Gly Thr Leu Ser Leu Ser Pro Gly Asp Arg
   1               5                   10                  15
   Ala Thr Phe Ser Cys Arg Ser Ser His Asn Ile Arg Ser Arg Arg Val
               20                  25                  30
   Ala Trp Tyr Gln His Lys Pro Gly Gln Ala Pro Arg Leu Val Ile His
           35                  40                  45
   Gly Val Ser Asn Arg Ala Ser Gly Ile Ser Asp Arg Phe Ser Gly Ser
   50 55 60
   Gly Ser Gly Thr Asp Phe Thr Leu Thr Ile Thr Arg Leu Glu Pro Glu
   65 70 75 80
   Asp Phe Ala Leu Tyr Tyr Cys Gln Val Tyr Gly Ala Ser Ser Tyr Thr
   85 90 95
   Phe Gly Gln Gly Thr Lys Leu Asp Phe Lys Arg
   100 105
(2) INFORMATION FOR SEQ ID NO:115:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:115:
   Glu Leu Thr Gln Ser Pro Gly Thr Leu Ser Leu Ser Pro Gly Glu Arg
   1 5 10 15
   Ala Thr Phe Ser Cys Arg Ser Ser His Asn Ile Arg Ser Arg Arg Val
   20 25 30
   Ala Trp Tyr Gln His Lys Pro Gly Gln Ala Pro Arg Leu Val Ile His
   35 40 45
   Gly Val Ser Asn Arg Ala Thr Gly Ile Ser Asp Arg Phe Ser Gly Ser
   50 55 60
   Gly Ser Gly Thr Asp Phe Thr Leu Thr Ile Thr Arg Leu Glu Pro Glu
   65 70 75 80
   Asp Phe Ala Leu Tyr Tyr Cys Gln Val Tyr Gly Ala Ser Ser Tyr Thr
   85 90 95
   Phe Gly Gln Gly Thr Lys Leu Asp Phe Lys Arg
   100 105
(2) INFORMATION FOR SEQ ID NO:116:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:116:
   Glu Leu Thr Gln Ser Pro Asp Thr Leu Ser Leu Asn Val Gly Glu Arg
   1               5                   10                  15
   Ala Thr Leu Ser Cys Arg Ala Ser His Arg Ile Ser Ser Arg Arg Leu
               20                  25                  30
   Ala Trp Tyr Gln His Lys Arg Gly Gln Ala Pro Arg Leu Leu Ile Tyr
           35                  40                  45
   Gly Val Ser Ser Arg Ala Gly Gly Val Pro Asp Arg Phe Ser Gly Ser
       50                  55                  60
   Gly Ser Gly Thr Asp Phe Ser Leu Thr Ile Ser Arg Leu Glu Pro Glu
   65                  70                  75                  80
   Asp Phe Ala Met Tyr Tyr Cys Gln Thr Tyr Gly Gly Ser Ser Tyr Thr
                   85                  90                  95
   Phe Gly Gln Gly Thr Lys Val Asp Ile Lys Arg
               100                 105
(2) INFORMATION FOR SEQ ID NO:117:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:117:
   Glu Leu Thr Gln Ser Pro Asp Thr Leu Ser Leu Asn Ala Gly Glu Arg
   1               5                   10                  15
   Ala Thr Leu Ser Cys Arg Ala Ser His Arg Ile Ser Ser Arg Arg Leu
               20                  25                  30
   Ala Trp Tyr Gln His Lys Arg Gly Gln Ala Pro Arg Leu Leu Ile Tyr
           35                  40                  45
   Gly Val Ser Asn Arg Ala Gly Gly Val Pro Asp Arg Phe Ser Gly Ser
       50                  55                  60
   Gly Ser Gly Thr Asp Phe Ser Leu Thr Ile Ser Arg Leu Glu Pro Glu
   65 70 75 80
   Asp Phe Ala Ile Tyr Tyr Cys Gln Thr Tyr Gly Gly Ser Ser Tyr Thr
   85 90 95
   Phe Gly Gln Gly Thr Thr Val Asp Ile Lys Arg
   100 105
(2) INFORMATION FOR SEQ ID NO:118:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:118:
   Glu Leu Thr Gln Ser Pro Asp Thr Leu Ser Leu Asn Thr Gly Glu Arg
   1 5 10 15
   Ala Thr Leu Ser Cys Arg Ala Ser His Arg Ile Gly Ser Arg Arg Leu
   20 25 30
   Ala Trp Tyr Gln His Arg Arg Gly Gln Ala Pro Arg Leu Leu Ile Tyr
   35 40 45
   Gly Val Ser Asn Arg Ala Gly Gly Val Pro Asp Arg Phe Ser Gly Ser
   50 55 60
   Gly Ser Gly Thr Asp Phe Thr Leu Thr Ile Ser Arg Leu Glu Pro Glu
   65 70 75 80
   Asp Phe Ala Ile Tyr Tyr Cys Gln Thr Tyr Gly Gly Ser Ser Tyr Thr
   85 90 95
   Phe Gly Gln Gly Thr Lys Val Asp Ile Lys Arg
   100 105
(2) INFORMATION FOR SEQ ID NO:119:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:119:
   Glu Leu Thr Gln Ser Pro Gly Thr Leu Ser Leu Thr Pro Gly Glu Arg
   1 5 10 15
   Ala Ile Leu Ser Cys Lys Thr Ser His Asn Ile Trp Ser Arg Arg Leu
   20 25 30
   Ala Trp Tyr Gln Leu Lys Ser Gly Gln Ala Pro Arg Leu Leu Ile Tyr
   35 40 45
   Gly Val Ser Lys Arg Ala Gly Gly Ile Pro Asp Arg Phe Ser Gly Ser
   50 55 60
   Gly Ser Ala Thr Asp Phe Thr Leu Thr Ile Ser Arg Val Glu Pro Glu
   65 70 75 80
   Asp Phe Ala Val Tyr Tyr Cys Gln Thr Tyr Gly Gly Ser Ala Tyr Thr
   85 90 95
   Phe Gly Gln Gly Thr Lys Leu Asp Ile Lys Arg
   100 105
(2) INFORMATION FOR SEQ ID NO:120:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:120:
   Glu Leu Thr Gln Ser Pro Gly Thr Leu Ser Leu Thr Pro Gly Glu Arg
   1 5 10 15
   Ala Ile Leu Ser Cys Lys Thr Ser His Asn Ile Trp Ser Arg Arg Leu
   20 25 30
   Ala Trp Tyr Gln Leu Lys Ser Gly Gln Ala Pro Arg Leu Leu Ile Tyr
   35 40 45
   Gly Val Ser Lys Arg Ala Gly Gly Ile Pro Asp Arg Phe Ser Gly Ser
   50 55 60
   Gly Ser Ala Thr Asp Phe Thr Leu Thr Ile Ser Arg Val Glu Pro Glu
   65 70 75 80
   Asp Phe Ala Val Tyr Tyr Cys Gln Thr Tyr Gly Gly Ser Ala Tyr Thr
   85 90 95
   Phe Gly Gln Gly Thr Lys Leu Glu Ile Lys Arg
   100 105
(2) INFORMATION FOR SEQ ID NO:121:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:121:
   Glu Leu Thr Gln Ser Pro Gly Thr Leu Ser Ser Thr Pro Gly Glu Arg
   1 5 10 15
   Ala Ile Leu Ser Cys Lys Thr Ser His Asn Ile Trp Ser Arg Arg Leu
   20 25 30
   Ala Trp Tyr Gln Val Lys Ser Gly Leu Pro Pro Arg Leu Leu Ile His
   35 40 45
   Gly Val Ser Arg Arg Ala Gly Gly Ile Pro Asp Arg Phe Ser Gly Ser
   50 55 60
   Gly Ser Ala Arg Asp Phe Thr Leu Thr Ile Ser Arg Leu Glu Pro Ala
   65 70 75 80
   Asp Phe Ala Val Tyr Tyr Cys Gln Thr Tyr Gly Gly Ser Ser Tyr Ser
   85 90 95
   Phe Gly Gln Gly Thr Lys Leu Asp Phe Asn Arg
   100 105
(2) INFORMATION FOR SEQ ID NO:122:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO:122:
   Glu Leu Thr Gln Ser Pro Gly Thr Leu Ser Leu Asn Pro Gly Glu Arg
   1 5 10 15
   Ala Val Leu Ser Cys Arg Thr Ser Arg Asn Ile Trp Ser Arg Arg Leu
   20 25 30
   Ala Trp Tyr Gln Val Arg Arg Gly Gln Ala Pro Arg Leu Leu Ile His
   35 40 45
   Gly Val Ser Lys Arg Ala Gly Gly Val Pro Asp Arg Phe Ser Gly Ser
   50 55 60
   Gly Ser Ala Arg Asp Phe Thr Leu Thr Ile Ser Arg Leu Glu Pro Glu
   65 70 75 80
   Asp Phe Ala Val Tyr Phe Cys Gln Thr Tyr Gly Gly Ser Ser Tyr Thr
   85 90 95
   Phe Gly Gln Gly Asn Lys Leu Asp Ile Arg Arg
   100 105
(2) INFORMATION FOR SEQ ID NO:123:
   (i) SEQUENCE CHARACTERISTICS:
      (A) LENGTH: 126 amino acids
      (B) TYPE: amino acid
      (D) TOPOLOGY: linear
   (ii) MOLECULE TYPE: protein
   (xi) SEQUENCE DESCRIPTION: SEQ ID NO:123:
   Gln Val Lys Leu Leu Glu Gln Ser Gly Ala Glu Val Lys Lys Pro Gly
   1 5 10 15
   Ala Ser Val Lys Val Ser Cys Gln Ala Ser Gly Tyr Arg Phe Ser Asn
   20 25 30
   Phe Val Leu His Trp Ala Arg Gln Ala Pro Gly His Arg Pro Glu Trp
   35 40 45
   Met Gly Trp Ile Asn Pro Ala Asn Gly Val Thr Glu Ile Pro Pro Lys
   50 55 60
   Phe Gln Asp Arg Val Ser Leu Thr Arg Asp Thr Ser Ala Gly Thr Val
   65 70 75 80
   Tyr Leu Glu Leu Thr Asn Leu Arg Phe Ala Asp Thr Ala Val Tyr Tyr
   85 90 95
   Cys Ala Arg Val Gly Glu Trp Thr Trp Asp Asp Ser Pro Gln Asp Asn
   100 105 110
   Tyr Tyr Met Asp Val Trp Gly Lys Gly Thr Thr Val Thr Val
   115 120 125
(2) INFORMATION FOR SEQ ID NO:124:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:124:
   Gln Val Lys Leu Leu Glu Gln Ser Gly Ala Glu Val Lys Lys Pro Gly
   1 5 10 15
   Ala Ser Val Lys Val Ser Cys Gln Ala Ser Gly Tyr Arg Phe Ser Asn
   20 25 30
   Phe Val Leu His Trp Ala Arg Gln Ala Pro Gly His Arg Pro Glu Trp
   35 40 45
   Met Gly Trp Ile Asn Pro Ala Asn Gly Val Thr Glu Ile Ser Pro Lys
   50 55 60
   Phe Gln Asp Arg Val Ser Leu Thr Gly Asp Thr Ser Ala Ser Thr Val
   65 70 75 80
   Tyr Leu Glu Leu Arg Asn Leu Arg Phe Ala Asp Thr Ala Val Tyr Tyr
   85 90 95
   Cys Ala Arg Val Gly Glu Trp Thr Trp Asp Asp Ser Pro Gln Asp Asn
   100 105 110
   Tyr Tyr Met Asp Val Trp Gly Arg Gly Thr Thr Val Thr
   115 120 125
(2) INFORMATION FOR SEQ ID NO:125:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:125:
   Gln Val Lys Leu Leu Glu Gln Ser Gly Ala Glu Val Lys Lys Pro Gly
   1               5                   10                  15
   Ala Ser Val Lys Val Ser Cys Gln Ala Ser Gly Tyr Arg Phe Ser Asn
               20                  25                  30
   Phe Val Leu His Trp Ala Arg Gln Ala Pro Gly His Arg Pro Glu Trp
           35                  40                  45
   Met Gly Trp Ile Asn Pro Ala Asn Gly Val Thr Glu Ile Ser Pro Lys
       50                  55                  60
   Phe Gln Asp Arg Val Ser Leu Thr Gly Asp Thr Ser Ala Ser Thr Val
   65                  70                  75                  80
   Tyr Leu Glu Leu Arg Ser Leu Arg Phe Ala Asp Thr Ala Val Tyr Tyr
                   85                  90                  95
   Cys Ala Arg Val Gly Glu Trp Thr Trp Asp Asp Ser Pro Gln Asp Asn
               100                 105                 110
   Tyr Tyr Met Asp Val Trp Gly Lys Gly Thr Thr Val
           115                 120
(2) INFORMATION FOR SEQ ID NO:126:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:126:
   Gln Val Lys Leu Leu Glu Gln Ser Gly Ala Glu Val Lys Lys Pro Gly
   1               5                   10                  15
   Ala Ser Val Lys Ile Ser Cys Gln Ala Ser Gly Tyr Arg Phe Thr Asn
               20                  25                  30
   Phe Val Leu His Trp Ala Arg Gln Ala Pro Gly Gln Arg Pro Glu Trp
           35                  40                  45
   Met Gly Trp Phe Asn Pro Ala Asn Gly Ile Lys Glu Ile Ser Pro Lys
       50                  55                  60
   Phe Gln Asp Arg Val Ser Phe Thr Gly Asp Thr Ser Ala Ser Thr Ala
   65 70 75 80
   Tyr Val Glu Leu Arg Asn Leu Arg Ser Ala Asp Thr Ala Val Tyr Tyr
   85 90 95
   Cys Ala Arg Val Gly Pro Trp Thr Trp Asp Asp Ser Pro Gln Asp Asn
   100 105 110
   Tyr Tyr Met Asp Val Trp Gly Lys Gly Thr Thr Val
   115 120
(2) INFORMATION FOR SEQ ID NO:127:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:127:
   Gln Val Lys Leu Leu Glu Gln Ser Gly Ala Glu Val Lys Lys Pro Gly
   1 5 10 15
   Ala Ser Val Lys Val Ser Cys Gln Ala Ser Gly Tyr Arg Phe Ser Asn
   20 25 30
   Phe Val Leu His Trp Ala Arg Gln Ala Pro Gly His Arg Pro Glu Trp
   35 40 45
   Met Gly Trp Ile Asn Pro Ala Asn Gly Val Thr Glu Ile Ser Pro Lys
   50 55 60
   Phe Gln Asp Arg Val Ser Leu Thr Gly Asp Thr Ser Ala Ser Thr Val
   65 70 75 80
   Tyr Leu Glu Leu Arg Asn Leu Arg Phe Ala Asp Thr Ala Val Tyr Tyr
   85 90 95
   Cys Ala Arg Val Gly Glu Trp Thr Trp Asp Asp Phe Pro Gln Asp Asn
   100 105 110
   Tyr Tyr Met Asp Val Trp Gly Lys Gly Thr Thr Val
   115 120
(2) INFORMATION FOR SEQ ID NO:128:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:128:
   Gln Val Lys Leu Leu Glu Gln Ser Gly Ala Glu Val Lys Lys Pro Gly
   1               5                   10                  15
   Ala Ser Val Lys Leu Ser Cys Gln Ala Ser Gly Tyr Arg Phe Ser Asn
               20                  25                  30
   Phe Val Leu His Trp Ala Arg Gln Ala Pro Gly His Arg Pro Glu Trp
           35                  40                  45
   Met Gly Trp Ile Asn Pro Ala Asn Gly Val Thr Glu Ile Ser Pro Lys
       50                  55                  60
   Phe Gln Asp Arg Val Ser Leu Thr Gly Asp Thr Ser Ala Ser Thr Val
   65                  70                  75                  80
   Tyr Leu Glu Leu Arg Asn Leu Arg Phe Ala Asp Thr Ala Val Tyr Tyr
                   85                  90                  95
   Cys Ala Arg Val Gly Glu Trp Thr Trp Asp Asp Ser Pro Gln Asp Asn
               100                 105                 110
   Tyr Tyr Met Asp Val Trp Gly Lys Gly Thr Thr Val Thr
           115                 120                 125
(2) INFORMATION FOR SEQ ID NO:129:
   (i) SEQUENCE CHARACTERISTICS:
      (A) LENGTH: 125 amino acids
      (B) TYPE: amino acid
      (D) TOPOLOGY: linear
   (ii) MOLECULE TYPE: protein
   (xi) SEQUENCE DESCRIPTION: SEQ ID NO:129:
   Gln Val Lys Leu Leu Glu Gln Ser Gly Thr Glu Val Lys Lys Pro Gly
   1               5                   10                  15
   Ala Ser Val Lys Ile Ser Cys Lys Ala Ser Gly Tyr Arg Phe Thr Asn
               20                  25                  30
   Phe Pro Leu His Trp Val Arg Gln Ala Pro Gly Gln Arg Pro Glu Trp
   35 40 45
   Met Gly Trp Ile Lys Ile Val Asn Gly Glu Lys Lys Tyr Ser Gln Lys
   50 55 60
   Phe Val Asp Arg Val Thr Phe Thr Gly Asp Thr Ser Ala Asn Thr Ala
   65 70 75 80
   Tyr Met Glu Val Arg Gly Leu Arg Ser Ala Asp Thr Ala Thr Tyr Tyr
   85 90 95
   Cys Ala Arg Val Gly Glu Trp Thr Trp Asp Met Asp Pro Gln Ala Asn
   100 105 110
   Tyr Tyr Met Asp Val Trp Gly Lys Gly Thr Thr Val Thr
   115 120 125
(2) INFORMATION FOR SEQ ID NO:130:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:130:
   Gln Val Lys Leu Leu Glu Gln Ser Gly Ala Glu Val Lys Lys Pro Gly
   1 5 10 15
   Ala Ser Val Lys Val Ser Cys Gln Ala Ser Gly Tyr Arg Phe Ser Asn
   20 25 30
   Phe Val Ile His Trp Val Arg Gln Ala Pro Gly Gln Arg Phe Glu Trp
   35 40 45
   Met Gly Trp Ile Asn Pro Tyr Asn Gly Asn Lys Glu Phe Ser Ala Lys
   50 55 60
   Phe Arg Asp Arg Val Thr Phe Thr Ala Asp Thr Asp Ala Asn Thr Ala
   65 70 75 80
   Tyr Met Glu Leu Arg Ser Leu Arg Ser Ala Asp Thr Ala Ile Tyr Tyr
   85 90 95
   Cys Ala Arg Val Gly Pro Tyr Thr Trp Asp Asp Ser Pro Gln Asp Asn
   100 105 110
   Tyr Tyr Met Asp Val Trp Gly Lys Gly Thr Thr Val
   115 120
(2) INFORMATION FOR SEQ ID NO:131:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:131:
   Gln Val Lys Leu Leu Glu Gln Ser Gly Ala Glu Val Lys Lys Pro Gly
   1 5 10 15
   Ala Ser Val Lys Val Ser Cys Gln Ala Ser Gly Tyr Arg Phe Ser Asn
   20 25 30
   Phe Val Leu His Trp Ala Arg Gln Ala Pro Thr Gln Asp Leu Glu Trp
   35 40 45
   Met Gly Trp Ile Asn Pro Ala Asn Gly Val Lys Glu Ile Ser Pro Lys
   50 55 60
   Phe Gln Asp Arg Val Ser Leu Thr Gly Asp Thr Ser Ala Ser Thr Val
   65 70 75 80
   Tyr Leu Glu Leu Arg Ser Leu Arg Phe Ala Asp Thr Ala Val Tyr Tyr
   85 90 95
   Cys Ala Arg Val Gly Glu Trp Thr Trp Asp Asp Ser Pro Gln Asp Asn
   100 105 110
   Tyr Tyr Met Asp Val Trp Gly Lys Gly Thr Thr Val
   115 120
(2) INFORMATION FOR SEQ ID NO:132:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:132:
   Gln Val Lys Leu Leu Glu Gln Ser Gly Ala Glu Val Lys Lys Pro Gly
   1               5                   10                  15
   Ala Ser Val Lys Val Ser Cys Gln Ala Ser Gly Tyr Arg Phe Ser Asn
               20                  25                  30
   Phe Val Leu His Trp Ala Arg Gln Ala Pro Gly His Arg Pro Glu Trp
           35                  40                  45
   Met Gly Trp Ile Asn Pro Ala Asn Gly Val Thr Glu Ile Pro Pro Lys
       50                  55                  60
   Phe Gln Asp Arg Val Ser Leu Thr Arg Asp Thr Ser Ala Gly Thr Val
   65                  70                  75                  80
   Tyr Leu Glu Leu Thr Asn Leu Arg Phe Ala Asp Thr Ala Val Tyr Tyr
                   85                  90                  95
   Cys Ala Arg Val Gly Glu Trp Thr Trp Asp Asp Ser Pro Gln Asp Asn
               100                 105                 110
   Tyr Tyr Met Asp Val Trp Gly Lys Gly Thr Thr Val
           115                 120
(2) INFORMATION FOR SEQ ID NO:133:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:133:
   TCGAGGGTCG GTCGGTCTCT AGACGGTCGG TCGGTCA 37
(2) INFORMATION FOR SEQ ID NO:134:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:134:
   CTAGTGACCG ACCGACCGTC TAGAGACCGA CCGACCC 37
(2) INFORMATION FOR SEQ ID NO:135:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:135:
   CGGTCGGTCG GTCCTCGAGG GTCGGTCGGT CT 32
(2) INFORMATION FOR SEQ ID NO:136:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:136:
   CTAGAGACCG ACCGACCCTC GAGGACCGAC CGACCGAGCT 40
(2) INFORMATION FOR SEQ ID NO:137:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:137:
   CAAGGAGACA GGATCCATGA AATAC 25
(2) INFORMATION FOR SEQ ID NO:138:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:138:
   AGGGCGAATT GGATCCCGGG CCCCC 25
(2) INFORMATION FOR SEQ ID NO:139:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:139:
   CTAGTCATCA TCATCATCAT TAAGCTAGC 29
(2) INFORMATION FOR SEQ ID NO:140:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (xi) SEQUENCE DESCRIPTION: SEQ ID NO:140:
   CTAGGCTAGC TTAATGATGA TGATGATGA                                   29
(2) INFORMATION FOR SEQ ID NO:141:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 1
      (D) OTHER INFORMATION: /label= J
   (ix) FEATURE:
      (A) NAME/KEY: Modified-site
      (B) LOCATION: 13
      (D) OTHER INFORMATION: /label= ZC
   (xi) SEQUENCE DESCRIPTION: SEQ ID NO:141:
   Ser Ile Ser Ile Gly Pro Gly Arg Ala Phe Tyr Thr Gly
   1               5                   10
(2) INFORMATION FOR SEQ ID NO:142:
   (i) SEQUENCE CHARACTERISTICS:
      (A) LENGTH: 126 amino acids
      (B) TYPE: amino acid
      (D) TOPOLOGY: linear
   (ii) MOLECULE TYPE: protein
   (xi) SEQUENCE DESCRIPTION: SEQ ID NO:142:
   Leu Leu Glu Ser Gly Pro Gly Leu Val Lys Pro Ser Glu Thr Leu Ser
   1               5                   10                  15
   Leu Thr Cys Thr Val Ser Gly Gly Ser Leu Ser Ser Phe Asp Trp Asn
               20                  25                  30
   Trp Ile Arg Gln Pro Ala Gly Lys Gly Leu Glu Trp Ile Gly Arg Ile
           35                  40                  45
   Tyr Pro Ser Gly Asn Thr His Tyr Asn Pro Ser Leu Arg Ser Arg Val
       50                  55                  60
   Thr Met Ser Arg Asp Thr Ser Lys Asn Gln Phe Ser Val Lys Leu Thr
   65                  70                  75                  80
   Ser Val Thr Ala Ala Asp Thr Ala Leu Tyr Tyr Cys Ala Arg Glu Asn
                   85                  90                  95
   Thr Gly Arg Thr Ile Glu Glu Ile Gly Asn Phe Phe Asp Ile Trp Gly
               100                 105                 110
   Gln Gly Thr Leu Val Thr Val Ser Ser Ala Ser Thr Lys Gly
           115                 120                 125
(2) INFORMATION FOR SEQ ID NO:143:
   (i) SEQUENCE CHARACTERISTICS:
      (A) LENGTH: 122 amino acids
      (B) TYPE: amino acid
      (D) TOPOLOGY: linear
   (ii) MOLECULE TYPE: protein
   (xi) SEQUENCE DESCRIPTION: SEQ ID NO:143:
   Leu Leu Lys Ser Gly Gly Gly Leu Val Lys Pro Gly Gly Ser Leu Arg
   1 5 10 15
   Leu Ser Cys Val Ile Ser Ala Phe Ser Phe Ser Gly Tyr Asn Ile Asn
   20 25 30
   Trp Val Arg Gln Ala Pro Gly Lys Gly Leu Glu Trp Val Ser Ser Ile
   35 40 45
   Ser Met Ser Thr Gly Ser Leu Ser Tyr Ala Asp Ser Met Lys Gly Arg
   50 55 60
   Phe Thr Ile Ser Arg Asp Asn Ala Lys Asn Ser Val Tyr Leu Glu Met
   65 70 75 80
   Ser Ser Leu Thr Ala Glu Asp Thr Ala Met Tyr Tyr Cys Ala Ala Arg
   85 90 95
   Thr Pro Leu Val Gly Arg Ala Leu Asp Ile Trp Gly Gln Gly Thr Val
   100 105 110
   Val Thr Val Ser Ser Ala Ser Thr Lys Gly
   115 120
(2) INFORMATION FOR SEQ ID NO:144:
   (i) SEQUENCE CHARACTERISTICS:
      (A) LENGTH: 132 amino acids
      (B) TYPE: amino acid
      (D) TOPOLOGY: linear
   (ii) MOLECULE TYPE: protein
   (xi) SEQUENCE DESCRIPTION: SEQ ID NO:144:
   Leu Leu Glu Ser Gly Gly Gly Leu Val Lys Pro Gly Gly Ser Leu Arg
   1 5 10 15
   Leu Ser Cys Ser Ala Ser Gly Phe Thr Phe Ser Ser Tyr Gly Met Asn
   20 25 30
   Trp Val Arg Gln Ala Pro Gly Lys Gly Pro Glu Trp Val Ala Tyr Ile
   35 40 45
   Ser Ser Ser Arg Lys Tyr Thr Glu Tyr Ala Asp Ser Val Lys Gly Arg
   50 55 60
   Phe Thr Ile Ser Arg Glu Asn Ala Lys Tyr Ser Val Phe Leu Gln Leu
   65 70 75 80
   Asp Ser Leu Thr Ala Glu Asp Thr Ala Ile Tyr Tyr Cys Ala Arg Gly
   85 90 95
   Arg Asp Phe Tyr Ser Gly Phe Gly Arg Arg Asp Asp Phe His Leu His
   100 105 110
   Tyr Met Asp Val Trp Gly Lys Gly Thr Thr Val Thr Val Ser Ser Ala
   115 120 125
   Ser Thr Lys Gly
   130
(2) INFORMATION FOR SEQ ID NO:145:
   (i) SEQUENCE CHARACTERISTICS:
      (A) LENGTH: 126 amino acids
      (B) TYPE: amino acid
      (D) TOPOLOGY: linear
   (ii) MOLECULE TYPE: protein
   (xi) SEQUENCE DESCRIPTION: SEQ ID NO:145:
   Leu Leu Glu Gln Ser Gly Gly Gly Leu Val Gln Pro Gly Gly Ser Leu
   1 5 10 15
   Arg Ile Ser Cys Val Ala Ser Gly Asp Ile Phe Tyr Ser Tyr Ala Met
   20 25 30
   Ser Trp Val Arg Gln Ala Pro Gly Lys Gly Leu Glu Trp Val Ala Ser
   35 40 45
   Ile Ser Gly Thr Gly Gly Ser Asn Tyr Tyr Ala Asp Ser Val Lys Gly
   50 55 60
   Arg Phe Thr Ile Ser Arg Asp Asn Ser Lys Ser Thr Leu Tyr Leu Gln
   65 70 75 80
   Met Asn Ser Leu Arg Ala Glu Asp Thr Ala Leu Tyr Tyr Cys Ala Arg
   85 90 95
   Asp Arg Gly Pro Arg Ile Gly Ile Arg Gly Trp Phe Asp Ser Trp Gly
   100 105 110
   Gln Gly Thr Leu Val Thr Val Ser Ser Ala Ser Thr Lys Gly
   115 120 125
(2) INFORMATION FOR SEQ ID NO:146:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:146:
   Leu Leu Glu Ser Gly Gly Gly Leu Val Gln Pro Gly Gly Ser Leu Arg
   1 5 10 15
   Leu Ser Cys Ala Ala Ser Gly Phe Leu Tyr Ser Ser Phe Ala Met Ser
   20 25 30
   Trp Val Arg Gln Ala Pro Gly Lys Gly Leu Ala Trp Val Ser Thr Ile
   35 40 45
   Ser Ala Ser Gly Gly Ser Thr Lys Tyr Ala Asp Ser Val Lys Gly Arg
   50 55 60
   Phe Ile Ile Ser Arg Asp Asn Ser Lys Asn Thr Ile Tyr Leu Gln Met
   65 70 75 80
   Asp Ser Leu Arg Ala Glu Asp Thr Ala Val Tyr Tyr Cys Ala Lys Asn
   85 90 95
   Phe Arg Ala Phe Ala Arg Asp Pro Trp Gly Asp Trp Gly Gln Gly Thr
   100 105 110
   Leu Val Thr Val Ser Ser Ala Ser Ala Ser Thr Lys
   115 120
(2) INFORMATION FOR SEQ ID NO:147:
   (i) SEQUENCE CHARACTERISTICS:
      (A) LENGTH: 109 amino acids
      (B) TYPE: amino acid
      (D) TOPOLOGY: linear
   (ii) MOLECULE TYPE: protein
   (xi) SEQUENCE DESCRIPTION: SEQ ID NO:147:
   Met Ala Glu Leu Thr Gln Ser Pro Gly Thr Leu Ser Leu Ser Pro Gly
   1 5 10 15
   Glu Arg Val Ile Val Ser Cys Arg Ala Ser Gln Ser Val Ser Ser Asn
   20 25 30
   Tyr Leu Ala Trp Tyr Gln Gln Lys Pro Gly Gln Ala Pro Arg Leu Leu
   35 40 45
   Ile Tyr Gly Ala Ser Asn Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Gly
   85 90 95
   Thr Phe Gly Gln Gly Thr Lys Val Glu Ile Lys Arg Thr
   100 105
(2) INFORMATION FOR SEQ ID NO:148:
   (i) SEQUENCE CHARACTERISTICS:
      (A) LENGTH: 112 amino acids
      (B) TYPE: amino acid
      (D) TOPOLOGY: linear
   (ii) MOLECULE TYPE: protein
   (xi) SEQUENCE DESCRIPTION: SEQ ID NO:148:
   Met Ala Glu Leu Thr Gln Ser Pro Gly Thr Leu Ser Leu Ser Pro Gly
   1 5 10 15
   Glu Arg Ala Thr Phe Ser Cys Arg Ser Ser His Ser Ile His Thr Arg
   20 25 30
   Arg Val Ala Trp Tyr Gln His Lys Pro Gly Gln Ala Pro Arg Leu Val
   35 40 45
   Ile His Gly Val Ser Asn Arg Ala Ser Gly Ile Ser Asp Arg Phe Ser
   50 55 60
   Gly Ser Gly Ser Gly Thr Asp Phe Thr Leu Thr Ile Thr Arg Val Glu
   65 70 75 80
   Pro Glu Asp Phe Ala Leu Tyr Tyr Cys Gln Val Tyr Gly Ala Ser Ser
   85 90 95
   Tyr Thr Phe Gly Gln Gly Thr Lys Leu Glu Arg Lys Arg Thr Val Val
   100 105 110
(2) INFORMATION FOR SEQ ID NO:149:
   (i) SEQUENCE CHARACTERISTICS:
      (A) LENGTH: 111 amino acids
      (B) TYPE: amino acid
      (D) TOPOLOGY: linear
   (ii) MOLECULE TYPE: protein
   (xi) SEQUENCE DESCRIPTION: SEQ ID NO:149:
   Met Ala Glu Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Asn Gly
               20                  25                  30
   Tyr Leu Ala Trp Tyr Gln Gln Lys Pro Gly Gln Ala Pro Arg Leu Leu
           35                  40                  45
   Ile Tyr Gly Ala Ser Thr Arg Ala Thr Asp Ile Pro Asp Arg Phe Ser
       50                  55                  60
   Gly Ser Gly Ser Gly Ala Asp Phe Thr Leu Ala Ile Ser Arg Leu Glu
   65                  70                  75                  80
   Pro Glu Asp Phe Ala Val Tyr Tyr Cys Gln Gln Tyr Ala Gly Ser His
                   85                  90                  95
   Thr Phe Gly Gln Gly Thr Lys Leu Glu Ile Lys Arg Thr Val Ala
               100                 105                 110
(2) INFORMATION FOR SEQ ID NO:150:
   (i) SEQUENCE CHARACTERISTICS:
      (A) LENGTH: 111 amino acids
      (B) TYPE: amino acid
      (D) TOPOLOGY: linear
   (ii) MOLECULE TYPE: protein
   (xi) SEQUENCE DESCRIPTION: SEQ ID NO:150:
   Met Ala Glu Leu Thr Gln Ser Pro Ser Ser Leu Ser Ala Ser Val Gly
   1               5                   10                  15
   Asp Arg Val Thr Ile Thr Cys Arg Pro Ser Gln Gly Ile Gly Arg Phe
               20                  25                  30
   Phe Asn Trp Tyr Gln Gln Lys Pro Gly Lys Ala Pro Asn Leu Leu Ile
                   35                   40                   45
   Tyr Ala Ala Asp Ile Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Tyr
                   85                  90                  95
   Thr Phe Gly Gln Gly Thr Arg Leu Asp Ile Lys Arg Thr Val Ala
               100                 105                 110
(2) INFORMATION FOR SEQ ID NO:151:
   (i) SEQUENCE CHARACTERISTICS:
      (A) LENGTH: 112 amino acids
      (B) TYPE: amino acid
      (D) TOPOLOGY: linear
   (ii) MOLECULE TYPE: protein
   (xi) SEQUENCE DESCRIPTION: SEQ ID NO:151:
   Met Ala Glu Leu Thr Gln Ser Pro Ser Ser Leu Ser Ala Ser Val Gly
   1               5                   10                  15
   Asp Arg Val Thr Ile Thr Cys Arg Ala Ser Gln Gly Val Ser Ser Ser
               20                  25                  30
   Tyr Leu Ala Trp Tyr Gln Gln Lys Pro Gly Gln Ala Pro Arg Leu Val
           35                  40                  45
   Ile Phe Gly Ala Tyr Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Ile Thr Phe Gly Pro Gly Thr Lys Val Asp Ile Lys Arg Thr Val Ala
               100                 105                 110

## Description

### Technical Field

The present invention relates generally to the field of immunology and specifically to human monoclonal antibodies which bind and neutralize human immunodeficiency virus (HIV).

### Background

### 1. HIV Immunotherapy

HIV is the focus of intense studies as it is the causative agent for acquired immunodeficiency syndrome (AIDS). Immunotherapeutic methods are one of several approaches to prevention, cure or remediation of HIV infection and HIV-induced diseases. Specifically, the use of neutralizing antibodies in passive immunotherapies is of central importance to the present invention.

Passive immunization of HIV-1 infected humans using human sera containing polyclonal antibodies immunoreactive with HIV has been reported. See for example, Jackson et al., Lancet, September 17:647-652, (1988); Karpas et al., Proc. Natl. Acad. Sci., USA, 87:7613-7616 (1990).

Numerous groups have reported the preparation of human monoclonal antibodies that neutralize HIV isolates in vitro. The described antibodies typically have immunospecificities for epitopes on the HIV glycoprotein gp120 or the related external surface envelope glycoprotein gp120 or the transmembrane glycoprotein gp41. See, for example Levy, Micro. Rev., 57:183-289 (1993); Karwowska et al., Aids Research and Human Retroviruses, 8:1099-1106 (1992); Takeda et al., J. Clin. Invest., 89:1952-1957 (1992); Tilley et al., Aids Research and Human Retroviruses, 8:461-467 (1992); Laman et al., J. Virol., 66:1823-1831 (1992); Thali et al., J. Virol., 65:6188-6193 (1991); Ho et al., Proc. Natl. Acad. Sci. USA, 88:8549-8952 (1991); D'Souza et al., AIDS, 5:1061-1070 (1991); Tilley et al., Res. Virol., 142:247-259 (1991); Broliden et al., Immunol., 73:371-376 (1991); Matour et al., J. Immunol., 146:4325-4332 (1991); and Gorny et al., Proc. Natl. Acad. Sci., USA, 88:3238-3242 (1991).

To date, none of the reported human monoclonal antibodies have been shown to be effective in passive immunization therapies. Further, as monoclonal antibodies, they all each react with an individual epitope on the HIV envelope glycoprotein, gp120 or gp160. The epitope against which an effective neutralizing antibody immunoreacts has not been identified.

There continues to be a need to develop human monoclonal antibody preparations with significant HIV neutralization activity. In addition, there is a need for monoclonal antibodies immunoreactive with additional and diverse neutralizing epitopes on HIV gp120 in view of recent studies suggesting that gp120 is involved in both binding of the HIV virus to the cell as well as in postbinding events including envelope shedding and cleavage. See, for review, Levy, Micro. Rev., 57:183-289 (1993). Additional (new) epitope specificities are required because, upon passive immunization, the administered patient can produce an immune response against the administered antibody, thereby inactivating the particular therapeutic antibody.

### 2. Human Monoclonal Antibodies Produced From Combinatorial Phagremid Libraries

The use of filamentous phage display vectors, referred to as phagemids, has been repeatedly shown to allow the efficient preparation of large libraries of monoclonal antibodies having diverse and novel immunospecificities. The technology uses a filamentous phage coat protein membrane anchor domain as a means for linking gene-product and gene during the assembly stage of filamentous phage replication, and has been used for the cloning and expression of antibodies from combinatorial libraries. Kang et al., Proc. Natl. Acad. Sci., USA, 88:4363-4366 (1991). Combinatorial libraries of antibodies have been produced using both the cpVIII membrane anchor (Kang et al., supra) and the cpIII membrane anchor. Barbas et al., Proc. Natl. Acad. Sci., USA, 88:7978-7982 (1991).

The diversity of a filamentous phage-based combinatorial antibody library can be increased by shuffling of the heavy and light chain genes (Kang et al., Proc. Natl. Acad. Sci., USA, 88:11120-11123 (1991)), by altering the CDR3 regions of the cloned heavy chain genes of the library (Barbas et al., Proc. Natl. Acad. Sci., USA, 89:4457-4461 (1992)), and by introducing random mutations into the library by error-prone polymerase chain reactions (PCR) [Gram et al., Proc. Natl. Acad. Sci., USA, 89:3576-3580 (1992)].

Filamentous phage display vectors have also been utilized to produce human monoclonal antibodies immunoreactive with hepatitis B virus (HBV) or HIV antigens. See, for example Zebedee et al., Proc. Natl. Acad. Sci., USA, 89:3175-3179 (1992); and Burton et al., Proc. Natl. Acad. Sci., USA, 88:10134-10137 (1991), respectively. None of the previously described human monoclonal antibodies produced by phagemid vectors that are immunoreactive with HIV have been shown to neutralize HIV.

### Brief Description of the Invention

Methods have now been discovered using the phagemid vectors to identify and isolate from combinatorial libraries human monoclonal antibodies that neutralize HIV, and allow the rapid preparation of large numbers of neutralizing antibodies of completely human derivation. The identified neutralizing antibodies define new epitopes on the HIV gp120 glycoprotein, thereby increasing the availability of new immunotherapeutic human monoclonal antibodies.

The invention provides human monoclonal antibodies as defined in the appended claims that neutralize HIV, and also provides cell lines used to produce these monoclonal antibodies.

Also disclosed are amino acid sequences which confer neutralization function to the antigen binding domain of a monoclonal antibody, and which can be used immunogenically to identify other antibodies that specifically bind and neutralize HIV. The monoclonal antibodies of the invention find particular utility as reagents for the diagnosis and immunotherapy of HIV-induced disease.

A major advantage of the monoclonal antibodies of the invention derives from the fact that they are encoded by a human polynucleotide sequence. Thus, in vivo use of the monoclonal antibodies of the invention for diagnosis and immunotherapy of HIV-induced disease greatly reduces the problems of significant host immune response to the passively administered antibodies which is a problem commonly encountered when monoclonal antibodies of xenogeneic or chimeric derivation are utilized.

In one embodiment, the invention contemplates a human monoclonal antibody capable of immunoreacting with human immunodeficiency virus (HIV) glycoprotein gp120 and neutralizing HIV. A preferred human monoclonal antibody has the binding specificity of a monoclonal antibody comprising a heavy chain immunoglobulin variable region amino acid residue sequence selected from the group consisting of SEQ ID NOs 66, 67, 68, 70, 72, 73, 74, 75, 78 and 79. Another preferred human monoclonal antibody has the binding specificity of a monoclonal antibody comprising a light chain immunoglobulin variable region amino acid residue sequence selected from the group consisting of SEQ ID NOs 95, 96, 97, 98, 101, 102, 103, 104, 105, 107, 110, 115, 118, 121, 122, 124 and 132.

In another embodiment, the invention describes a polynucleotide sequence encoding a heavy or light chain immunoglobulin variable region amino acid residue sequence portion of a human monoclonal antibody of this invention. Also contemplated are DNA expression vectors containing the polynucleotide, and host cells containing the vectors and polynucleotides of the invention.

The invention also contemplates an in vitro method of detecting human immunodeficiency virus (HIV) comprising contacting a sample suspected of containing HIV with a diagnostically effective amount of the monoclonal antibody of this invention, and determining whether the monoclonal antibody immunoreacts with the sample. The method may include a variety of methods for determining the presence of an immunoreaction product.

In another embodiment, the invention describes the use of the monoclonal antibodies of the invention in the manufacture of a medicament for providing passive immunotherapy to human immunodeficiency virus (HIV) disease in a human. The medicament can be provided prophylactically, and by a parenteral administration. Pharmaceutical compositions containing one or more of the different human monoclonal antibodies are described for use in the described therapeutic methods.

### Brief Description of the Drawings

In the drawings forming a portion of this disclosure:

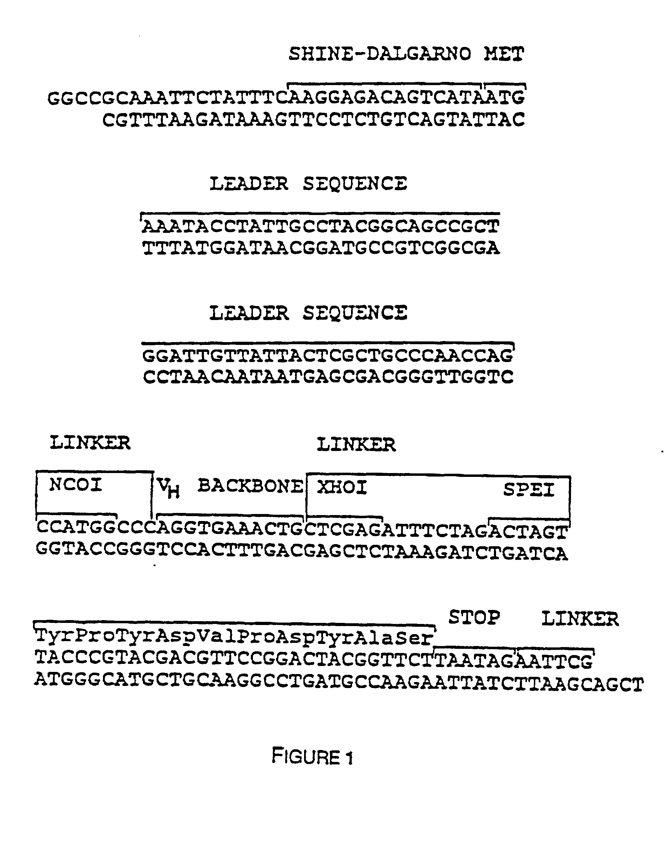

Figure 1 illustrates the sequence of the double-stranded synthetic DNA inserted into Lambda Zap to produce a Lambda Hc2 expression vector. The preparation of the double-stranded synthetic DNA insert is described in Example 1a2). The various features required for this vector to express the V_{H}-coding DNA homologs include the Shine-Dalgarno ribosome binding site, a leader sequence to direct the expressed protein to the periplasm as described by Mouva et al., J. Biol. Chem., 255:27, 1980, and various restriction enzyme sites used to operatively link the V_{H} homologs to the expression vector. The V_{H} expression vector sequence also contains a short nucleic acid sequence that codes for amino acids typically found in variable regions heavy chain (V_{H} backbone). This V_{H} backbone is just upstream and in the proper reading as the V_{H} DNA homologs that are operatively linked into the Xho I and Spe I cloning sites. The sequences of the top and bottom strands of the double-stranded synthetic DNA insert are listed respectively in SEQ ID NO 1 and SEQ ID NO 2. The ten amino acid sequence comprising the decapeptide tag is listed in SEQ ID NO 5. The synthetic DNA insert is directionally ligated into Lambda Zap II digested with the restriction enzymes Not 1 and Xho I to form Lambda Hc2 expression vector.

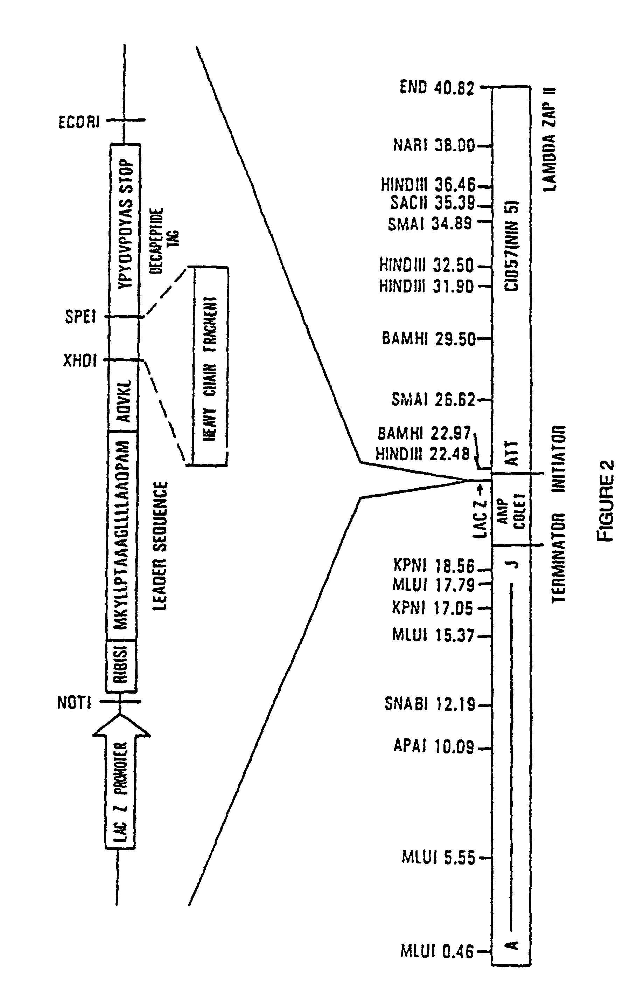

Figure 2 illustrates the major features of the bacterial expression vector Lambda Hc2 (V_{H} expression vector). The orientation of the insert in Lambda Zap II is shown. The V_{H} DNA homologs are inserted into the Xho I and Spe I cloning sites. The read through transcription produces the decapeptide epitope (tag) that is located just 3' of the cloning site. The amino acid residue sequence of the decapeptide tag and the Pel B leader sequence/spacer are respectively listed in SEQ ID NO 5 and 6.

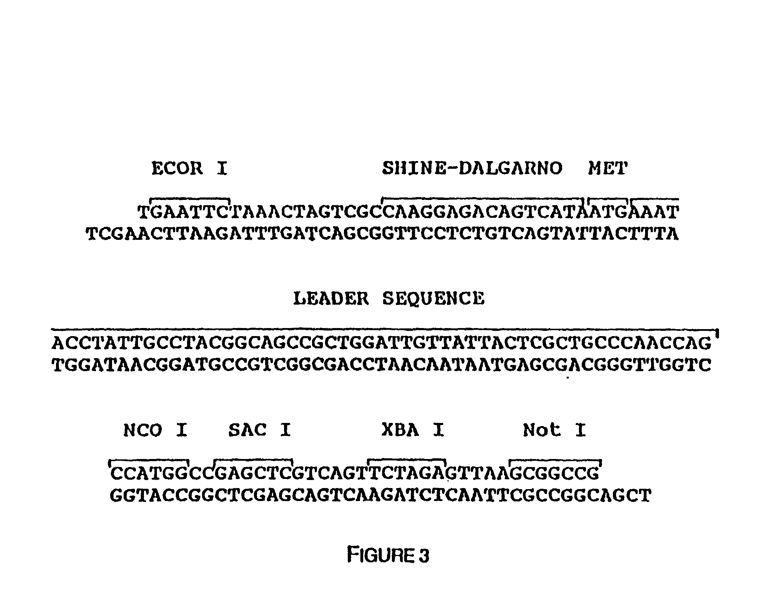

Figure 3 illustrates the sequence of the double-stranded synthetic DNA inserted into Lambda Zap to produce a Lambda Lc2 expression vector. The various features required for this vector to express the V_{L}-coding DNA homologs are described in Figure 1. The v_{L}-coding DNA homologs are operatively linked into the Lc2 sequence at the Sac I and Xho I restriction sites. The sequences of the top and bottom strands of the double-stranded synthetic DNA insert are listed respectively in SEQ ID NO 3 and SEQ ID NO 4. The synthetic DNA insert is directionally ligated into Lambda Zap II digested with the restriction enzymes Sac I and Not I to form Lambda Lc2 expression vector.

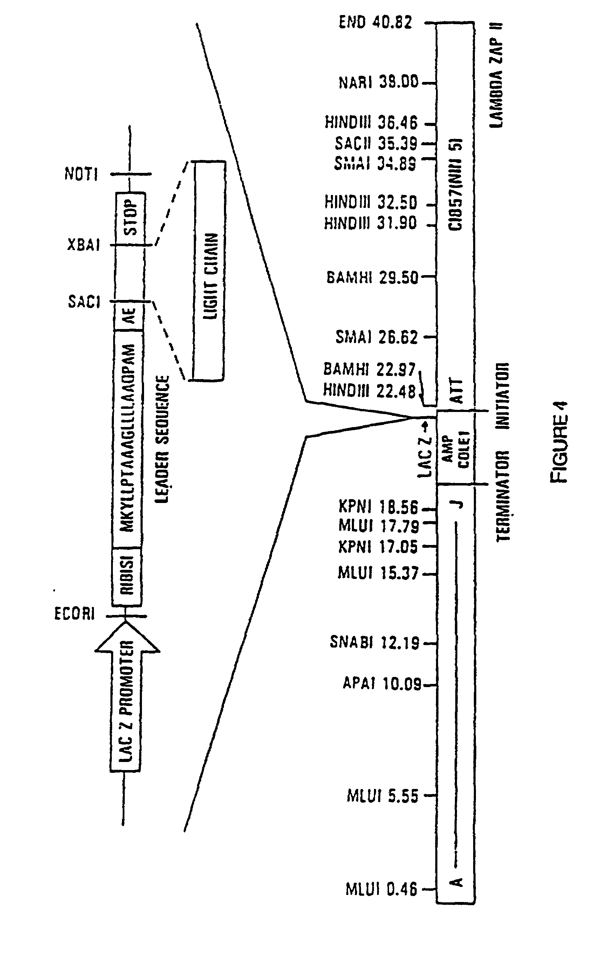

Figure 4 illustrates the major features of the bacterial expression vector Lc2 (V_{L} expression vector). The synthetic DNA sequence from Figure 3 is shown at the top along with the LacZ promoter from Lambda Zap II. The orientation of the insert in Lambda Zap II is shown. The V_{L} DNA homologs are inserted into the Sac I and Xho I cloning sites. The amino acid residue sequence of the Pel B leader sequence/spacer is listed in SEQ ID NO 7.

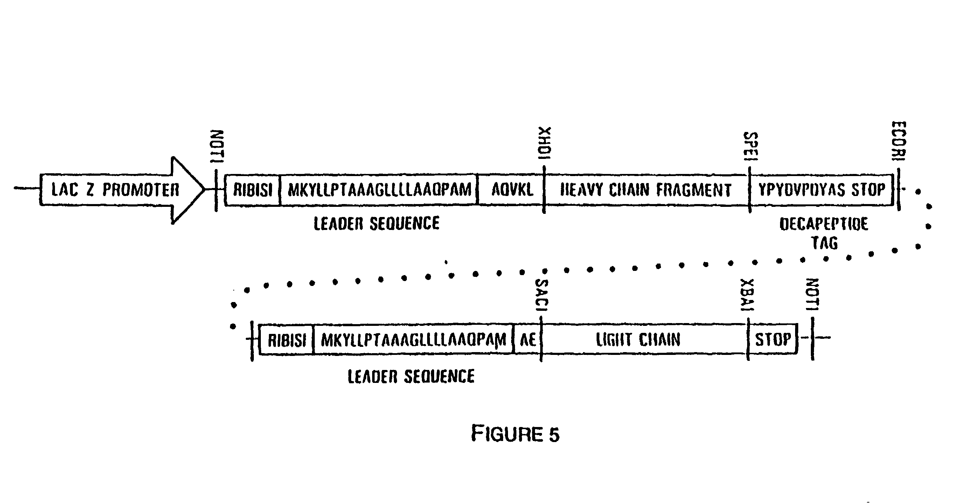

Figure 5 illustrates the dicistronic expression vector, pComb, in the form of a phagemid expression vector.

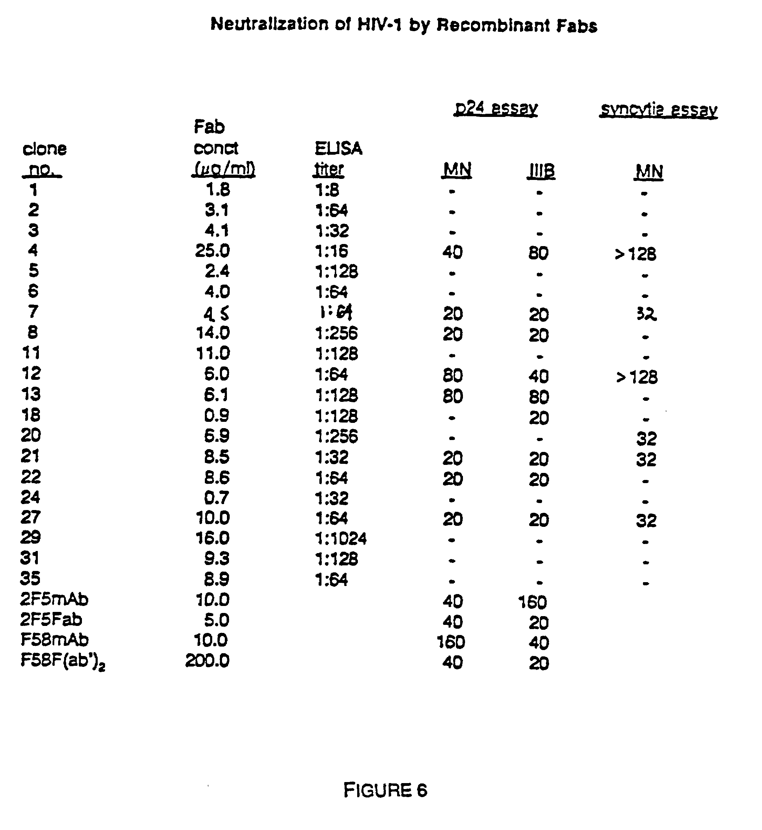

Figure 6 illustrates the neutralization of HIV-1 by recombinant Fabs. The same supernate preparations were used in p24 and syncytia assays. The figures indicate neutralization titers. Refer to Example 3 for details of the assay procedures and discussion of the results. The ELISA titers and Fab concentrations were determined as described in Example 2b.

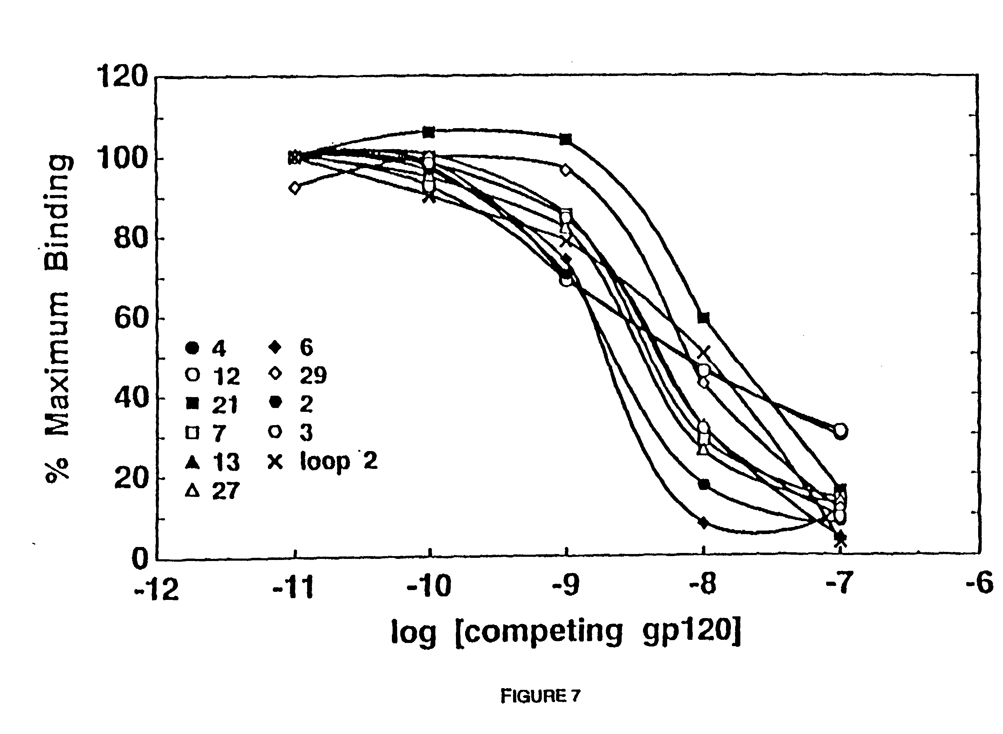

Figure 7 illustrates the relative affinities of Fab fragments for gp120 (IIIB) as illustrated by inhibition ELISA performed as described in Example 2b6). Fabs 27, 6, 29, 2 and 3 are all prototype members of the different groups discussed in Example 4. Loop 2 is an Fab fragment selected from the same library as the other Fabs but which recognizes the V3 loop. The data is plotted as the percentage of maximum binding on the Y-axis against increasing concentrations (10⁻¹¹ M to 10⁻⁷ M) of soluble gp120 on the X-axis.

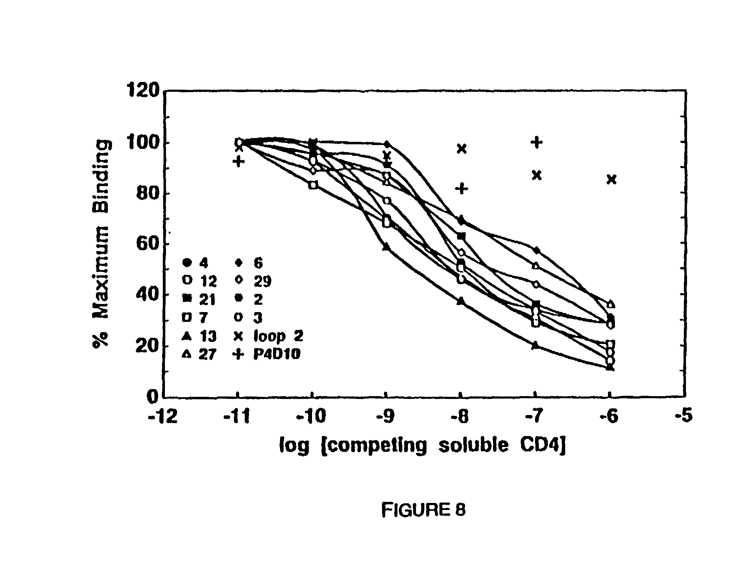

Figure 8 illustrates the soluble CD4 competition with Fab fragments for gp120 (IIIB). P4D10 and loop2 are controls. P4D10 is a mouse monoclonal antibody reacting with the V3 loop of gp120 (IIIB). The data, discussed in Example 2b6), is plotted as described in Figure 7.

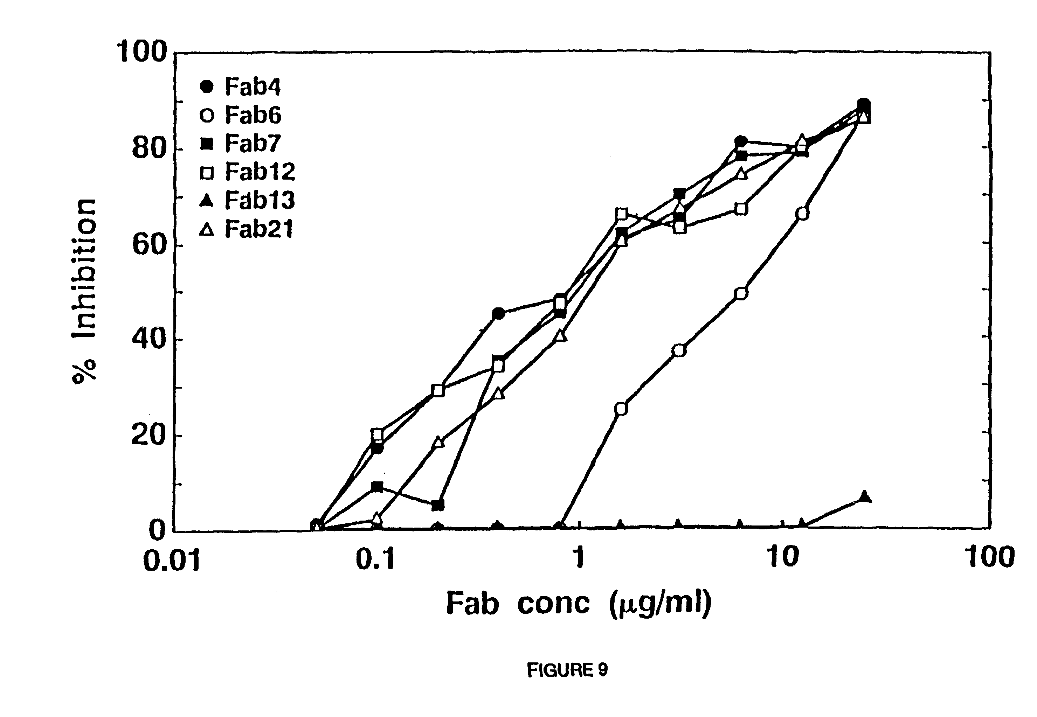

Figure 9 illustrates the neutralization of HIV by purified Fabs prepared as described in Example 3. The results shown are derived from the syncytia assay using the MN strain. The data is plotted as percent of inhibition of binding on the Y-axis against increasing Fab concentrations [0.1 to greater than 10 micrograms/milliliter (µg/ml)] on the X-axis.

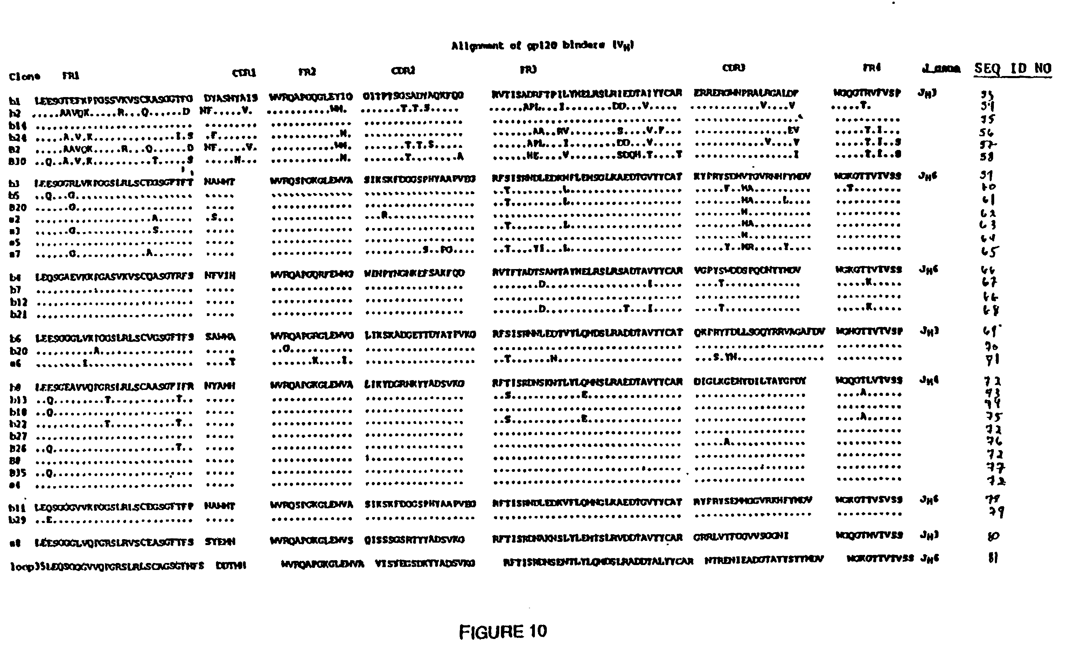

Figure 10 illustrates the amino acid residue sequences of variable heavy (V_{H}) domains of Fabs binding to gp120. Seven distinct groups have been identified as described in Example 4 based on sequence homology. Identity with the first sequence in a group is indicated by dots. The Fab clone names are indicated in the left hand column. The corresponding SEQ ID NOs are indicated in the right hand column. The sequenced regions from right to left are framework region 1 (FR1), complementary determining region 1 (CDR1), framework region 2 (FR2), complementary determining region 2 (CDR2), framework region 3 (FR3), complementary determining region 3 (CDR3), and framework region 4 (FR4). The five amino-terminal residue sequence beginning with LEQ arises from the VH1a while the 5 amino-terminal residue sequence beginning with LEE arises from the VH3a primers. The b11 and b29 sequences are very similar to the b3 group and could be argued to be intraclonal variants within that group; they are placed in their own group because of differences at the V-D and D-J interface.

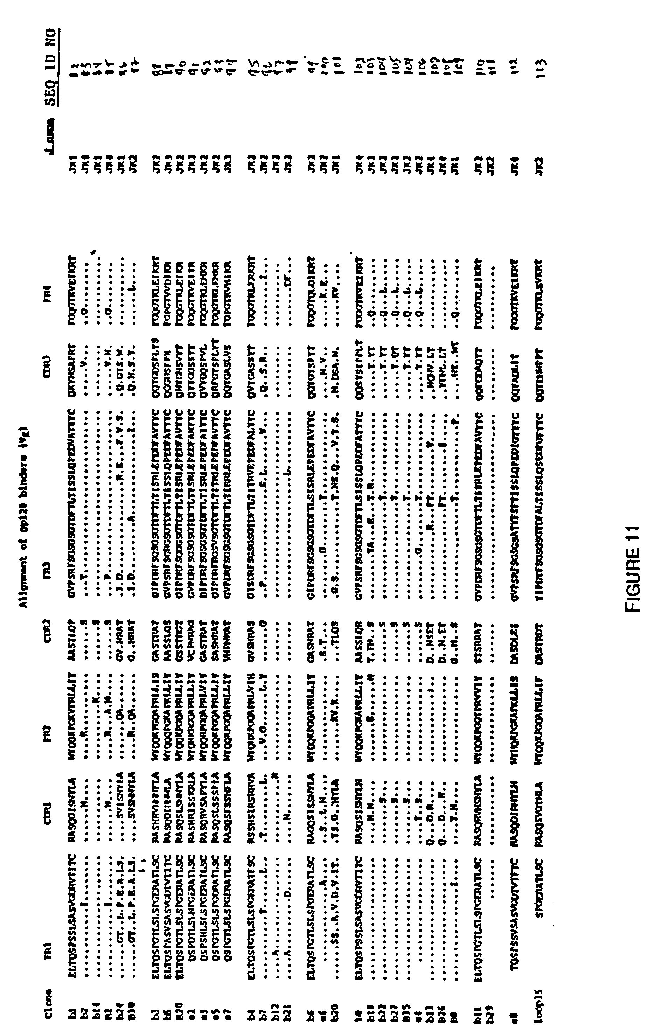

Figure 11 illustrates the amino acid residue sequences of variable light (V_{L}) domains of Fabs binding to gp120. Refer to Figure 10 for the description of the figure and to Example 4 for analysis of the sequences.

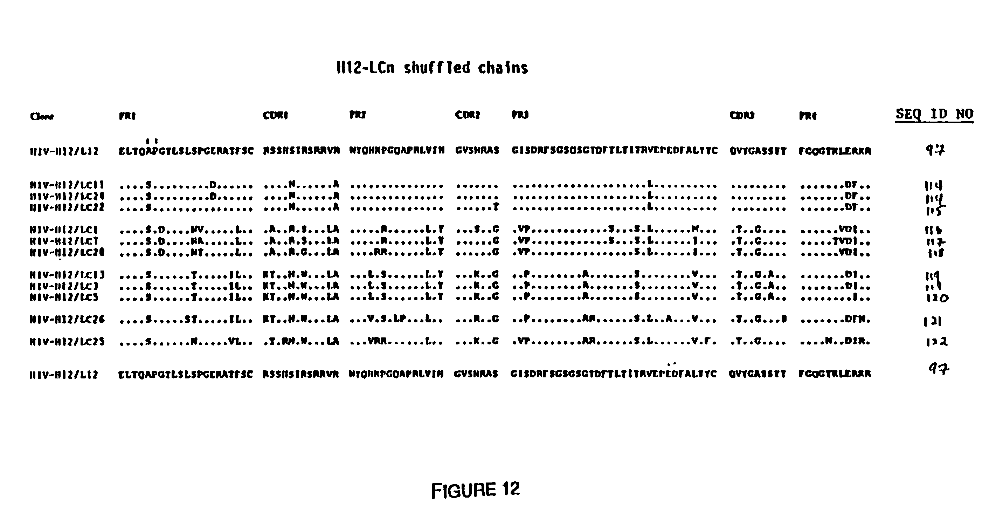

Figure 12 illustrates the amino acid residue sequences of V_{L} domains from Fabs binding to gp120 and generated by shuffling the heavy chain from clone b12 against a library of light chains (H12-LCn Fabs) as described in Example 5. Note that the new V_{L} sequences have designated clone numbers that do not relate to those numbers from the original library. The unique sequences are listed in the Sequence Listing from SEQ ID NO 114 to 122. The new V_{L} domain sequences are compared to that of the original clone b12 V_{L} sequence.

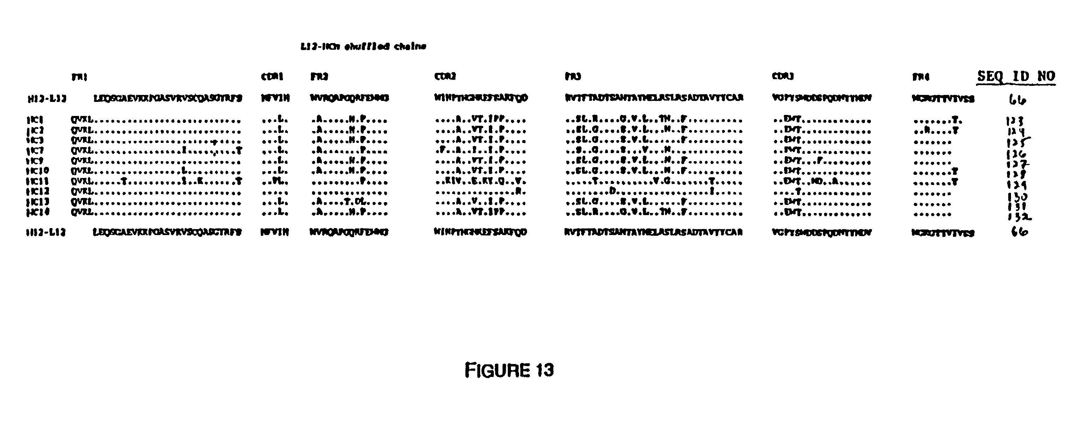

Figure 13 illustrates the amino acid residue sequences of V_{H} domains from Fabs binding to gp120 and generated by shuffling the light chain from clone b12 against a library of heavy chains (L12-HCn Fabs) as described in Example 5. Note that the new V_{H} sequences have designated clone numbers that do not relate to those numbers from the original library. The unique sequences are listed in the Sequence Listing from SEQ ID NO 123 to 132. The new V_{H} domain sequences are compared to that of the original clone b12 V_{H} sequence.

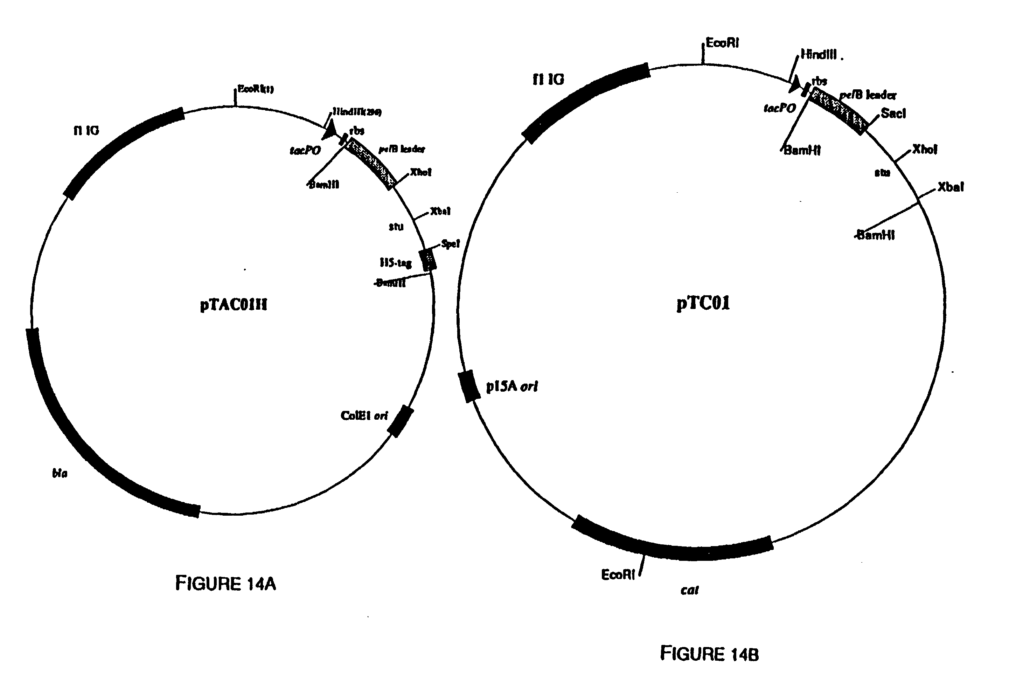

Figure 14 illustrates, in two figures, Figure 14A and 14B, plasmid maps of the heavy (pTAC01H) and light chain (pTC01) replicon-compatible chain-shuffling vectors, respectively. Both plasmids are very similar in the section containing the promoter and the cloning site. Abbreviations: tacPO, tac promoter/operon; 5 histidine amino acid residue tag (histidine)5-tail; f1IG, intergenic region of fl-phage; stu, stuffer fragment ready for in-frame replacement by light and heavy chain, respectively; cat, chloramphenicol transferase gene; bla, b-lactamase gene; ori, origin of replication. The map is drawn approximately to scale.

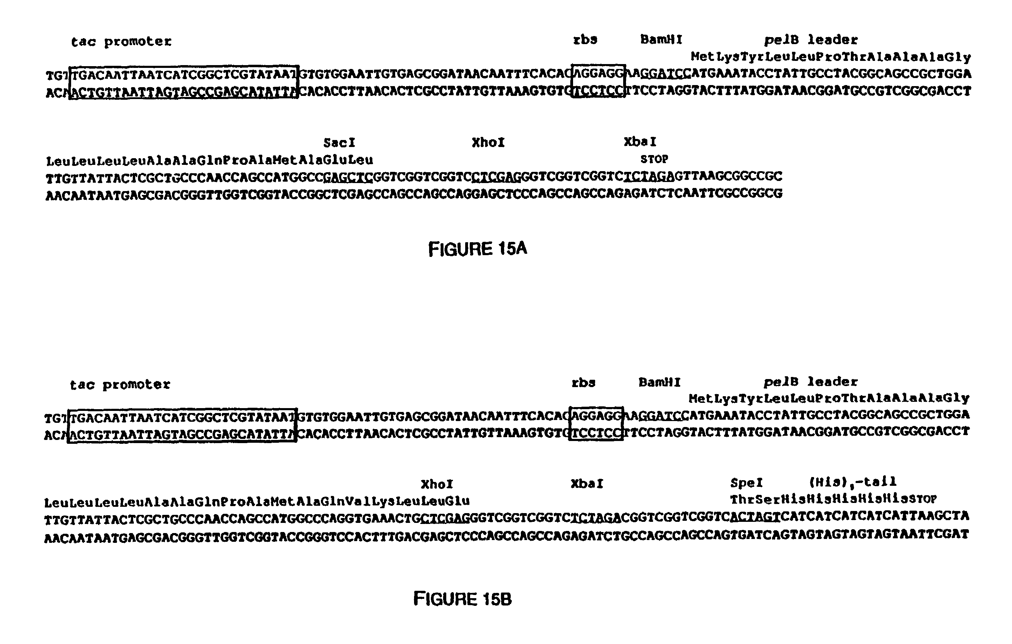

Figure 15 illustrates the nucleotide sequences of the binary shuffling vectors in two Figures, 15A and 15B. The construction and use of the vectors is described in Example 6. In Figure 15A, the double-stranded nucleotide sequence of the multiple cloning site in light chain vector, pTC01, is shown. The sequences of the top and bottom nucleotide base strands are listed respectively in SEQ ID NO 8 and SEQ ID NO 9. The amino acid residue sequence comprising the pelB leader ending in the Sac I restriction site is listed in SEQ ID NO 10. In Figure 15B, the nucleotide sequence of the multiple cloning site in heavy chain vector, pTAC01H, is shown. The sequences of the top and bottom nucleotide base strands are listed respectively in SEQ ID NO 11 and SEQ ID NO 12. The amino acid residue sequence comprising the pelB leader ending in the Xho I restriction site is listed as SEQ ID NO 13. The amino acid residue sequence comprising the histidine tail is listed in SEQ ID NO 14. Relevant restriction sites are underlined. tac promoter and ribosome binding site (rbs) are indicated by boxes.

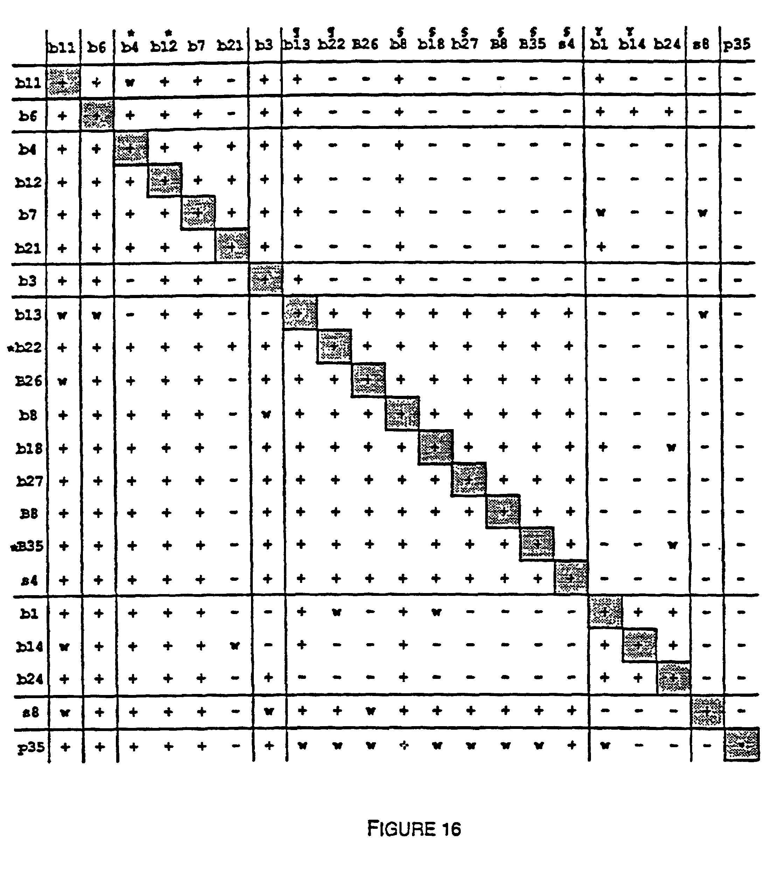

Figure 16 illustrates the complete set of directed crosses between heavy and light chains of all Fab fragments isolated from the original library by panning with gp160 (IIIB) (bl-b27), gp120 (IIIB) (B8-B35), gp120 (SF2) (s4-s8), and the loop peptide (p35) assayed by ELISA against IIIB gp120 as described in Example 6. Heavy chains are listed horizontally and light chains are listed vertically. Clones are sorted according to the grouping established in Example 4. Different groups are separated by horizontal and vertical lines. A "-" at the intersection of a particular heavy chain and light chain signifies a clear negative (a signal of 3 times background or less) for that particular cross, a "+" shows a clear positive comparable to the original heavy and light chain combination, and a "w" denotes an intermediate value in the ELISA. "•": the HCp35/LCp35 combination is negative when gp120 (IIIB) is used, but positive when assayed with gp120 (IIIB). Identical chains carry the same identifier (either *, ¶, §, or ¥).

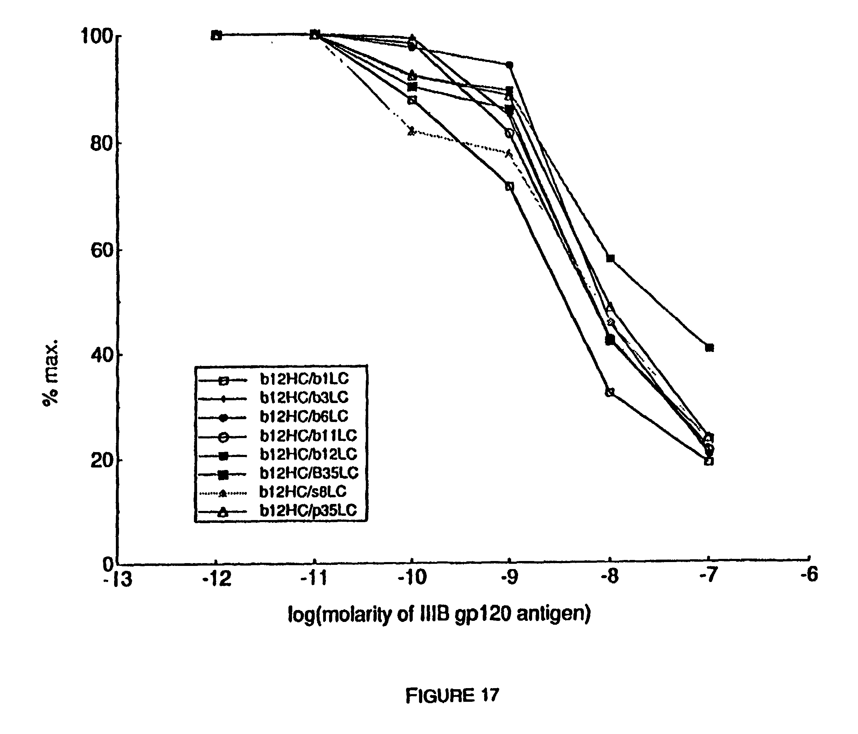

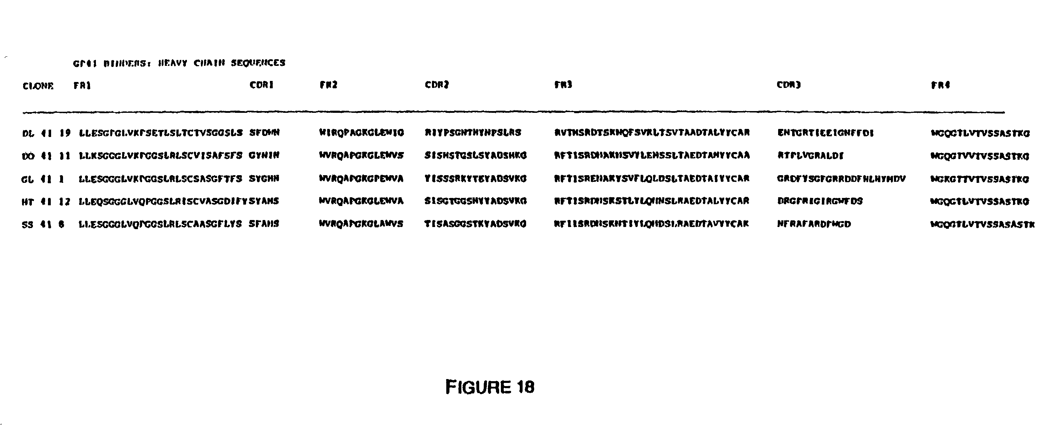

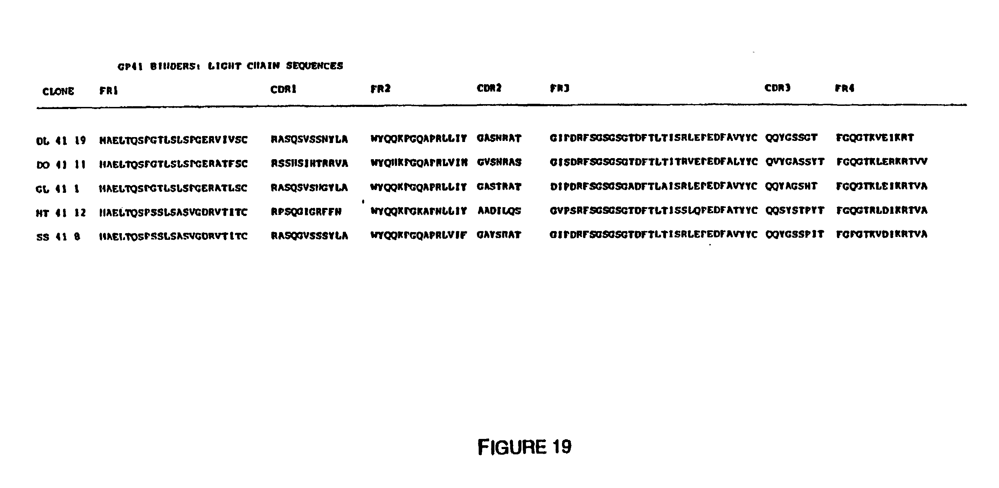

Figure 17 illustrates the affinity of antibody-antigen interaction for b12 heavy chain crosses with light chains from all pannings analyzed by competitive ELISA using soluble IIIB gp120 as competing antigen as described in Example 6. The data is plotted as the percentage of maximum binding on the Y-axis against increasing concentrations of soluble gp120 (IIIB) (10⁻¹² M to 10⁻⁷ M) on the X-axis.

### Detailed Description of the Invention

### A. Definitions

Amino Acid Residue: An amino acid formed upon chemical digestion (hydrolysis) of a polypeptide at its peptide linkages. The amino acid residues described herein are preferably in the "L" isomeric form. However, residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxy terminus of a polypeptide. In keeping with standard polypeptide nomenclature (described in J. Biol. Chem., 243:3552-59 (1969) and adopted at 37 CFR §1.822(b)(2)), abbreviations for amino acid residues are shown in the following Table of Correspondence:

| TABLE OF CORRESPONDENCE | | |
|---|---|---|
| SYMBOL | | AMINO ACID |
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | lle | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| Z | Glx | Glu and/or Gln |
| W | Trp | tryptophan |
| R | Arg | arginine |
| D | Asp | aspartic acid |
| N | Asn | asparagine |
| B | Asx | Asn and/or Asp |
| C | Cys | cysteine |
| X | Xaa | Unknown or other |

It should be noted that all amino acid residue sequences represented herein by formulae have a left-to-right orientation in the conventional direction of amino terminus to carboxy terminus. In addition, the phrase "amino acid residue" is broadly defined to include the amino acids listed in the Table of Correspondence and modified and unusual amino acids, such as those listed in 37 CFR 1.822(b)(4), and incorporated herein by reference. Furthermore, it should be noted that a dash at the beginning or end of an amino acid residue sequence indicates a peptide bond to a further sequence of one or more amino acid residues or a covalent bond to an amino-terminal group such as NH₂ or acetyl or to a carboxy-terminal group such as COOH.

Recombinant DNA (rDNA) molecule: A DNA molecule produced by operatively linking two DNA segments. Thus, a recombinant DNA molecule is a hybrid DNA molecule comprising at least two nucleotide sequences not normally found together in nature. rDNA's not having a common biological origin, i.e., evolutionarily different, are said to be "heterologous".

Vector: A rDNA molecule capable of autonomous replication in a cell and to which a DNA segment, e.g., gene or polynucleotide, can be operatively linked so as to bring about replication of the attached segment. Vectors capable of directing the expression of genes encoding for one or more polypeptides are referred to herein as "expression vectors". Particularly important vectors allow cloning of cDNA (complementary DNA) from mRNAs produced using reverse transcriptase.

Receptor: A receptor is a molecule, such as a protein, glycoprotein and the like, that can specifically (non-randomly) bind to another molecule.

Antibody: The term antibody in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab')₂ and F(v).

Antibody Combining Site: An antibody combining site is that structural portion of an antibody molecule comprised of a heavy and light chain variable and hypervariable regions that specifically binds (immunoreacts with) an antigen. The term immunoreact in its various forms means specific binding between an antigenic determinant-containing molecule and a molecule containing an antibody combining site such as a whole antibody molecule or a portion thereof.

Monoclonal Antibody: A monoclonal antibody in its various grammatical forms refers to a population of antibody molecules that contain only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g., a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Fusion Polypeptide: A polypeptide comprised of at least two polypeptides and a linking sequence to operatively link the two polypeptides into one continuous polypeptide. The two polypeptides linked in a fusion polypeptide are typically derived from two independent sources, and therefore a fusion polypeptide comprises two linked polypeptides not normally found linked in nature.

Upstream: In the direction opposite to the direction of DNA transcription, and therefore going from 5' to 3' on the non-coding strand, or 3' to 5' on the mRNA.

Downstream: Further along a DNA sequence in the direction of sequence transcription or read out, that is traveling in a 3'- to 5'-direction along the non-coding strand of the DNA or 5'- to 3'-direction along the RNA transcript.

Cistron: Sequence of nucleotides in a DNA molecule coding for an amino acid residue sequence and including upstream and downstream DNA expression control elements.

Leader Polypeptide: A short length of amino acid sequence at the amino end of a polypeptide, which carries or directs the polypeptide through the inner membrane and so ensures its eventual secretion into the periplasmic space and perhaps beyond. The leader sequence peptide is commonly removed before the polypeptide becomes active.

Reading Frame: Particular sequence of contiguous nucleotide triplets (codons) employed in translation. The reading frame depends on the location of the translation initiation codon.

### B. Human Monoclonal Antibodies

The present invention relates to human monoclonal antibodies which are specific for, and neutralize human immunodeficiency virus (HIV). In the invention, human monoclonal antibodies are disclosed which are capable of binding epitopic polypeptide sequences in glycoprotein gp120 of HIV. Also disclosed is an antibody having a specified amino acid sequence, which sequence confers the ability to bind a specific epitope and to neutralize HIV when the virus is bound by these antibodies. A human monoclonal antibody with a claimed specificity, and like human nonocional antibodies with like specificity, are useful in the diagnosis and immunotherapy of HIV-induced disease.

The term "HIV-induced disease" means any disease caused, directly or indirectly, by HIV. An example of a HIV-induced disease is acquired autoimmunodeficiency syndrome (AIDS), and any of the numerous conditions associated generally with AIDS which are caused by HIV infection.

Thus, in one aspect, the present invention is directed to human monoclonal antibodies which are reactive with a HIV neutralization site and cell lines which produce such antibodies. The isolation of cell lines producing monoclonal antibodies of the invention is described in great detail further herein, and can be accomplished using the phagemid vector library methods described herein, and using routine screening techniques which permit determination of the elementary immunoreaction and neutralization patterns of the monoclonal antibody of interest. Thus, if a human monoclonal antibody being tested binds and neutralizes HIV in a manner similar to a human monoclonal antibody produced by the cell lines of the invention then the tested antibody is considered equivalent to an antibody of the invention.

It is also possible to determine, without undue experimentation, if a human monoclonal antibody has the same (i.e., equivalent) specificity as a human monoclonal antibody of this invention by ascertaining whether the former prevents the latter from binding to HIV. If the human monoclonal antibody being tested competes with the human monoclonal antibody of the invention, as shown by a decrease in binding by the human monoclonal antibody of the invention in standard competition assays for binding to a solid phase antigen, for example to gp120, then it is likely that the two monoclonal antibodies bind to the same, or a closely related, epitope.

Still another way to determine whether a human monoclonal antibody has the specificity of a human monoclonal antibody of the invention is to pre-incubate the human monoclonal antibody of the invention with HIV with which it is normally reactive, and then add the human monoclonal antibody being tested to determine if the human monoclonal antibody being tested is inhibited in its ability to bind HIV. If the human monoclonal antibody being tested is inhibited then, in all likelihood, it has the same, or functionally equivalent, epitopic specificity as the monoclonal antibody of the invention. Screening of human monoclonal antibodies of the invention, can be also carried out utilizing HIV neutralization assays and determining whether the monoclonal antibody neutralizes HIV.

The ability to neutralize HIV at one or more stages of virus infection is a desirable quality of a human monoclonal antibody of the present invention. Virus neutralization can be measured by a variety of in vitro and in vivo methodologies. Exemplary methods described herein for determining the capacity for neutralization are the in vitro assays that measure inhibition of HIV-induced syncytia formation, plaque assays and assays that measure the inhibition of output of core p24 antigen from a cell infected with HIV.

As shown herein, the immunospecificity of a human monoclonal antibody of this invention can be directed to epitopes that are shared across serotypes and/or strains of HIV, or can be specific for a single strain of HIV, depending upon the epitope. Thus, a preferred human monoclonal antibody can immunoreact with HIV-1, HIV-2, or both, and can immunoreact with one or more of the HIV-1 strains IIIB, MN, RF, SF-2, Z2, 26, CDC4, of the HIV-1 strains IIIB, MN, RF, SF-2, Z2, Z6, CDC4, ELI and the like strains.

The immunospecificity of an antibody, its HIV-neutralizing capacity, and the attendant affinity the antibody exhibits for the epitope, are defined by the epitope with which the antibody immunoreacts. The epitope specificity is defined at least in part by the amino acid residue sequence of the variable region of the heavy chain of the immunoglobulin the antibody, and in part by the light chain variable region amino acid residue sequence. Preferred human monoclonal antibodies immunoreact with the CD4 binding site of glycoprotein gp120.

A preferred human monoclonal antibody of this invention has the binding specificity of a monoclonal antibody comprising a heavy chain immunoglobulin variable region amino acid residue sequence selected from the group of sequences consisting of SEQ ID NOs 66, 67, 68, 70, 72, 73, 74, 75, 78 and 79, and conservative substitutions thereof.

Another preferred human monoclonal antibody of this invention has the binding specificity of a monoclonal antibody having a light chain immunoglobulin variable region amino acid residue sequence selected from the group of sequences consisting of SEQ ID NOs 95, 96, 97, 98, 101, 102, 103, 104, 105, 107, 110, 115, 118, 121, 122, 124 and 132, and conservative substitutions thereof.

As shown by the present teachings and using the combinatorial library shuffling and screening methods, one can identify new heavy and light chain pairs (H:L) that function as a HIV-neutralizing monoclonal antibody. In particular, one can shuffle a known heavy chain, derived from an HIV-neutralizing human monoclonal antibody, with a library of light chains to identify new H:L pairs that form a functional antibody according to the present invention. Similarly, one can shuffle a known light chain, derived from an HIV-neutralizing human monoclonal antibody, with a library of heavy chains to identify new H:L pairs that form a functional antibody according to the present invention.

Particularly preferred human monoclonal antibodies are those having the gp120 immunoreaction (binding) specificity of a monoclonal antibody having heavy and light chain immunoglobulin variable region amino acid residue sequences in pairs (H:L) selected from the group consisting of SEQ ID NOs 66:95, 67:96, 72:102, 66:97, 73:107, 74:103, 70:101, 68:98, 75:104, 72:105, 78:110, 66:118, 66:122, 66:121, 66:115, 79:124, 79:132 and 66:98, and conservative substitutions thereof. The designation of two SEQ ID NOs with a colon, e.g., 66:95, is to connote a H:L pair formed by the heavy and light chain, SEQ ID NO 66 and SEQ ID NO 95, respectively.

Particularly preferred are human monoclonal antibodies having the binding specificity of the monoclonal antibody produced by the E. coli microorganisms deposited with the ATCC, as described further herein.

Particularly preferred are human monoclonal antibodies having the binding specificity of the monoclonal antibodies produced by the E. coli microorganisms designated ATCC 69078, 69079 and 69080. By "having the binding specificity" is meant equivalent monoclonal antibodies which exhibit the same or similar immunoreaction and neutralization properties, and which compete for binding to an HIV antigen. Preferred are the human monoclonal antibodies produced by ATCC 69078, 69079 and 69080.

The term "conservative variation" as used herein denotes the replacement of an amino acid residue by another, biologically similar residue. Examples of conservative variations include the substitution of one hydrophobic residue such as isoleucine, valine, leucine or methionine for another, or the substitution of one polar residue for another, such as the substitution of arginine for lysine, glutamic for aspartic acids, or glutamine for asparagine, and the like. The term "conservative variation" also includes the use of a substituted amino acid in place of an unsubstituted parent amino acid provided that antibodies having the substituted polypeptide also neutralize HIV. Analogously, another preferred embodiment of the invention relates to polynucleotides which encode the above noted heavy and/or light chain polypeptides and to polynucleotide sequences which are complementary to these polynucleotide sequences. Complementary polynucleotide sequences include those sequences which hybridize to the polynucleotide sequences of the invention under stringent hybridization conditions.

By using the human monoclonal antibodies of the invention, it is now possible to produce anti-idiotypic antibodies which can be used to screen human monoclonal antibodies to identify whether the antibody has the same binding specificity as a human monoclonal antibody of the invention and also used for active immunization (Herlyn et al., Science, 232:100 (1986)). Such anti-idiotypic antibodies can be produced using well-known hybridoma techniques (Kohler et al., Nature, 256:495 (1975)). An anti-idiotypic antibody is an antibody which recognizes unique determinants present on the human monoclonal antibody produced by the cell line of interest. These determinants are located in the hypervariable region of the antibody. It is this region which binds to a given epitope and, thus, is responsible for the specificity of the antibody. An anti-idiotypic antibody can be prepared by immunizing an animal with the monoclonal antibody of interest. The immunized animal will recognize and respond to the idiotypic determinants of the immunizing antibody and produce an antibody to these idiotypic determinants. By using the anti-idiotypic antibodies of the immunized animal, which are specific for the human monoclonal antibody of the invention produced by a cell line which was used to immunize the second animal, it is now possible to identify other clones with the same idiotype as the antibody of the hybridoma used for immunization. Idiotypic identity between human monoclonal antibodies of two cell lines demonstrates that the two monoclonal antibodies are the same with respect to their recognition of the same epitopic determinant. Thus, by using anti-idiotypic antibodies, it is possible to identify other hybridomas expressing monoclonal antibodies having the same epitopic specificity.

It is also possible to use the anti-idiotype technology to produce monoclonal antibodies which mimic an epitope. For example, an anti-idiotypic monoclonal antibody made to a first monoclonal antibody will have a binding domain in the hypervariable region which is the "image" of the epitope bound by the first monoclonal antibody. Thus, the anti-idiotypic monoclonal antibody can be used for immunization, since the anti-idiotype monoclonal antibody binding domain effectively acts as an antigen.

In one preferred embodiment, the invention contemplates a truncated immunoglobulin molecule comprising a Fab fragment derived from a human monoclonal antibody of this invention. The Fab fragment, lacking Fc receptor, is soluble, and affords therapeutic advantages in serum half life, and diagnostic advantages in modes of using the soluble Fab fragment. The preparation of a soluble Fab fragment is generally known in the immunological arts and can be accomplished by a variety of methods. A preferred method of producing a soluble Fab fragment is described herein.

### C. Immunotherapeutic Methods and Compositions

The human monoclonal antibodies can also be used immunotherapeutically for HIV disease. The term "immunotherapeutically" or "immunotherapy" as used herein in conjunction with the monoclonal antibodies of the invention denotes both prophylactic as well as therapeutic administration. Thus, the monoclonal antibodies can be administered to high-risk patients in order to lessen the likelihood and/or severity of HIV-induced disease, administered to patients already evidencing active HIV infection, or administered to patients at risk of HIV infection.

### 1. Therapeutic Compositions

The present invention therefore contemplates therapeutic compositions useful for practicing the therapeutic methods described herein. Therapeutic compositions of the present invention contain a physiologically tolerable carrier together with at least one species of human monoclonal antibody as described herein, dissolved or dispersed therein as an active ingredient. In a preferred embodiment, the therapeutic composition is not immunogenic when administered to a human patient for therapeutic purposes, unless that purpose is to induce an immune response, as described elsewhere herein.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a human without the production of undesirable physiological effects such as nausea, dizziness, gastric upset and the like.

The preparation of a pharmacological composition that contains active ingredients dissolved or dispersed therein is well understood in the art. Typically such compositions are prepared as sterile injectables either as liquid solutions or suspensions, aqueous or non-aqueous, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The therapeutic composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes.

Liquid compositions can also contain liquid phases in addition to and to the exclusion of water. Exemplary of such additional liquid phases are glycerin, vegetable oils such as cottonseed oil, organic esters such as ethyl oleate, and water-oil emulsions.

A therapeutic composition contains an HIV-neutralizing of a human monoclonal antibody of the present invention, typically an amount of at least 0.1 weight percent of antibody per weight of total therapeutic composition. A weight percent is a ratio by weight of antibody to total composition. Thus, for example, 0.1 weight percent is 0.1 grams of antibody per 100 grams of total composition.

### 2. Therapeutic Methods

In view of the demonstrated HIV neutralizing ability of the human monoclonal antibodies of the present invention, the present disclosure provides for a method for neutralizing HIV in vitro or in vivo. The method comprises contacting a sample believed to contain HIV with a composition comprising a therapeutically effective amount of a human monoclonal antibody of this invention.

For in vivo modalities, the method comprises administering to the patient a therapeutically effective amount of a physiologically tolerable composition containing a human monoclonal antibody of the invention. Thus, the present invention describes in one embodiment a method for providing passive immunotherapy to HIV disease in a human comprising administering to the human an immunotherapeutically effective amount of the monoclonal antibody of this invention.

A representative patient for practicing the present passive immunotherapeutic methods is any human exhibiting symptoms of HIV-induced disease, including AIDS or related conditions believed to be caused by HIV infection, and humans at risk of HIV infection. Patients at risk of infection by HIV include babies of HIV-infected pregnant mothers, recipients of transfusions known to contain HIV, users of HIV contaminated needles, individuals who have participated in high risk sexual activities with known HIV-infected individuals, and the like risk situations.

In one embodiment, the passive immunization method comprises administering a composition comprising more than one species of human monoclonal antibody of this invention, preferably directed to non-competing epitopes or directed to distinct serotypes or strains of HIV, as to afford increased effectiveness of the passive immunotherapy.

A therapeutically (immunotherapeutically) effective amount of a human monoclonal antibody is a predetermined amount calculated to achieve the desired effect, i.e., to neutralize the HIV present in the sample or in the patient, and thereby decrease the amount of detectable HIV in the sample or patient. In the case of in vivo therapies, an effective amount can be measured by improvements in one or more symptoms associated with HIV-induced disease occurring in the patient, or by serological decreases in HIV antigens.

Thus, the dosage ranges for the administration of the monoclonal antibodies of the invention are those large enough to produce the desired effect in which the symptoms of the HIV disease are ameliorated or the likelihood of infection decreased. The dosage should not be so large as to cause adverse side effects, such as hyperviscosity syndromes, pulmonary edema, congestive heart failure, and the like. Generally, the dosage will vary with the age, condition, sex and extent of the disease in the patient and can be determined by one of skill in the art.

The dosage can be adjusted by the individual physician in the event of any complication.

A therapeutically effective amount of an antibody of this invention is typically an amount of antibody such that when administered in a physiologically tolerable composition is sufficient to achieve a plasma concentration of from about 0.1 microgram (ug) per milliliter (ml) to about 100 ug/ml, preferably from about 1 ug/ml to about 5 ug/ml, and usually about 5 ug/ml. Stated differently, the dosage can vary from about 0.1 mg/kg to about 300 mg/kg, preferably from about 0.2 mg/kg to about 200 mg/kg, most preferably from about 0.5 mg/kg to about 20 mg/kg, in one or more dose administrations daily, for one or several days.

The human monoclonal antibodies of the invention can be administered parenterally by injection or by gradual infusion over time. Although the HIV infection is typically systemic and therefore most often treated by intravenous administration of therapeutic compositions, other tissues and delivery means are contemplated where there is a likelihood that the tissue targeted contains infectious HIV. Thus, human monoclonal antibodies of the invention can be administered intravenously, intraperitoneally, intramuscularly, subcutaneously, intracavity, transdermally, and can be delivered by peristaltic means.

The therapeutic compositions containing a human monoclonal antibody of this invention are conventionally administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; i.e., carrier, or vehicle.

The compositions are administered in a manner compatible with the dosage formulation, and in a therapeutically effective amount. The quantity to be administered depends on the subject to be treated, capacity of the subject's system to utilize the active ingredient, and degree of therapeutic effect desired. Precise amounts of active ingredient required to be administered depend on the judgement of the practitioner and are peculiar to each individual. However, suitable dosage ranges for systemic application are disclosed herein and depend on the route of administration. Suitable regimes for administration are also variable, but are typified by an initial administration followed by repeated doses at one or more hour intervals by a subsequent injection or other administration. Alternatively, continuous intravenous infusion sufficient to maintain concentrations in the blood in the ranges specified for in vivo therapies are contemplated.

As an aid to the administration of effective amounts of a monoclonal antibody, a diagnostic method for detecting a monoclonal antibody in the subject's blood is useful to characterize the fate of the administered therapeutic composition.

The invention also relates to a method for preparing a medicament or pharmaceutical composition comprising the human monoclonal antibodies of the invention, the medicament being used for immunotherapy of HIV disease.

### D. Diagnostic Assay Methods

The present invention contemplates various assay methods for determining the presence, and preferably amount, of HIV in a sample such as a biological fluid or tissue sample using a human monoclonal antibody of this invention as an immunochemical reagent to form an immunoreaction product whose amount relates, either directly or indirectly, to the amount of HIV in the sample.

Those skilled in the art will understand that there are numerous well known clinical diagnostic chemistry procedures in which an immunochemical reagent of this invention can be used to form an immunoreaction product whose amount relates to the amount of HIV present in a body sample. Thus, while exemplary assay methods are described herein, the invention is not so limited.

Various heterogenous and homogeneous protocols, either competitive or noncompetitive, can be employed in performing an assay method of this invention. Examples of types of immunoassays which can utilize monoclonal antibodies of the invention are competitive and non-competitive immunoassays in either a direct or indirect format. Examples of such immunoassays are the radioimmunoassay (RIA) and the sandwich (immunometric) assay. Detection of the antigens using the monoclonal antibodies of the invention can be done utilizing immunoassays which are run in either the forward, reverse, or simultaneous modes, including immunohistochemical assays on physiological samples. Those of skill in the art will know, or can readily discern, other immunoassay formats without undue experimentation.

The monoclonal antibodies of the invention can be bound to many different carriers and used to detect the presence of HIV. Examples of well-known carriers include glass, polystyrene, polypropylene, polyethylene, dextran, nylon, amylases, natural and modified celluloses, polyacrylamides, agaroses and magnetite. The nature of the carrier can be either soluble or insoluble for purposes of the invention. Those skilled in the art will know of other suitable carriers for binding monoclonal antibodies, or will be able to ascertain such, using routine experimentation.

There are many different labels and methods of labeling known to those of ordinary skill in the art. Examples of the types of labels which can be used in the present invention include enzymes, radioisotopes, fluorescent compounds, colloidal metals, chemiluminescent compounds, and bio-luminescent compounds. Those of ordinary skill in the art will know of other suitable labels for binding to the monoclonal antibodies of the invention, or will be able to ascertain such, using routine experimentation. Furthermore, the binding of these labels to the monoclonal antibodies of the invention can be done using standard techniques common to those of ordinary skill in the art.

For purposes of the invention, HIV may be detected by the monoclonal antibodies of the invention when present in samples of biological fluids and tissues. Any sample containing a detectable amount of HIV can be used. A sample can be a liquid such as urine, saliva, cerebrospinal fluid, blood, serum and the like, or a solid or semi-solid such as tissues, feces, and the like, or, alternatively, a solid tissue such as those commonly used in histological diagnosis.

Another labeling technique which may result in greater sensitivity consists of coupling the antibodies to low molecular weight haptens. These haptens can then be specifically detected by means of a second reaction. For example, it is common to use haptens such as biotin, which reacts with avidin, or dinitrophenol, pyridoxal, or fluorescein, which can react with specific anti-hapten antibodies.

The monoclonal antibodies of the invention are suited for use in vitro, for example, in immunoassays in which they can be utilized in liquid phase or bound to a solid phase carrier for the detection of HIV in samples, as described above. The monoclonal antibodies in these immunoassays can be detectably labeled in various ways for in vitro use.

In using the human monoclonal antibodies of the invention for the in vivo detection of antigen, the detectably labeled human monoclonal antibody is given in a dose which is diagnostically effective. The term "diagnostically effective" means that the amount of detectably labeled human monoclonal antibody is administered in sufficient quantity to enable detection of the site having the HIV antigen for which the monoclonal antibodies are specific.

The concentration of detectably labeled human monoclonal antibody which is administered should be sufficient such that the binding to HIV is detectable compared to the background. Further, it is desirable that the detectably labeled monoclonal antibody be rapidly cleared from the circulatory system in order to give the best target-to-background signal ratio.

As a rule, the dosage of detectably labeled human monoclonal antibody for in vivo diagnosis will vary depending on such factors as age, sex, and extent of disease of the individual. The dosage of human monoclonal antibody can vary from about 0.01 mg/m² to about 500 mg/m², preferably 0.1 mg/m² to about 200 mg/m², most preferably about 0.1 mg/m², to about 10 mg/m². Such dosages may vary, for example, depending on whether multiple injections are given, tissue, and other factors known to those of skill in the art.

For in vivo diagnostic imaging, the type of detection instrument available is a major factor in selecting a given radioisotope. The radioisotope chosen must have a type of decay which is detectable for a given type of instrument. Still another important factor in selecting a radioisotope for in vivo diagnosis is that the half-life of the radioisotope be long enough so that it is still detectable at the time of maximum uptake by the target, but short enough so that deleterious radiation with respect to the host is minimized. Ideally, a radioisotope used for in vivo imaging will lack a particle emission, but produce a large number of photons in the 140-250 keV range, which may be readily detected by conventional gamma cameras.

For in vivo diagnosis radioisotopes may be bound to immunoglobulin either directly or indirectly by using an intermediate functional group. Intermediate functional groups which often are used to bind radioisotopes which exist as metallic ions to immunoglobulins are the bifunctional chelating agents such as diethylenetriaminepentacetic acid (DTPA) and ethylenediaminetetraacetic acid (EDTA) and similar molecules. Typical examples of metallic ions which can be bound to the monoclonal antibodies of the invention are ¹¹¹In, ⁹⁷Ru, ⁶⁷Ga, ⁶⁸Ga, ⁷²As, ⁸⁹Zr, and ²⁰¹Tl.

The monoclonal antibodies of the invention can also be labeled with a paramagnetic isotope for purposes of in vivo diagnosis, as in magnetic resonance imaging (MRI) or electron spin resonance (ESR). In general, any conventional method for visualizing diagnostic imaging can be utilized. Usually gamma and positron emitting radioisotopes are used for camera imaging and paramagnetic isotopes for MRI. Elements which are particularly useful in such techniques include ¹⁵⁷Gd, ⁵⁵Mn, ¹⁶²Dy, ⁵²Cr, and ⁵⁶Fe.

The human monoclonal antibodies of the invention can be used in vitro and in vivo to monitor the course of HIV disease therapy. Thus, for example, by measuring the increase or decrease in the number of cells infected with HIV or changes in the concentration of HIV present in the body or in various body fluids, it would be possible to determine whether a particular therapeutic regimen aimed at ameliorating the HIV disease is effective.

### E. Diagnostic Systems

The present invention also describes a diagnostic system, preferably in kit form, for assaying for the presence of HIV in a sample according to the diagnostic methods described herein. A diagnostic system includes, in an amount sufficient to perform at least one assay, a subject human monoclonal antibody, as a separately packaged reagent.

In another embodiment, a diagnostic system is contemplated for assaying for the presence of an anti-HIV monoclonal antibody in a body fluid sample such as for monitoring the fate of therapeutically administered antibody. The system includes, in an amount sufficient for at least one assay, a subject antibody as a control reagent, and preferably a preselected amount of HIV antigen, each as separately packaged immunochemical reagents.

Instructions for use of the packaged reagent are also typically included.

"Instructions for use" typically include a tangible expression describing the reagent concentration or at least one assay method parameter such as the relative amounts of reagent and sample to be admixed, maintenance time periods for reagent/sample admixtures, temperature, buffer conditions and the like.

In embodiments for detecting HIV in a body fluid, a diagnostic system of the present invention can include a label or indicating means capable of signaling the formation of an immunocomplex containing a human monoclonal antibody of the present invention.

The word "complex" as used herein refers to the product of a specific binding reaction such as an antibody-antigen reaction. Exemplary complexes are immunoreaction products.

As used herein, the terms "label" and "indicating means" in their various grammatical forms refer to single atoms and molecules that are either directly or indirectly involved in the production of a detectable signal to indicate the presence of a complex. Any label or indicating means can be linked to or incorporated in an expressed protein, polypeptide, or antibody molecule that is part of an antibody or monoclonal antibody composition of the present invention, or used separately, and those atoms or molecules can be used alone or in conjunction with additional reagents. Such labels are themselves well-known in clinical diagnostic chemistry and constitute a part of this invention only insofar as they are utilized with otherwise novel proteins methods and/or systems.

The labeling means can be a fluorescent labeling agent that chemically binds to antibodies or antigens without denaturing them to form a fluorochrome (dye) that is a useful immunofluorescent tracer. Suitable fluorescent labeling agents are fluorochromes such as fluorescein isocyanate (FIC), fluorescein isothiocyanate (FITC), 5-dimethylamine-1-naphthalenesulfonyl chloride (DANSC), tetramethylrhodamine isothiocyanate (TRITC), lissamine, rhodamine 8200 sulphonyl chloride (RB 200 SC) and the like. A description of immunofluorescence analysis techniques is found in DeLuca, "Immunofluorescence Analysis", in Antibody As a Tool, Marchalonis et al., eds., John Wiley & Sons, Ltd., pp. 189-231 (1982), which is incorporated herein by reference.

In preferred embodiments, the indicating group is an enzyme, such as horseradish peroxidase (HRP), glucose oxidase, or the like. In such cases where the principal indicating group is an enzyme such as HRP or glucose oxidase, additional reagents are required to visualize the fact that a receptor-ligand complex (immunoreactant) has formed. Such additional reagents for HRP include hydrogen peroxide and an oxidation dye precursor such as diaminobenzidine. An additional reagent useful with glucose oxidase is 2,2'-amino-di-(3-ethyl-benzthiazoline-G-sulfonic acid) (ABTS).

Radioactive elements are also useful labeling agents and are used illustratively herein. An exemplary radiolabeling agent is a radioactive element that produces gamma ray emissions. Elements which themselves emit gamma rays, such as ¹²⁴I, ¹²⁵I, ¹²⁸I, ¹³²I and ⁵¹Cr represent one class of gamma ray emission-producing radioactive element indicating groups. Particularly preferred is ¹²⁵I. Another group of useful labeling means are those elements such as ¹¹C, ¹⁸F, ¹⁵O and ¹³N which themselves emit positrons. The positrons so emitted produce gamma rays upon encounters with electrons present in the animal's body. Also useful is a beta emitter, such ¹¹¹ indium of ³H.

The linking of labels, i.e., labeling of, polypeptides and proteins is well known in the art. For instance, antibody molecules produced by a hybridoma can be labeled by metabolic incorporation of radioisotope-containing amino acids provided as a component in the culture medium. See, for example, Galfre et al., Meth. Enzymol., 73:3-46 (1981). The techniques of protein conjugation or coupling through activated functional groups are particularly applicable. See, for example, Aurameas et al., Scand. J. Immunol., Vol. 8 Suppl. 7:7-23 (1978), Rodwell et al., Biotech., 3:889-894 (1984), and U.S. Pat. No. 4,493,795.

The diagnostic systems can also include, preferably as a separate package, a specific binding agent. A "specific binding agent" is a molecular entity capable of selectively binding a reagent species of the present invention or a complex containing such a species, but is not itself a polypeptide or antibody molecule composition of the present invention. Exemplary specific binding agents are second antibody molecules, complement proteins or fragments thereof, S. aureus protein A, and the like. Preferably the specific binding agent binds the reagent species when that species is present as part of a complex.

In preferred embodiments, the specific binding agent is labeled. However, when the diagnostic system includes a specific binding agent that is not labeled, the agent is typically used as an amplifying means or reagent. In these embodiments, the labeled specific binding agent is capable of specifically binding the amplifying means when the amplifying means is bound to a reagent species-containing complex.

The diagnostic kits of the present invention can be used in an "ELISA" format to detect the quantity of an APC inhibitor of this invention in a vascular fluid sample such as blood, serum, or plasma. "ELISA" refers to an enzyme-linked immunosorbent assay that employs an antibody or antigen bound to a solid phase and an enzyme-antigen or enzyme-antibody conjugate to detect and quantify the amount of an antigen present in a sample. A description of the ELISA technique is found in Chapter 22 of the 4th Edition of Basic and Clinical Immunology by D.P. Sites et al., published by Lange Medical Publications of Los Altos, CA in 1982 and in U.S. Patents No. 3,654,090; No. 3,850,752; and No. 4,016,043, which are all incorporated herein by reference.

Thus, in some embodiments, a human monoclonal antibody of the present invention can be affixed to a solid matrix to form a solid support that comprises a package in the subject diagnostic systems.

A reagent is typically affixed to a solid matrix by adsorption from an aqueous medium although other modes of affixation applicable to proteins and polypeptides well known to those skilled in the art, can be used.

Useful solid matrices are also well known in the art. Such materials are water insoluble and include the cross-linked dextran available under the trademark SEPHADEX from Pharmacia Fine Chemicals (Piscataway, NJ); agarose; beads of polystyrene beads about 1 micron to about 5 millimeters in diameter available from Abbott Laboratories of North Chicago, IL; polyvinyl chloride, polystyrene, cross-linked polyacrylamide, nitrocellulose- or nylon-based webs such as sheets, strips or paddles; or tubes, plates or the wells of a microtiter plate such as those made from polystyrene or polyvinylchloride.

The reagent species, labeled specific binding agent or amplifying reagent of any diagnostic system described herein can be provided in solution, as a liquid dispersion or as a substantially dry power, e.g., in lyophilized form. Where the indicating means is an enzyme, the enzyme's substrate can also be provided in a separate package of a system. A solid support such as the before-described microtiter plate and one or more buffers can also be included as separately packaged elements in this diagnostic assay system.

The packaging materials discussed herein in relation to diagnostic systems are those customarily utilized in diagnostic systems.

The term "package" refers to a solid matrix or material such as glass, plastic (e.g., polyethylene, polypropylene and polycarbonate), paper, foil and the like capable of holding within fixed limits a diagnostic reagent such as a monoclonal antibody of the present invention. Thus, for example, a package can be a bottle, vial, plastic and plastic-foil laminated envelope or the like container used to contain a contemplated diagnostic reagent or it can be a microtiter plate well to which microgram quantities of a contemplated diagnostic reagent have been operatively affixed, i.e., linked so as to be capable of being immunologically bound by an antibody or polypeptide to be detected.

The materials for use in the assay of the invention are ideally suited for the preparation of a kit. Such a kit may comprise a carrier means being compartmentalized to receive in close confinement one or more container means such as vials, tubes, and the like, each of the container means comprising one of the separate elements to be used in the method. For example, one of the container means may comprise a human monoclonal antibody of the invention which is, or can be, detectably labelled. The kit may also have containers containing any of the other above-recited immunochemical reagents used to practice the diagnostic methods.

### F. Methods for Producing an HIV-Neutralizing Human Monoclonal Antibody

The present invention describes methods for producing novel HIV-neutralizing human monoclonal antibodies. The methods are based generally on the use of combinatorial libraries of antibody molecules which can be produced from a variety of sources, and include naive libraries, modified libraries, and libraries produced directly from human donors exhibiting an HIV-specific immune response.

The combinatorial library production and manipulation methods have been extensively described in the literature, and will not be reviewed in detail herein, except for those feature required to make and use unique embodiments of the present invention. However, the methods generally involve the use of a filamentous phage (phagemid) surface expression vector system for cloning and expressing antibody species of the library. Various phagemid cloning systems to produce combinatorial libraries have been described by others. See, for example the preparation of combinatorial antibody libraries on phagemids as described by Kang et al., Proc. Natl. Acad. Sci., USA, 88:4363-4366 (1991); Barbas et al., Proc. Natl. Acad. Sci., USA, 88:7978-7982 (1991); Zebedee et al., Proc. Natl. Acad. Sci., USA, 89:3175-3179 (1992); Kang et al., Proc. Natl. Acad. Sci., USA, 88:11120-11123 (1991); Barbas et al., Proc. Natl. Acad. Sci., USA, 89:4457-4461 (1992); and Gram et al., Proc. Natl. Acad. Sci., USA, 89:3576-3580 (1992), which references are hereby incorporated by reference.

In one embodiment, the method involves preparing a phagemid library of human monoclonal antibodies by using donor immune cell messenger RNA from HIV-infected donors. The donors can be symptomatic of AIDS, but in preferred embodiments the donor is asymptomatic, as the resulting library contains a substantially higher number of HIV-neutralizing human monoclonal antibodies.

In another embodiment, the donor is naive relative to an immune response to HIV, i.e., the donor is not HIV-infected. Alternatively, the library can be synthetic, or can be derived from a donor who has an immune response to other antigens.

The method for producing a human monoclonal antibody generally involves (1) preparing separate H and L chain-encoding gene libraries in cloning vectors using human immunoglobulin genes as a source for the libraries, (2) combining the H and L chain encoding gene libraries into a single dicistronic expression vector capable of expressing and assembling a heterodimeric antibody molecule, (3) expressing the assembled heterodimeric antibody molecule on the surface of a filamentous phage particle, (4) isolating the surface-expressed phage particle using immunoaffinity techniques such as panning of phage particles against a preselected antigen, thereby isolating one or more species of phagemid containing particular H and L chain-encoding genes and antibody molecules that immunoreact with the preselected antigen.

As described herein the Examples, the resulting phagemid library can be manipulated to increase and/or alter the immunospecificities of the monoclonal antibodies of the library to produce and subsequently identify additional, desirable, human monoclonal antibodies of the present invention.

For example, the heavy (H) chain and light (L) chain immunoglobulin molecule encoding genes can be randomly mixed (shuffled) to create new HL pairs in an assembled immunoglobulin molecule. Additionally, either or both the H and L chain encoding genes can be mutagenized in the complementarity determining region (CDR) of the variable region of the immunoglobulin polypeptide, and subsequently screened for desirable immunoreaction and neutralization capabilities.

In one embodiment, the H and L genes can be cloned into separate, monocistronic expression vectors, referred to as a "binary" system described further herein. In this method, step (2) above differs in that the combining of H and L chain encoding genes occurs by the co-introduction of the two binary plasmids into a single host cell for expression and assembly of a phagemid having the surface accessible antibody heterodimer molecule.

In one shuffling embodiment, the shuffling can be accomplished with the binary expression vectors, each capable of expressing a single heavy or light chain encoding gene, as described in Example 6.

In the present methods, the antibody molecules are monoclonal because the cloning methods allow for the preparation of clonally pure species of antibody producing cell lines. In addition, the monoclonal antibodies are human because the H and L chain encoding genes are derived from human immunoglobulin producing immune cells, such as spleen, thymus, bone marrow, and the like.

The method of producing a HIV-neutralizing human monoclonal antibody also requires that the resulting antibody library, immunoreactive with a preselected HIV antigen, is screened for the presence of antibody species which have the capacity to neutralize HIV in one or more of the assays described herein for determining neutralization capacity. Thus, a preferred library of antibody molecules is first produced which binds to an HIV antigen, preferably gp160, gp120, gp41, the V3 loop region of gp160, or the CD4 binding site of gp120 and gp41, and then is screened for the presence of HIV-neutralizing antibodies as described herein.

Additional libraries can be screened from shuffled libraries for additional HIV-immunoreactive and neutralizing human monoclonal antibodies.

As a further characterization of the present invention the nucleotide and corresponding amino acid residue sequence of the antibody molecule's H or L chain encoding gene is determined by nucleic acid sequencing. The primary amino acid residue sequence information provides essential information regarding the antibody molecule's epitope reactivity.

Sequence comparisons of identified HIV-immunoreactive monoclonal antibody variable chain region sequences are shown herein in Figures 10-13. The sequences are aligned based on sequence homology, and groups of related antibody molecules are identified thereby in which heavy chain or light chain genes share substantial sequence homology.

An exemplary preparation of a human monoclonal antibody is described in the Examples. The isolation of a particular vector capable of expressing an antibody of interest involves the introduction of the dicistronic expression vector into a host cell permissive for expression of filamentous phage genes and the assembly of phage particles. Where the binary vector system is used, both vectors are introduced in the host cell. Typically, the host is E. coli. Thereafter, a helper phage genome is introduced into the host cell containing the immunoglobulin expression vector(s) to provide the genetic complementation necessary to allow phage particles to be assembled. The resulting host cell is cultured to allow the introduced phage genes and immunoglobulin genes to be expressed, and for phage particles to be assembled and shed from the host cell. The shed phage particles are then harvested (collected) from the host cell culture media and screened for desirable immunoreaction and neutralization properties. Typically, the harvested particles are "panned" for immunoreaction with a preselected antigen. The strongly immunoreactive particles are then collected, and individual species of particles are clonally isolated and further screened for HIV neutralization. Phage which produce neutralizing antibodies are selected and used as a source of a human HIV neutralizing monoclonal antibody of this invention.

Human monoclonal antibodies of this invention can also be produced by altering the nucleotide sequence of a polynucleotide sequence that encodes a heavy or light chain of a monoclonal antibody of this invention. For example, by site directed mutagenesis, one can alter the nucleotide sequence of an expression vector and thereby introduce changes in the resulting expressed amino acid residue sequence. Thus one can take the polynucleotide of SEQ ID NO 66, for example, and convert it into the polynucleotide of SEQ ID NO 67. Similarly, one can take a known polynucleotide and randomly alter it by random mutagenesis, reintroduce the altered polynucleotide into an expression system and subsequently screen the product H:L pair for HIV-neutralizing activity.

Site-directed and random mutagenesis methods are well known in the polynucleotide arts, and are not to be construed as limiting as methods for altering the nucleotide sequence of a subject polynucleotide.

Due to the presence of the phage particle in an immunoaffinity isolated antibody, one embodiment involves the manipulation of the resulting cloned genes to truncate the immunoglobulin-coding gene such that a soluble Fab fragment is secreted by the host E. coli cell containing the phagemid vector. Thus, the resulting manipulated cloned immunoglobulin genes produce a soluble Fab which can be readily characterized in ELISA assays for epitope binding studies, in competition assays with known anti-HIV antibody molecules, and in HIV neutralization assays. The solubilized Fab provides a reproducible and comparable antibody preparation for comparative and characterization studies.

The preparation of soluble Fab is generally described in the immunological arts, and can be conducted as described herein in Example 2b6), or as described by Burton et al., Proc. Natl. Acad. Sci., USA, 88:10134-10137 (1991).

### G. Expression Vectors and Polynucleotides for Expressing Anti-HIV Monoclonal Antibodies

The preparation of human monoclonal antibodies of this invention depends, in one embodiment, on the cloning and expression vectors used to prepare the combinatorial antibody libraries described herein. The cloned immunoglobulin heavy and light chain genes can be shuttled between lambda vectors, phagemid vectors and plasmid vectors at various stages of the methods described herein.

The phagemid vectors produce fusion proteins that are expressed on the surface of an assembled filamentous phage particle.

A preferred phagemid vector of the present invention is a recombinant DNA (rDNA) molecule containing a nucleotide sequence that codes for and is capable of expressing a fusion polypeptide containing, in the direction of amino- to carboxy-terminus, (1) a prokaryotic secretion signal domain, (2) a heterologous polypeptide defining an immunoglobulin heavy or light chain variable region, and (3) a filamentous phage membrane anchor domain. The vector includes DNA expression control sequences for expressing the fusion polypeptide, preferably prokaryotic control sequences.

The filamentous phage membrane anchor is preferably a domain of the cpIII or cpVIII coat protein capable of associating with the matrix of a filamentous phage particle, thereby incorporating the fusion polypeptide onto the phage surface.

The secretion signal is a leader peptide domain of a protein that targets the protein to the periplasmic membrane of gram negative bacteria. A preferred secretion signal is a pelB secretion signal. The predicted amino acid residue sequences of the secretion signal domain from two pelB gene product variants from Erwinia carotova are described in Lei et al., Nature, 331:543-546 (1988).

The leader sequence of the pelB protein has previously been used as a secretion signal for fusion proteins (Better et al., Science, 240:1041-1043 (1988); Sastry et al., Proc. Natl. Acad. Sci., USA, 86:5728-5732 (1989); and Mullinax et al., Proc. Natl. Acad. Sci., USA, 87:8095-8099 (1990)). Amino acid residue sequences for other secretion signal polypeptide domains from E. coli useful in this invention as described in Oliver, Escherichia coli and Salmonella Typhimurium, Neidhard, F.C. (ed.), American Society for Microbiology, Washington, D.C., 1:56-69 (1987).

Preferred membrane anchors for the vector are obtainable from filamentous phage M13, f1, fd, and equivalent filamentous phage. Preferred membrane anchor domains are found in the coat proteins encoded by gene III and gene VIII. The membrane anchor domain of a filamentous phage coat protein is a portion of the carboxy terminal region of the coat protein and includes a region of hydrophobic amino acid residues for spanning a lipid bilayer membrane, and a region of charged amino acid residues normally found at the cytoplasmic face of the membrane and extending away from the membrane.

In the phage f1, gene VIII coat protein's membrane spanning region comprises residue Trp-26 through Lys-40, and the cytoplasmic region comprises the carboxy-terminal 11 residues from 41 to 52 (Ohkawa et al., J. Biol. Chem., 256:9951-9958 (1981)). An exemplary membrane anchor would consist of residues 26 to 40 of cpVIII. Thus, the amino acid residue sequence of a preferred membrane anchor domain is derived from the M13 filamentous phage gene VIII coat protein (also designated cpVIII or CP 8). Gene VIII coat protein is present on a mature filamentous phage over the majority of the phage particle with typically about 2500 to 3000 copies of the coat protein.

In addition, the amino acid residue sequence of another preferred membrane anchor domain is derived from the M13 filamentous phage gene III coat protein (also designated cpIII). Gene III coat protein is present on a mature filamentous phage at one end of the phage particle with typically about 4 to 6 copies of the coat protein.

For detailed descriptions of the structure of filamentous phage particles, their coat proteins and particle assembly, see the reviews by Rached et al., Microbiol. Rev., 50:401-427 (1986); and Model et al., in "The Bacteriophages: Vol. 2", R. Calendar, ed. Plenum Publishing Co., pp. 375-456 (1988).

DNA expression control sequences comprise a set of DNA expression signals for expressing a structural gene product and include both 5' and 3' elements, as is well known, operatively linked to the cistron such that the cistron is able to express a structural gene product. The 5' control sequences define a promoter for initiating transcription and a ribosome binding site operatively linked at the 5' terminus of the upstream translatable DNA sequence.

To achieve high levels of gene expression in E. coli, it is necessary to use not only strong promoters to generate large quantities of mRNA, but also ribosome binding sites to ensure that the mRNA is efficiently translated. In E. coli, the ribosome binding site includes an initiation codon (AUG) and a sequence 3-9 nucleotides long located 3-11 nucleotides upstream from the initiation codon (Shine et al., Nature, 254:34 (1975). The sequence, AGGAGGU, which is called the Shine-Dalgarno (SD) sequence, is complementary to the 3' end of E. coli 16S rRNA. Binding of the ribosome to mRNA and the sequence at the 3' end of the mRNA can be affected by several factors:
(i) The degree of complementarity between the SD sequence and 3' end of the 16S rRNA.
(ii) The spacing and possibly the DNA sequence lying between the SD sequence and the AUG. Roberts et al., Proc. Natl. Acad. Sci., USA, 76:760, (1979a); Roberts et al., Proc. Natl. Acad. Sci. USA, 76:5596 (1979b); Guarente et al., Science, 209:1428 (1980); and Guarente et al., Cell, 20:543 (1980). Optimization is achieved by measuring the level of expression of genes in plasmids in which this spacing is systematically altered. comparison of different mRNAs shows that there are statistically preferred sequences from positions -20 to +13 (where the A of the AUG is position 0). Gold et al., Annu. Rev. Microbiol., 35:365 (1981). Leader sequences have been shown to influence translation dramatically. Roberts et al., 1979 a, b supra.
(iii) The nucleotide sequence following the AUG, which affects ribosome binding. Taniguchi et al., J. Mol. Biol., 118:533 (1978).

The 3' control sequences define at least one termination (stop) codon in frame with and operatively linked to the heterologous fusion polypeptide.

In preferred embodiments, the vector utilized includes a prokaryotic origin of replication or replicon, i.e., a DNA sequence having the ability to direct autonomous replication and maintenance of the recombinant DNA molecule extra chromosomally in a prokaryotic host cell, such as a bacterial host cell, transformed therewith. Such origins of replication are well known in the art. Preferred origins of replication are those that are efficient in the host organism. A preferred host cell is E. coli. For use of a vector in E. coli. a preferred origin of replication is ColE1 found in pBR322 and a variety of other common plasmids. Also preferred is the p15A origin of replication found on pACYC and its derivatives. The ColEl and p15A replicon have been extensively utilized in molecular biology, are available on a variety of plasmids and are described at least by Sambrook et al., in "Molecular Cloning: a Laboratory Manual", 2nd edition, Cold Spring Harbor Laboratory Press (1989).

The ColE1 and p15A replicons are particularly preferred for use in one embodiment of the present invention where two "binary" plasmids are utilized because they each have the ability to direct the replication of plasmid in E. coli while the other replicon is present in a second plasmid in the same E. coli cell. In other words, ColE1 and p15A are non-interfering replicons that allow the maintenance of two plasmids in the same host (see, for example, Sambrook *et al., supra,* at pages 1.3-1.4). This feature is particularly important in the binary vectors embodiment of the present invention because a single host cell permissive for phage replication must support the independent and simultaneous replication of two separate vectors, namely a first vector for expressing a heavy chain polypeptide, and a second vector for expressing a light chain polypeptide.

In addition, those embodiments that include a prokaryotic replicon can also include a gene whose expression confers a selective advantage, such as drug resistance, to a bacterial host transformed therewith. Typical bacterial drug resistance genes are those that confer resistance to ampicillin, tetracycline, neomycin/kanamycin or cholamphenicol. Vectors typically also contain convenient restriction sites for insertion of translatable DNA sequences. Exemplary vectors are the plasmids pUC8, pUC9, pBR322, and pBR329 available from BioRad Laboratories, (Richmond, CA) and pPL and pKK223 available from Pharmacia, (Piscataway, NJ).

A vector for expression of a monoclonal antibody of the invention on the surface of a filamentous phage particle is a recombinant DNA (rDNA) molecule adapted for receiving and expressing translatable first and second DNA sequences in the form of first and second polypeptides wherein one of the polypeptides is fused to a filamentous phage coat protein membrane anchor. That is, at least one of the polypeptides is a fusion polypeptide containing a filamentous phage membrane anchor domain, a prokaryotic secretion signal domain, and an immunoglobulin heavy or light chain variable domain.

A DNA expression vector for expressing a heterodimeric antibody molecule provides a system for independently cloning (inserting) the two translatable DNA sequences into two separate cassettes present in the vector, to form two separate cistrons for expressing the first and second polypeptides of the antibody molecule, or the ligand binding portions of the polypeptides that comprise the antibody molecule (i.e., the H and L variable regions of an immunoglobulin molecule). The DNA expression vector for expressing two cistrons is referred to as a dicistronic expression vector.

The vector comprises a first cassette that includes upstream and downstream translatable DNA sequences operatively linked via a sequence of nucleotides adapted for directional ligation to an insert DNA. The upstream translatable sequence encodes the secretion signal as defined herein. The downstream translatable sequence encodes the filamentous phage membrane anchor as defined herein. The cassette preferably includes DNA expression control sequences for expressing the receptor polypeptide that is produced when an insert translatable DNA sequence (insert DNA) is directionally inserted into the cassette via the sequence of nucleotides adapted for directional ligation. The filamentous phage membrane anchor is preferably a domain of the cpIII or cpVIII coat protein capable of binding the matrix of a filamentous phage particle, thereby incorporating the fusion polypeptide onto the phage surface.

The receptor expressing vector also contains a second cassette for expressing a second receptor polypeptide. The second cassette includes a second translatable DNA sequence that encodes a secretion signal, as defined herein, operatively linked at its 3' terminus via a sequence of nucleotides adapted for directional ligation to a downstream DNA sequence of the vector that typically defines at least one stop codon in the reading frame of the cassette. The second translatable DNA sequence is operatively linked at its 5' terminus to DNA expression control sequences forming the 5' elements. The second cassette is capable, upon insertion of a translatable DNA sequence (insert DNA), of expressing the second fusion polypeptide comprising a receptor of the secretion signal with a polypeptide coded by the insert DNA.

An upstream translatable DNA sequence encodes a prokaryotic secretion signal as described earlier. The upstream translatable DNA sequence encoding the pelB secretion signal is a preferred DNA sequence for inclusion in a receptor expression vector. A downstream translatable DNA sequence encodes a filamentous phage membrane anchor as described earlier. Thus, a downstream translatable DNA sequence encodes an amino acid residue sequence that corresponds, and preferably is identical, to the membrane anchor domain of either a filamentous phage gene III or gene VIII coat polypeptide.

A cassette in a DNA expression vector of this invention is the region of the vector that forms, upon insertion of a translatable DNA sequence (insert DNA), a sequence of nucleotides capable of expressing, in an appropriate host, a fusion polypeptide. The expression-competent sequence of nucleotides is referred to as a cistron. Thus, the cassette comprises DNA expression control elements operatively linked to the upstream and downstream translatable DNA sequences. A cistron is formed when a translatable DNA sequence is directionally inserted (directionally ligated) between the upstream and downstream sequences via the sequence of nucleotides adapted for that purpose. The resulting three translatable DNA sequences, namely the upstream, the inserted and the downstream sequences, are all operatively linked in the same reading frame.

Thus, a DNA expression vector for expressing an antibody molecule provides a system for cloning translatable DNA sequences into the cassette portions of the vector to produce cistrons capable of expressing the first and second polypeptides, i.e., the heavy and light chains of a monoclonal antibody.

As used herein, the term "vector" refers to a nucleic acid molecule capable of transporting between different genetic environments another nucleic acid to which it has been operatively linked. Preferred vectors are those capable of autonomous replication and expression of structural gene products present in the DNA segments to which they are operatively linked. Vectors, therefore, preferably contain the replicons and selectable markers described earlier.

As used herein with regard to DNA sequences or segments, the phrase "operatively linked" means the sequences or segments have been covalently joined, preferably by conventional phosphodiester bonds, into one strand of DNA, whether in single or double stranded form. The choice of vector to which transcription unit or a cassette of this invention is operatively linked depends directly, as is well known in the art, on the functional properties desired, e.g., vector replication and protein expression, and the host cell to be transformed, these being limitations inherent in the art of constructing recombinant DNA molecules.

A sequence of nucleotides adapted for directional ligation, i.e., a polylinker, is a region of the DNA expression vector that (1) operatively links for replication and transport the upstream and downstream translatable DNA sequences and (2) provides a site or means for directional ligation of a DNA sequence into the vector. Typically, a directional polylinker is a sequence of nucleotides that defines two or more restriction endonuclease recognition sequences, or restriction sites. Upon restriction cleavage, the two sites yield cohesive termini to which a translatable DNA sequence can be ligated to the DNA expression vector. Preferably, the two restriction sites provide, upon restriction cleavage, cohesive termini that are non-complementary and thereby permit directional insertion of a translatable DNA sequence into the cassette. In one embodiment, the directional ligation means is provided by nucleotides present in the upstream translatable DNA sequence, downstream translatable DNA sequence, or both. In another embodiment, the sequence of nucleotides adapted for directional ligation comprises a sequence of nucleotides that defines multiple directional cloning means. Where the sequence of nucleotides adapted for directional ligation defines numerous restriction sites, it is referred to as a multiple cloning site.

In a preferred embodiment, a DNA expression vector is designed for convenient manipulation in the form of a filamentous phage particle encapsulating a genome according to the teachings of the present invention. In this embodiment, a DNA expression vector further contains a nucleotide sequence that defines a filamentous phage origin of replication such that the vector, upon presentation of the appropriate genetic complementation, can replicate as a filamentous phage in single stranded replicative form and be packaged into filamentous phage particles. This feature provides the ability of the DNA expression vector to be packaged into phage particles for subsequent segregation of the particle, and vector contained therein, away from other particles that comprise a population of phage particles.

A filamentous phage origin of replication is a region of the phage genome, as is well known, that defines sites for initiation of replication, termination of replication and packaging of the replicative form produced by replication (see, for example, Rasched et al., Microbiol. Rev., 50:401-427 (1986); and Horiuchi, J. Mol. Biol., 188:215-223 (1986)).

A preferred filamentous phage origin of replication for use in the present invention is an M13, f1 or fd phage origin of replication (Short et al., Nucl. Acids Res., 16:7583-7600 (1988)). Preferred DNA expression vectors for cloning and expression a human monoclonal antibody of this invention are the dicistronic expression vectors pComb8, pComb2-8, pComb3, pComb2-3 and pComb2-3', described herein.

A particularly preferred vector of the present invention includes a polynucleotide sequence that encodes a heavy or light chain variable region of a human monoclonal antibody of the present invention. Particularly preferred are vectors that include a nucleotide sequence that encodes a heavy or light chain amino acid residue sequence shown in Figures 10-13, that encodes a heavy or light chain having the binding specificity of those sequences shown in Figures 10-13, or that encodes a heavy or light chain having conservative substitutions relative to a sequence shown in Figures 10-13, and complementary polynucleotide sequences thereto.

Insofar as polynucleotides are component parts of a DNA expression vector for producing a human monoclonal antibody heavy or light chain immunoglobulin variable region amino acid residue sequence, the invention also contemplates isolated polynucleotides that encode such heavy or light chain sequences.

It is to be understood that, due to the genetic code and its attendant redundancies, numerous polynucleotide sequences can be designed that encode a contemplated heavy or light chain immunoglobulin variable region amino acid residue sequence. Thus, the invention contemplates such alternate polynucleotide sequences incorporating the features of the redundancy of the genetic code.

Insofar as the expression vector for producing a human monoclonal antibody of this invention is carried in a host cell compatible with expression of the antibody, the invention contemplates a host cell containing a vector or polynucleotide of this invention. A preferred host cell is E. coli, as described herein.

E. coli cultures containing preferred expression vectors that produce a human monoclonal antibody of this invention were deposited pursuant to Budapest Treaty requirements with the American Type Culture Collection (ATCC), Rockville, MD, as described herein.

### Examples

The following examples are intended to illustrate, but not limit, the scope of the invention.

### 1. Construction of a Dicistronic Expression Vector for Producing a Heterodimeric Receptor on Phage Particles

To obtain a vector system for generating a large number of Fab antibody fragments that can be screened directly, expression libraries in bacteriophage Lambda have previously been constructed as described in Huse et al., Science, 246:1275-1281 (1989). These systems did not contain design features that provide for the expressed Fab to be targeted to the surface of a filamentous phage particle.

The main criterion used in choosing a vector system was the necessity of generating the largest number of Fab fragments which could be screened directly. Bacteriophage Lambda was selected as the starting point to develop an expression vector for three reasons. First, in vitro packaging of phage DNA was the most efficient method of reintroducing DNA into host cells. Second, it was possible to detect protein expression at the level of single phage plaques. Finally, the screening of phage libraries typically involved less difficulty with nonspecific binding. The alternative, plasmid cloning vectors, are only advantageous in the analysis of clones after they have been identified. This advantage was not lost in the present system because of the use of a dicistronic expression vector such as pCombVIII, thereby permitting a plasmid containing the heavy chain, light chain, or Fab expressing inserts to be excised.

### a. Construction of Dicistronic Expression Vector pCOMB

### 1) Preparation of Lambda Zap ™II

Lambda Zap™ II is a derivative of the original Lambda Zap (ATCC Accession No. 40,298) that maintains all of the characteristics of the original Lambda Zap including 6 unique cloning sites, fusion protein expression, and the ability to rapidly excise the insert in the form of a phagemid (Bluescript SK-), but lacks the SAM 100 mutation, allowing growth on many Non-Sup F strains, including XL1-Blue. The Lambda Zap™ II was constructed as described in Short et al., Nuc. Acids Res., 16:7583-7600, 1988, by replacing the Lambda S gene contained in a 4254 base pair (bp) DNA fragment produced by digesting Lambda Zap with the restriction enzyme Nco I. This 4254 bp DNA fragment was replaced with the 4254 bp DNA fragment containing the Lambda S gene isolated from Lambda gt10 (ATCC # 40,179) after digesting the vector with the restriction enzyme Nco I. The 4254 bp DNA fragment isolated from lambda gt10 was ligated into the original Lambda Zap vector using T4 DNA ligase and standard protocols such as those described in Current Protocols in Molecular Biology, Ausubel et al., eds., John Wiley and Sons, NY, 1987, to form Lambda Zap™ II.

### 2) Preparation of Lambda Hc2

To express a plurality of V_{H}-coding DNA homologs in an E. coli host cell, a vector designated Lambda Hc2 was constructed. The vector provided the following: the capacity to place the V_{H}-coding DNA homologs in the proper reading frame; a ribosome binding site as described by Shine et al., Nature, 254:34 (1975); a leader sequence directing the expressed protein to the periplasmic space designated the pe1B secretion signal; a polynucleotide sequence that coded for a known epitope (epitope tag); and also a polynucleotide that coded for a spacer protein between the V_{H}-coding DNA homolog and the polynucleotide coding for the epitope tag. Lambda Hc2 has been previously described by Huse et al., Science, 246:1275-1281 (1989).

To prepare Lambda Hc2, a synthetic DNA sequence containing all of the above features was constructed by designing single stranded polynucleotide segments of 20-40 bases that would hybridize to each other and form the double stranded synthetic DNA sequence shown in Figure 1. The individual single-stranded polynucleotide segments are shown in Table 1.

Polynucleotides N2, N3, N9-4, N11, N10-5, N6, N7 and N8 (Table 1) were kinased by adding 1 µl of each polynucleotide 0.1 micrograms/microliter (µg/µl) and 20 units of T₄ polynucleotide kinase to a solution containing 70 mM Tris-HCl (Tris[hydroxymethyl] aminomethane hydrochloride) at pH 7.6, 10 mM MgCl₂, 5 mM dithiothreitol (DTT), 10 mM beta-mercaptoethanol, 500 micrograms per milliliter (µg/ml) bovine serum albumin (BSA). The solution was maintained at 37 degrees Centigrade (37°C) for 30 minutes and the reaction stopped by maintaining the solution at 65°C for 10 minutes. The two end polynucleotides, 20 nanograms (ng) of polynucleotides N1 and polynucleotides N12, were added to the above kinasing reaction solution together with 1/10 volume of a solution containing 20 mM Tris-HCl at pH 7.4, 2.0 mM MgCl₂ and 50 mM NaCl. This solution was heated to 70°C for 5 minutes and allowed to cool to room temperature, approximately 25°C, over 1.5 hours in a 500 ml beaker of water. During this time period all 10 polynucleotides annealed to form the double stranded synthetic DNA insert shown in Figure 1. The individual polynucleotides were covalently linked to each other to stabilize the synthetic DNA insert by adding 40 µl of the above reaction to a solution containing 50 mM Tris-HCl at pH 7.5, 7 mM MgCl₂, 1 mM DTT, 1 mM adenosine triphosphate (ATP) and 10 units of T4 DNA ligase. This solution was maintained at 37°C for 30 minutes and then the T4 DNA ligase was inactivated by maintaining the solution at 65°C for 10 minutes. The end polynucleotides were kinased by mixing 52 µl of the above reaction, 4 µl of a solution containing 10 mM ATP and 5 units of T4 polynucleotide kinase. This solution was maintained at 37°C for 30 minutes and then the T4 polynucleotide kinase was inactivated by maintaining the solution at 65°C for 10 minutes.

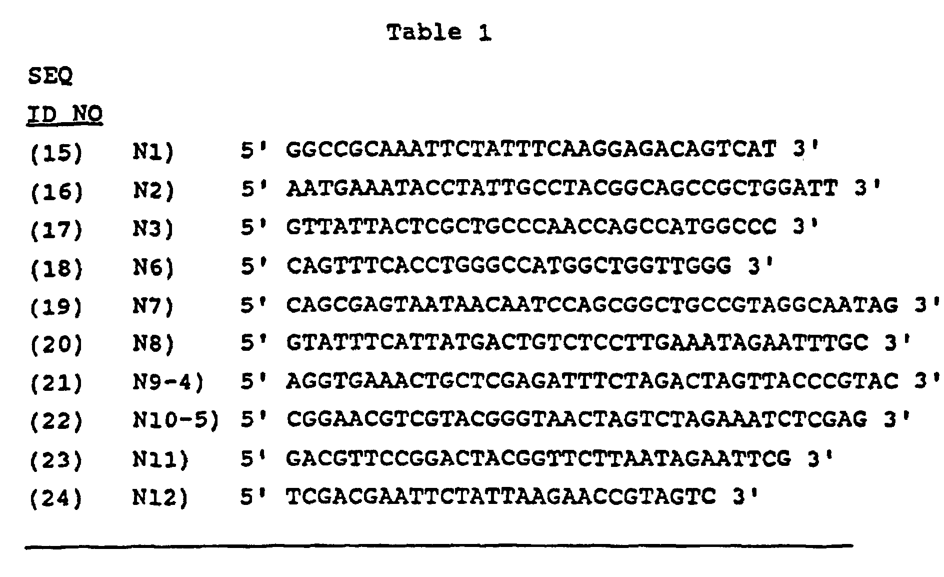

The completed synthetic DNA insert was ligated directly into the Lambda Zap™ II vector described in Example 1a1) that had been previously digested with the restriction enzymes, Not I and Xho I. The ligation mixture was packaged according to the manufacture's instructions using Gigapack II Gold packing extract available from Stratagene, La Jolla, California. The packaged ligation mixture was plated on XL1-Blue cells (Stratagene). Individual lambda plaques were cored and the inserts excised according to the in vivo excision protocol for Lambda Zap™ II provided by the manufacturer (Stratagene). This in vivo excision protocol moved the cloned insert from the Lambda Hc2 vector into a phagemid vector to allow easy for manipulation and sequencing. The accuracy of the above cloning steps was confirmed by sequencing the insert using the Sanger dideoxy method described in by Sanger et al., Proc. Natl. Acad. Sci., USA, 74:5463-5467 (1977) and using the manufacture's instructions in the AMV Reverse Transcriptase ³⁵S-ATP sequencing kit (Stratagene). The sequence of the resulting double-stranded synthetic DNA insert in the V_{H} expression vector (Lambda Hc2) is shown in Figure 1. The sequence of each strand (top and bottom) of Lambda Hc2 is listed in the Sequence Listing as SEQ ID NO 1 and SEQ ID NO 2, respectively. The resultant Lambda Hc2 expression vector is shown in Figure 2.

### 3) Preparation of Lambda Lc2

To express a plurality of V_{L}-coding DNA homologs in an E. coli host cell, a vector designated Lambda Lc2 was constructed having the capacity to place the V_{L}-coding DNA homologs in the proper reading frame, provided a ribosome binding site as described by Shine et al., Nature, 254:34 (1975), provided the pelB gene leader sequence secretion signal that has been previously used to successfully secrete Fab fragments in E. coli by Lei et al., J. Bac., 169:4379 (1987) and Better et al., Science, 240:1041 (1988), and also provided a polynucleotide containing a restriction endonuclease site for cloning. Lambda Lc2 has been previously described by Huse et al., Science, 246:1275-1281 (1989).

A synthetic DNA sequence containing all of the above features was constructed by designing single stranded polynucleotide segments of 20-60 bases that would hybridize to each other and form the double stranded synthetic DNA sequence shown in Figure 3. The sequence of each individual single-stranded polynucleotide segment (O1-O8) within the double stranded synthetic DNA sequence is shown in Table 2.

Polynucleotides 02, 03, 04, 05, 06 and 07 (Table 2) were kinased by adding 1 µl (0.1 µg/µl) of each polynucleotide and 20 units of T₄ polynucleotide kinase to a solution containing 70 mM Tris-HCl at pH 7.6, 10 mM MgCl₂, 5 mM DTT, 10 mM beta-mercaptoethanol, 500 µg/ml of BSA. The solution was maintained at 37°C for 30 minutes and the reaction stopped by maintaining the solution at 65°C for 10 minutes. The 20 ng each of the two end polynucleotides, 01 and 08, were added to the above kinasing reaction solution together with 1/10 volume of a solution containing 20.0 mM Tris-HCl at pH 7.4, 2.0 mM MgCl₂ and 15.0 mM sodium chloride (NaCl). This solution was heated to 70°C for 5 minutes and allowed to cool to room temperature, approximately 25°C, over 1.5 hours in a 500 ml beaker of water. During this time period all 8 polynucleotides annealed to form the double stranded synthetic DNA insert shown in Figure 3. The individual polynucleotides were covalently linked to each other to stabilize the synthetic DNA insert by adding 40 µl of the above reaction to a solution containing 50 mM Tris-HCl at pH 7.5, 7 mM MgCl₂, 1 mM DTT, 1 mM ATP and 10 units of T4 DNA ligase. This solution was maintained at 37°C for 30 minutes and then the T4 DNA ligase was inactivated by maintaining the solution at 65°C for 10 minutes. The end polynucleotides were kinased by mixing 52 µl of the above reaction, 4 µl of a solution containing 10 mM ATP and 5 units of T4 polynucleotide kinase. This solution was maintained at 37°C for 30 minutes and then the T4 polynucleotide kinase was inactivated by maintaining the solution at 65°C for 10 minutes.

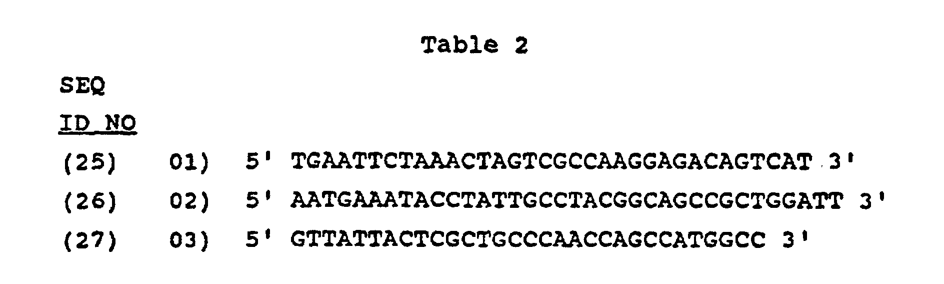

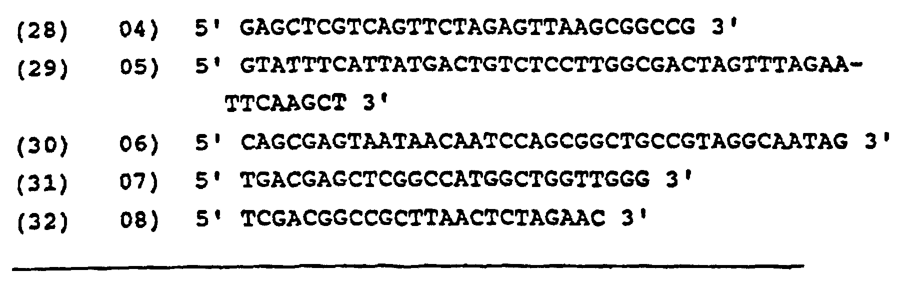

The completed synthetic DNA insert was ligated directly into the Lambda Zap™ II vector described in Example 1a1) that had been previously digested with the restriction enzymes Sac I and Xho I. The ligation mixture was packaged according to the manufacture's instructions using Gigapack II Gold packing extract (Stratagene). The packaged ligation mixture was plated on XL1-Blue cells (Stratagene). Individual lambda plaques were cored and the inserts excised according to the in vivo excision protocol for Lambda Zap™ II provided by the manufacturer (Stratagene). This in vivo excision protocol moved the cloned insert from the Lambda Lc2 vector into a plasmid phagemid vector allow for easy manipulation and sequencing. The accuracy of the above cloning steps was confirmed by sequencing the insert using the manufacture's instructions in the AMV Reverse Transcriptase ³⁵S-dATP sequencing kit (Stratagene). The sequence of the resulting Lc2 expression vector (Lambda Lc2) is shown in Figure 3. Each strand is separately listed in the Sequence Listing as SEQ ID NO 3 and SEQ ID NO 4. The resultant Lc2 vector is schematically diagrammed in Figure 4.

A preferred vector for use in this invention, designated Lambda Lc3, is a derivative of Lambda Lc2 prepared above. Lambda Lc2 contains a Spe I restriction site located 3' to the EcoR I restriction site and 5' to the Shine-Dalgarno ribosome binding site as shown in the sequence in Figure 3 and in SEQ ID NO 3. A Spe I restriction site is also present in Lambda Hc2 as shown in Figures 1 and 2 and in SEQ ID NO 1. A combinatorial vector, designated pComb, was constructed by combining portions of Lambda Hc2 and Lc2 together as described in Example 1a4) below. The resultant combinatorial pComb vector contained two Spe I restriction sites, one provided by Lambda Hc2 and one provided by Lambda Lc2, with an EcoR I site in between. Despite the presence of two Spe I restriction sites, DNA homologs having Spe I and EcoR I cohesive termini were successfully directionally ligated into a pComb expression vector previously digested with Spe I and EcoR I as described in Example 1b below. The proximity of the EcoR I restriction site to the 3' Spe I site, provided by the Lc2 vector, inhibited the complete digestion of the 3' Spe I site. Thus, digesting pComb with Spe I and EcoR I did not result in removal of the EcoR I site between the two Spe I sites.

The presence of a second Spe I restriction site may be undesirable for ligations into a pComb vector digested only with Spe I as the region between the two sites would be eliminated. Therefore, a derivative of Lambda Lc2 lacking the second or 3' Spe I site, designated Lambda Lc3, was produced by first digesting Lambda Lc2 with Spe I to form a linearized vector. The ends were filled in to form blunt ends which are ligated together to result in Lambda Lc3 lacking a Spe I site. Lambda Lc3 is a preferred vector for use in constructing a combinatorial vector as described below.

### 4) Preparation of pComb

Phagemids were excised from the expression vectors Lambda Hc2 or Lambda Lc2 using an in vivo excision protocol described above. Double stranded DNA was prepared from the phagemid-containing cells according to the methods described by Holmes et al., Anal. Biochem., 114:193 (1981). The phagemids resulting from in vivo excision contained the same nucleotide sequences for antibody fragment cloning and expression as did the parent vectors, and are designated phagemid Hc2 and Lc2, corresponding to Lambda Hc2 and Lc2, respectively.

For the construction of combinatorial phagemid vector pComb, produced by combining portions of phagemid Hc2 and phagemid Lc2, phagemid Hc2 was first digested with Sac I to remove the restriction site located 5' to the LacZ promoter. The linearized phagemid was then blunt ended with T4 polymerase and ligated to result in a Hc2 phagemid lacking a Sac I site. The modified Hc2 phagemid and the Lc2 phagemid were then separately restriction digested with Sca I and EcoR I to result in a Hc2 fragment having from 5' to 3' Sca I, Not I, Xho I, Spe I and EcoR I restriction sites and a Lc2 fragment having from 5' to 3' EcoR I, Sac I, Xba I and Sac I restriction sites. The linearized phagemids were then ligated together at their respective cohesive ends to form pComb, a circularized phagemid having a linear arrangement of restriction sites of Not I, Xho I, Spe I, EcoR I, Sac I, Xba I, Not I, Apa I and Sca I. The ligated phagemid vector was then inserted into an appropriate bacterial host and transformants were selected on the antibiotic ampicillin.

Selected ampicillin resistant transformants were screened for the presence of two Not I sites. The resulting ampicillin resistant combinatorial phagemid vector was designated pComb, the schematic organization of which is shown in Figure 5. The resultant combinatorial vector, pComb, consisted of a DNA molecule having two cassettes to express two fusion proteins and having nucleotide residue sequences for the following operatively linked elements listed in a 5' to 3' direction: a first cassette consisting of an inducible LacZ promoter upstream from the LacZ gene; a Not I restriction site; a ribosome binding site; a pe1B leader; a spacer; a cloning region bordered by a 5' Xho and 3' Spe I restriction site; a decapeptide tag followed by expression control stop sequences; an EcoR I restriction site located 5' to a second cassette consisting of an expression control ribosome binding site; a pelB leader; a spacer region; a cloning region bordered by a 5' Sac I and a 3' Xba I restriction site followed by expression control stop sequences and a second Not I restriction site.

A preferred combinatorial vector for use in this invention, designated pComb2, is constructed by combining portions of phagemid Hc2 and phagemid Lc3 as described above for preparing pComb. The resultant combinatorial vector, pComb2, consists of a DNA molecule having two cassettes identical to pComb to express two fusion proteins identically to pComb except that a second Spe I restriction site in the second cassette is eliminated.

### b. Construction of the pCombIII Vector for Expressing Fusion Proteins Having a Bacteriophage Coat Protein Membrane Anchor

Because of the multiple endonuclease restriction cloning sites, the pComb phagemid expression vector prepared above is a useful cloning vehicle for modification for the preparation of an expression vector for use in this invention. To that end, pComb was digested with EcoR I and Spe I followed by phosphatase treatment to produce linearized pComb.

### 1) Preparation of pCombIII

A separate phagemid expression vector was constructed using sequences encoding bacteriophage cpIII membrane anchor domain. A PCR product defining the DNA sequence encoding the filamentous phage coat protein, cpIII, membrane anchor containing a LacZ promotor region sequence 3' to the membrane anchor for expression of the light chain and Spe I and EcoR I cohesive termini was prepared from M13mp18, a commercially available bacteriophage vector (Pharmacia, Piscataway, New Jersey).

To prepare a modified cpIII, replicative form DNA from M13mp18 was first isolated. Briefly, into 2 ml of LB (Luria-Bertani medium), 50 µl of a culture of a bacterial strain carrying an F' episome (JM107, JM109 or TG1) was admixed with a one tenth suspension of bacteriophage particles derived from a single plaque. The admixture was incubated for 4 to 5 hours at 37°C with constant agitation. The admixture was then centrifuged at 12,000 x g for 5 minutes to pellet the infected bacteria. After the supernatant was removed, the pellet was resuspended by vigorous vortexing in 100 µl of ice-cold solution I. Solution I was prepared by admixing 50 mM glucose, 10 mM EDTA (disodium ethylenediaminetetraacetic acid) and 25 mM Tris-HCl at pH 8.0, and autoclaving for 15 minutes.

To the bacterial suspension, 200 µl of freshly prepared Solution II was admixed and the tube was rapidly inverted five times. Solution II was prepared by admixing 0.2 N NaOH and 1% SDS. To the bacterial suspension, 150 µl of ice-cold Solution III was admixed and the tube was vortexed gently in an inverted position for 10 seconds to disperse Solution III through the viscous bacterial lysate. Solution III was prepared by admixing 60 ml of 5 M potassium acetate, 11.5 ml of glacial acetic acid and 28.5 ml of water. The resultant bacterial lysate was then stored on ice for 5 minutes followed by centrifugation at 12,000 x g for 5 minutes at 4°C in a microfuge. The resultant supernatant was recovered and transferred to a new tube. To the supernatant was added an equal volume of phenol/chloroform and the admixture was vortexed. The admixture was then centrifuged at 12,000 x g for 2 minutes in a microfuge. The resultant supernatant was transferred to a new tube and the double-stranded bacteriophage DNA was precipitated with 2 volumes of ethanol at room temperature. After allowing the admixture to stand at room temperature for 2 minutes, the admixture was centrifuged to pellet the DNA. The supernatant was removed and the pelleted replicative form DNA was resuspended in 25 µl of Tris-HCl at pH 7.6, and 10 mM EDTA (TE).

The double-stranded M13mp18 replicative form DNA was then used as a template for isolating the gene encoding the membrane anchor domain at cpIII, the sequence of which is listed in the Sequence Listing as SEQ ID NO 33. The amino acid residue sequence of membrane anchor domain cpIII is listed in SEQ ID NO 34. M13mp18 replicative form DNA was prepared as described above and used as a template for two PCR amplifications for construction of a DNA fragment consisting of the mature gene for cpIII membrane anchor domain located 5' to a sequence encoding the LacZ promoter, operator and cap-binding site for controlling light chain expression. The restriction sites, Spe I and EcoR I, were created in the amplification reactions and were located at the 5' and 3' ends of the fragment respectively. The procedure for creating this fragment by combining the products of two separate PCR amplifications is described below.

The primer pair, G-3(F) (SEQ ID NO 35) and G-3(B) (SEQ ID NO 36) listed in Table 3, was used in the first PCR reaction as performed above to amplify the cpIII membrane anchor gene and incorporate Spe I and Nhe I restriction sites into the fragment. For the PCR reaction, 2 µl containing 1 ng of M13mp18 replicative form DNA was admixed with 10 µl of 10X PCR buffer purchased commercially (Promega Biotech, Madison, Wisconsin) in a 0.5 ml microfuge tube. To the DNA admixture, 8 µl of a 2.5 mM solution of dNTPs (dATP, dCTP, dGTP, dTTP) was admixed to result in a final concentration of 200 micromolar (µM). Three µl (equivalent to 60 picomoles (pM)) of the G-3(F) primer and 3 µl (60 pM) of the 3' backward G-3(B) primer was admixed into the DNA solution. To the admixture, 73 µl of sterile water and 1 µl/5 units of polymerase (Promega Biotech) was added. Two drops of mineral oil were placed on top of the admixture and 40 rounds of PCR amplification in a thermocycler were performed. The amplification cycle consisted of 52°C for 2 minutes, 72°C for 1.5 minutes and 91°C for 2 minutes. The resultant PCR modified cpIII membrane anchor domain DNA fragment from M13mp18 containing samples were then purified with Gene Clean (BIO101, La Jolla, California), extracted twice with phenol/chloroform, once with chloroform followed by ethanol precipitation and were stored at -70°C in 10 mM Tris-HCl at pH 7.5, and 1 mM EDTA.

The resultant PCR modified cpIII DNA fragment having Spe I and Nhe I sites in the 5' and 3' ends, respectively, of the fragment was verified by electrophoresis in a 1% agarose gel. The area in the agarose containing the modified cpIII DNA fragment was isolated from the agarose. The sequence of the PCR modified cpIII membrane anchor domain DNA fragment is listed in the Sequence Listing as SEQ ID NO 40. The resultant amplified PCR fragment also contained nucleotide sequences for encoding a five amino acid tether composed of four glycine residues and one serine juxtaposed between the heavy chain and cpIII encoding domains. Once expressed, the five amino acid residue sequence lacking an orderly secondary structure served to minimize the interaction between the Fab and cpIII domains.

A second PCR amplification using the primer pairs, Lac-F (SEQ ID NO 37) and Lac-B (SEQ ID NO 38) listed in Table 3, was performed on a separate aliquot of M13mp18 replicative form template DNA to amplify the LacZ promoter, operator and Cap-binding site having a 5' Nhe I site and a 3' EcoR I site. The primers used for this amplification were designed to incorporate a Nhe I site on the 5' end of the amplified fragment to overlap with a portion of the 3' end of the cpIII gene fragment and of the Nhe I site 3' to the amplified cpIII fragment. The reaction and purification of the PCR product was performed as described above. The sequence of the resultant PCR modified cpIII DNA fragment having a 5' Nhe I and 3' EcoR I restriction site is listed in the Sequence Listing as SEQ ID NO 41.

An alternative Lac-B primer for use in constructing the cpIII membrane anchor and LacZ promotor region was Lac-B' as shown in Table 3. The amplification reactions were performed as described above with the exception that in the second PCR amplification, Lac-B' was used with Lac-F instead of Lac-B. The product from the amplification reaction is listed in the sequence listing as SEQ ID NO 41 from nucleotide position 1 to nucleotide position 172. The use of Lac-B' resulted in a LacZ region lacking 29 nucleotides on the 3' end but was functionally equivalent to the longer fragment produced with the Lac-F and Lac-B primers.

The products of the first and second PCR amplifications using the primer pairs G-3(F) and G-3(B) and Lac-F and Lac-B were then recombined at the nucleotides corresponding to cpIII membrane anchor overlap and Nhe I restriction site and subjected to a second round of PCR using the G-3(F) (SEQ ID NO 35) and Lac-B (SEQ ID NO 38) primer pair to form a recombined PCR DNA fragment product consisting of the following: a 5' Spe I restriction site; a cpIII DNA membrane anchor domain beginning at the nucleotide residue sequence which corresponds to the amino acid residue 198 of the entire mature cpIII protein; an endogenous stop site provided by the membrane anchor at amino acid residue number 112; a Nhe I restriction site, a LacZ promoter, operator and Cap-binding site sequence; and a 3' EcoR I restriction site.

To construct a phagemid vector for the coordinate expression of a heavy chain-cpIII fusion protein as prepared in Example 2 with kappa light chain, the recombined PCR modified cpIII membrane anchor domain DNA fragment was then restriction digested with Spe I and EcoR I to produce a DNA fragment for directional ligation into a similarly digested pComb2 phagemid expression vector having only one Spe I site prepared in Example 1a4) to form a pComb2-III (also referred to as pComb2-III) phagemid expression vector. Thus, the resultant ampicillin resistance conferring pComb2-3 vector, having only one Spe I restriction site, contained separate LacZ promoter/operator sequences for directing the separate expression of the heavy chain (Fd)-cpIII fusion product and the light chain protein. The expressed proteins were directed to the periplasmic space by pelB leader sequences for functional assembly on the membrane. Inclusion of the phage F1 intergenic region in the vector allowed for packaging of single stranded phagemid with the aid of helper phage. The use of helper phage superinfection lead to expression of two forms of cpIII. Thus, normal phage morphogenesis was perturbed by competition between the Fab-cpIII fusion and the native cpIII of the helper phage for incorporation into the virion for Fab-cpVIII fusions. In addition, also contemplated for use in this invention are vectors conferring chloramphenicol resistance and the like.

A more preferred phagemid expression vector for use in this invention having additional restriction enzyme cloning sites, designated pComb-III' or pComb2-3', was prepared as described above for pComb2-3 with the addition of a 51 base pair fragment from pBluescript as described by Short et al., Nuc. Acids Res., 16:7583-7600 (1988) and commercially available from Stratagene. To prepare pComb2-3', pComb2-3 was first digested with Xho I and Spe I restriction enzymes to form a linearized pComb2-3. The vector pBluescript was digested with the same enzymes releasing a 51 base pair fragment containing the restriction enzyme sites Sal I, Acc I, Hinc II, Cla I, Hind III, EcoR V, Pst I, Sma I and BamH I. The 51 base pair fragment was ligated into the linearized pComb2-3 vector via the cohesive Xho I and Spe I termini to form pComb2-3'.

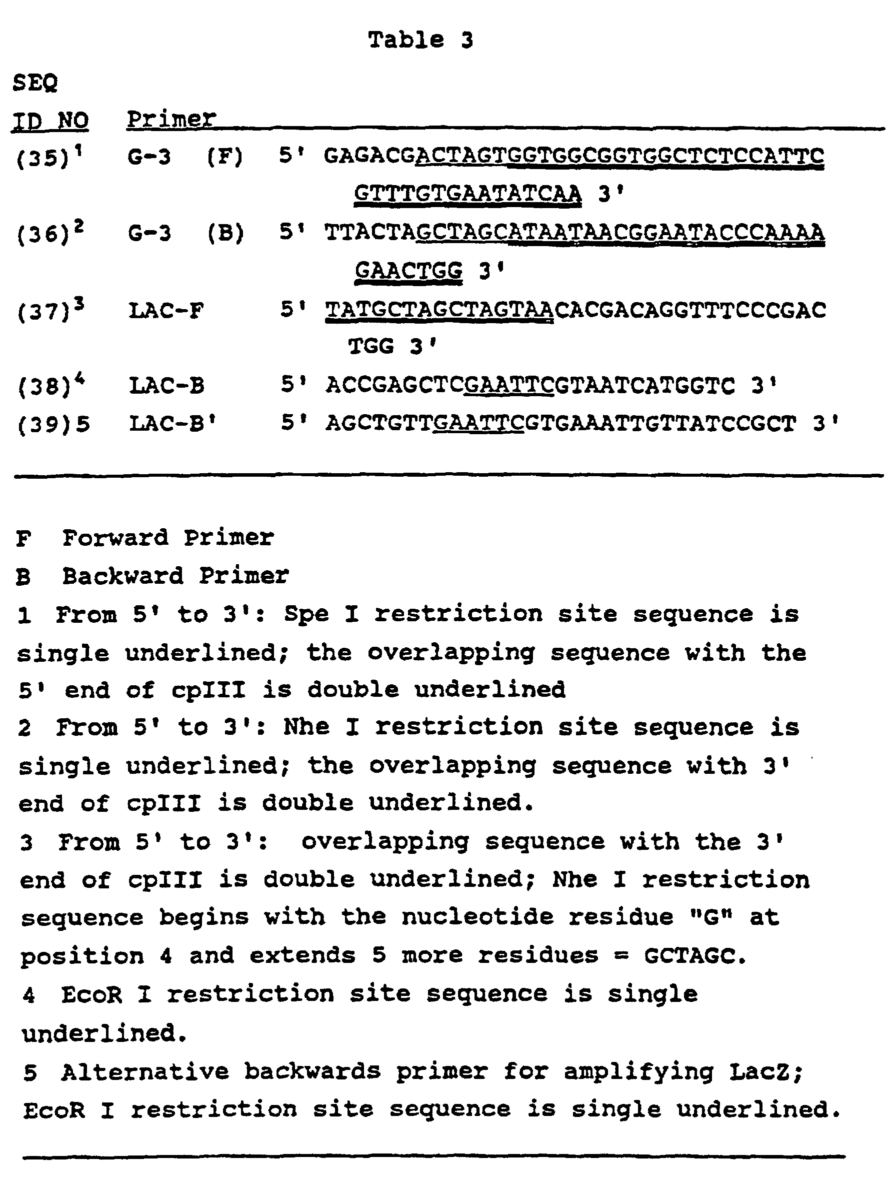

### 2. Isolation of HIV-1-Specific Monoclonal Antibodies Produced from the Dicistronic Expression Vector. pComb2-3

In practicing this invention, the heavy (Fd consisting of V_{H} and C_{H}l) and light (kappa) chains (V_{L}, C_{L}) of antibodies are first targeted to the periplasm of E. coli for the assembly of heterodimeric Fab molecules. In order to obtain expression of antibody Fab libraries on a phage surface, the nucleotide residue sequences encoding either the Fd or light chains must be operatively linked to the nucleotide residue sequence encoding a filamentous bacteriophage coat protein membrane anchor. A coat protein for use in this invention in providing a membrane anchor is III (cpIII or cp3). In the Examples described herein, methods for operatively linking a nucleotide residue sequence encoding a Fd chain to a cpIII membrane anchor in a fusion protein of this invention are described.

In a phagemid vector, a first and second cistron consisting of translatable DNA sequences are operatively linked to form a dicistronic DNA molecule. Each cistron in the dicistronic DNA molecule is linked to DNA expression control sequences for the coordinate expression of a fusion protein, Fd-cpIII, and a kappa light chain.

The first cistron encodes a periplasmic secretion signal (pelB leader) operatively linked to the fusion protein, Fd-cpIII. The second cistron encodes a second pelB leader operatively linked to a kappa light chain. The presence of the pelB leader facilitates the coordinated but separate secretion of both the fusion protein and light chain from the bacterial cytoplasm into the periplasmic space.

In this process, the phagemid expression vector carries an ampicillin selectable resistance marker gene (beta lactamase or bla) in addition to the Fd-cpIII fusion and the kappa chain. The f1 phage origin of replication facilitates the generation of single stranded phagemid. The isopropyl thiogalactopyranoside (IPTG) induced expression of a dicistronic message encoding the Fd-cpIII fusion (V_{H}, C_{H1}, cpIII) and the light chain (V_{L}, C_{L}) leads to the formation of heavy and light chains. Each chain is delivered to the periplasmic space by the pelB leader sequence, which is subsequently cleaved. The heavy chain is anchored in the membrane by the cpIII membrane anchor domain while the light chain is secreted into the periplasm. The heavy chain in the presence of light chain assembles to form Fab molecules. This same result can be achieved if, in the alternative, the light chain is anchored in the membrane via a light chain fusion protein having a membrane anchor and heavy chain is secreted via a pelB leader into the periplasm.

With subsequent infection of E. coli with a helper phage, as the assembly of the filamentous bacteriophage progresses, the coat protein III is incorporated on the tail of the bacteriophage.

### a. Preparation of Lymphocyte RNA

Five milliliters of bone marrow was removed by aspiration from HIV-1 asymptomatic seropositive individuals. Total cellular RNA was prepared from the bone marrow lymphocytes as described above using the RNA preparation methods described by Chomczynski et al., Anal Biochem., 162:156-159 (1987) and using the RNA isolation kit (Stratagene) according to the manufacturer's instructions. Briefly, for immediate homogenization of the cells in the isolated bone marrow, 10 ml of a denaturing solution containing 3.0 M guanidinium isothiocyanate containing 71 µl of beta-mercaptoethanol was admixed to the isolated bone marrow. One ml of sodium acetate at a concentration of 2 M at pH 4.0 was then admixed with the homogenized cells. one ml of phenol that had been previously saturated with H₂O was also admixed to the denaturing solution containing the homogenized spleen. Two ml of a chloroform:isoamyl alcohol (24:1 v/v) mixture was added to this homogenate. The homogenate was mixed vigorously for ten seconds and maintained on ice for 15 minutes. The homogenate was then transferred to a thick-walled 50 ml polypropylene centrifuged tube (Fisher Scientific Company, Pittsburgh, PA). The solution was centrifuged at 10,000 x g for 20 minutes at 4°C. The upper RNA-containing aqueous layer was transferred to a fresh 50 ml polypropylene centrifuge tube and mixed with an equal volume of isopropyl alcohol. This solution was maintained at -20°C for at least one hour to precipitate the RNA. The solution containing the precipitated RNA was centrifuged at 10,000 x g for twenty minutes at 4°C. The pelleted total cellular RNA was collected and dissolved in 3 ml of the denaturing solution described above. Three ml of isopropyl alcohol was added to the re-suspended total cellular RNA and vigorously mixed. This solution was maintained at -20°C for at least 1 hour to precipitate the RNA. The solution containing the precipitated RNA was centrifuged at 10,000 x g for ten minutes at 4°C. The pelleted RNA was washed once with a solution containing 75% ethanol. The pelleted RNA was dried under vacuum for 15 minutes and then re-suspended in dimethyl pyrocarbonate-treated (DEPC-H₂O) H₂O.

Messenger RNA (mRNA) enriched for sequences containing long poly A tracts was prepared from the total cellular RNA using methods described in Molecular Cloning: A Laboratory Manual, Maniatis et al., eds., Cold Spring Harbor, NY, (1982). Briefly, one half of the total RNA isolated from a single donor prepared as described above was resuspended in one ml of DEPC-H₂O and maintained at 65°C for five minutes. One ml of 2X high salt loading buffer consisting of 100 mM Tris-HCl, 1 M NaCl, 2.0 mM EDTA at pH 7.5, and 0.2% SDS was admixed to the resuspended RNA and the mixture allowed to cool to room temperature.

The total purified mRNA was then used in PCR amplification reactions as described in Example 2c. Alternatively, the mRNA was further purified to poly A+ RNA by the following procedure. The total MRNA was applied to an oligo-dT (Collaborative Research Type 2 or Type 3) column that was previously prepared by washing the oligo-dT with a solution containing 0.1 M sodium hydroxide and 5 mM EDTA and then equilibrating the column with DEPC-H₂O. The eluate was collected in a sterile polypropylene tube and reapplied to the same column after heating the eluate for 5 minutes at 65°C. The oligo-dT column was then washed with 2 ml of high salt loading buffer consisting of 50 mM Tris-HCl at pH 7.5, 500 mM sodium chloride, 1 mM EDTA at pH 7.5 and 0.1% SDS. The oligo dT column was then washed with 2 ml of 1X medium salt buffer consisting of 50 mM Tris-HCl, pH 7.5, 100 mM, 1 mM EDTA and 0.1% SDS. The messenger RNA was eluted from the oligo-dT column with 1 ml of buffer consisting of 10 mM Tris-HCl at pH 7.5, 1 mM EDTA at pH 7.5, and 0.05% SDS. The messenger RNA was purified by extracting this solution with phenol/chloroform followed by a single extraction with 100% chloroform. The messenger RNA was concentrated by ethanol precipitation and resuspended in DEPC H₂O.

The resultant purified mRNA contained a plurality of anti-HIV encoding V_{H} and V_{L} sequences for preparation of an anti-HIV-1 Fab DNA library.

### b. Construction of a Combinatorial HIV-1 Antibody Library

### 1) Selection of Oligonucleotide Primers

The nucleotide sequences encoding the immunoglobulin protein CDR's are highly variable. However, there are several regions of conserved sequences that flank the V region domains of either the light or heavy chain, for instance, and that contain substantially conserved nucleotide sequences, i.e., sequences that will hybridize to the same primer sequence. Therefore, polynucleotide synthesis (amplification) primers that hybridize to the conserved sequences and incorporate restriction sites into the DNA homolog produced that are suitable for operatively linking the synthesized DNA fragments to a vector were constructed. More specifically, the primers were designed so that the resulting DNA homologs produced can be inserted into an expression vector of this invention in reading frame with the upstream translatable DNA sequence at the region of the vector containing the directional ligation means.

For amplification of the V_{H} domains, primers were designed to introduce cohesive termini compatible with directional ligation into the unique Xho I and Spe I sites of the pComb2-3 expression vector. In all cases, the 5' primers VH1a (5' CAGGTGCAGCTCGAGCAGTCTGGG 3' SEQ ID NO 42) and VH3a (5' GAGGTGCAGCTCGAGGAGTCTGGG 3' SEQ ID NO 43) were designed to maximize homology with the V_{H}1 and V_{H}3 subgroup families, respectively, although considerable cross-priming of other subgroups was expected. The Xho I restriction site for cloning into the pComb2-3 vector is underlined. The 3' primer CGlz having the nucleotide sequence 5' GCATGTACTAGTTTTGTCACAAGATTTGGG 3' (SEQ ID NO 44) used in conjunction with the 5' primers is the primer for the heavy chain corresponding to part of the hinge region. The Spe I site for cloning into the pComb2-3 vector is underlined.

The nucleotide sequences encoding the V_{L} domain are highly variable. However, there are several regions of conserved sequences that flank the V_{L} domains including the J_{L}, V_{L} framework regions and V_{L} leader/promotor. Therefore, amplification primers were constructed that hybridized to the conserved sequences and incorporate restriction sites that allow cloning the amplified fragments into the pComb2-3 expression vector cut with Sac I and Xba I.

For amplification of the kappa V_{L} domains analogous to the heavy chain primers listed above, the 5' primers, VK1a (5' GACATCGAGCTCACCCAGTCTCCA 3' SEQ ID NO 45) and VK3a (5' GAAATTGAGCTCACGCAGTCTCCA 3' SEQ ID NO 46), were used. These primers also introduced a Sac I restriction endonuclease site indicated by the underlined nucleotides to allow the V_{L} DNA homolog to be cloned into the pComb2-3 expression vector. The 3' V_{L} amplification primer, CK1a having a nucleotide sequence 5' GCGCCCTCTAGAACTAACACTCTCCCCTGTTGAAGCTCTTTGTGACGGGcAAG 3' (SEQ ID NO 47) corresponding to the 3' end of the light chain was used to amplify the light chain while incorporating the underlined Xba I restriction endonuclease site required to insert the V_{L} DNA homolog into the pComb2-3 expression vector.

All primers and synthetic polynucleotides described herein, were either purchased from Research Genetics in Huntsville, Alabama or synthesized on an Applied Biosystems DNA synthesizer, model 381A, using the manufacturer's instruction.

### 2) PCR Amplification of V_{H} and V_{L} DNA Homologs

In preparation for PCR amplification, mRNA prepared above was used as a template for cDNA synthesis by a primer extension reaction. First, 20-50 µg of total mRNA in water was first hybridized (annealed) at 70°C for 10 minutes with 600 ng (60.0 pmol) of either the heavy or light chain 3' primers listed above. Subsequently, the hybridized admixture was used in a typical 50 µl reverse transcription reaction containing 200 µM each of dATP, dCTP, dGTP and dTTP, 40 mM Tris-HCl at pH 8.0, 8 mM MgCl₂, 50 mM NaCl, 2 mM spermidine and 600 units of reverse transcriptase (Superscript, BRL). The reaction admixture was then maintained for one hour at 37°C to form an RNA-cDNA admixture.

Three µl of the resultant RNA-cDNA admixture was then used in PCR amplification in a reaction volume of 100 µl containing a mixture of all four dNTPs at a concentration of 60 µM, 50 mM KCl, 10 mM Tris-HCl at pH 8.3, 15 mM MgCl₂, 0.1% gelatin and 5 units of Thermus aquaticus (Tag) DNA polymerase (Perkin-Elmer-Cetus, Emeryville, California), and 60 pmol of the appropriate 5' and 3' primers listed above. The separate reaction admixtures were overlaid with mineral oil and subjected to 35 cycles of amplification. Each amplification cycle included denaturation at 91°C for 1 minute, annealing at 52°C for 2 minutes and polynucleotide synthesis by primer extension (elongation) at 72°C for 1.5 minutes, followed by a final maintenance period of 10 minutes at 72°C. An aliquot of the reaction admixtures were then separately electrophoresed on a 2% agarose gel. After successful amplification as determined by gel electrophoretic migration, the remainder of the RNA-cDNA was amplified after which the PCR products of a common 3' primer were pooled into separate V_{H}-and V_{L}-coding DNA homolog-containing samples and were then extracted twice with phenol/chloroform, once with chloroform, ethanol precipitated and were stored at -70°C in 10 mM Tris-HCl at pH 7.5, and 1 mM EDTA.

### 3) Insertion of V_{H} and V_{L}-Coding DNA Homologs into pComb2-3 Expression Vector

The V_{H}-coding DNA homologs (heavy chain) prepared above were then digested with an excess of Xho I and Spe I for subsequent ligation into a similarly digested and linearized pComb2-3 in a total volume of 150 µl with 10 units of ligase at 16°C overnight. The construction of the library was performed as described by Burton et al., Proc. Natl. Acad. Sci., USA, 88:10134-10137 (1991). Briefly, following ligation, the pComb2-3 vector containing heavy chain DNA was then transformed by electroporation into 300 µl of XL1-Blue cells. After transformation and culturing, library size was determined by plating aliquots of the culture. Typically the library had about 10⁷ members. An overnight culture was then prepared from which phagemid DNA containing the heavy chain library was prepared.

For the cloning of the V_{L}-coding DNA homologs (light chain), 10 µg of phagemid DNA containing the heavy chain library was then digested with Sac I and SbaI. The resulting linearized vector was treated with phosphatase and purified by agarose gel electrophoresis. The desired fragment, 4.7 kb in length, was excised from the gel. Ligation of this vector with prepared light chain PCR DNA proceeded as described above for heavy chain. A library of approximately 10⁷ members having heavy chain fragments operatively linked to the cpIII anchor sequence (Fd-cpIII) and light chain fragments was thus produced.

### 4) Preparation of Phage Expressing Fab Heterodimers

Following transformation of the resultant library produced above into XL1-Blue cells, phage were prepared to allow for isolation of HIV-1 specific Fabs by panning on target antigens. To isolate phage on which heterodimer expression has been induced, 3 ml of SOC medium (SOC was prepared by admixture of 20 g bacto-tryptone, 5 g yeast extract and 0.5 g NaCl in one liter of water, adjusting the pH to 7.5 and admixing 20 ml of glucose just before use to induce the expression of the Fd-cpIII and light chain heterodimer) was admixed and the culture was shaken at 220 rpm for one hour at 37°C, after which time 10 ml of SB (SB was prepared by admixing 30 g tryptone, 20 g yeast extract, and 10 g Mops buffer per liter with pH adjusted to 7) containing 20 µg/ml carbenicillin and 10 µg/ml tetracycline and the admixture was shaken at 300 rpm for an additional hour. This resultant admixture was admixed to 100 ml SB containing 50 µg/ml carbenicillin and 10 µg/ml tetracycline and shaken for one hour, after which time helper phage VCSM13 (10¹² pfu) were admixed and the admixture was shaken for an additional two hours. After this time, 70 µg/ml kanamycin was admixed and maintained at 30°C overnight. The lower temperature resulted in better heterodimer incorporation on the surface of the phage. The supernatant was cleared by centrifugation (4000 rpm for 15 minutes in a JA10 rotor at 4°C). Phage were precipitated by admixture of 4% (w/v) polyethylene glycol 8000 and 3% (w/v) NaCl and maintained on ice for 30 minutes, followed by centrifugation (9000 rpm for 20 minutes in a JA10 rotor at 4°C). Phage pellets were resuspended in 2 ml of PBS and microcentrifuged for three minutes to pellet debris, transferred to fresh tubes and stored at -20°C for subsequent screening as described below.

For determining the titering colony forming units (cfu), phage (packaged phagemid) were diluted in SB and 1 µl was used to infect 50 µl of fresh (OD600 = 1) XL1-Blue cells grown in SB containing 10 µg/ml tetracycline. Phage and cells were maintained at room temperature for 15 minutes and then directly plated on LB/carbenicillin plates.

### 5) Selection of Anti-HIV-1 Heterodimers on Phage Surfaces

### (a) Multiple Pannings of the Phage Library

The phage library produced in Example 2b4) was panned against recombinant gp120 of HIV-1 strain IIIb as described herein on coated microtiter plate to select for anti-gp120 heterodimers. A second phage library was panned against recombinant gp41 (American Biotechnologies, Boston, MA) as described below to select for anti-gp41 heterodimers.

The panning procedure used was a modification of that originally described by Parmley and Smith (Parmley et al., Gene, 73:305-318 (1988). Four rounds of panning were performed to enrich for specific antigen-binding clones. For this procedure, four wells of a microtiter plate (Costar 3690) were coated overnight at 4°C with 25 µl of 40 µg/ml gp120 or gp41 (American Biotechnologies) prepared above in 0.1 M bicarbonate, pH 8.6. The wells were washed twice with water and blocked by completely filling the well with 3% (w/v) BSA in PBS and maintaining the plate at 37°C for one hour. After the blocking solution was shaken out, 50 µl of the phage library prepared above (typically 10¹¹ cfu) were admixed to each well, and the plate was maintained for two hours at 37°C.

Phage were removed and the plate was washed once with water. Each well was then washed ten times with TBS/Tween (50 mM Tris-HCl at pH 7.5, 150 mM NaCl, 0.5% Tween 20) over a period of one hour at room temperature where the washing consisted of pipetting up and down to wash the well, each time allowing the well to remain completely filled with TBS/Tween between washings. The plate was washed once more with distilled water and adherent phage were eluted by the addition of 50 µl of elution buffer (0.1 M HCl, adjusted to pH 2.2 with solid glycine, containing 1 mg/ml BSA) to each well followed by maintenance at room temperature for 10 minutes. The elution buffer was pipetted up and down several times, removed, and neutralized with 3 µl of 2 M Tris base per 50 µl of elution buffer used.

Eluted phage were used to infect 2 ml of fresh (OD₆₀₀ = 1) E. coli XL1-Blue cells for 15 minutes at room temperature, after which time 10 ml of SB containing 20 µg/ml carbenicillin and 10 µg/ml tetracycline was admixed. Aliquots of 20, 10, and 1/10 µl were removed from the culture for plating to determine the number of phage (packaged phagemids) that were eluted from the plate. The culture was shaken for one hour at 37°C, after which it was added to 100 ml of SB containing 50 µg/ml carbenicillin and 10 µg/ml tetracycline and shaken for one hour. Helper phage VCSM13 (10¹² pfu) were then added and the culture was shaken for an additional two hours. After this time, 70 µg/ml kanamycin was added and the culture was incubated at 37°C overnight. Phage preparation and further panning were repeated as described above.

Following each round of panning, the percentage yield of phage were determined, where % yield - (number of phage eluted/number of phage applied) X 100. The initial phage input ratio was determined by titering on selective plates to be approximately 10¹¹ cfu for each round of panning. The final phage output ratio was determined by infecting two ml of logarithmic phase XL1-Blue cells as described above and plating aliquots on selective plates. In the first panning for gp120-reactive phage, 4.6 X 10¹¹ phage were applied to four wells and 7.7 X 10⁵ phage were eluted. After the fourth panning 1.0 X 10⁸ phage were eluted. From this procedure, 20 clones were selected from the Fab library for their ability to bind to glycosylated recombinant gp120 from the IIIB strain of HIV-1. Five clones were selected from the Fab library specific for binding to gp41. The panned phage surface libraries were then converted into ones expressing soluble Fab fragments for further screening by ELISA as described below.

In addition to panning on gp120 of strain IIIB and gp41, also contemplated as antigens for panning of combinatorial libraries is recombinant gp120 (IIIB strain) produced in baculovirus and recombinant gp120 (SF2 strain) produced in Chinese Hamster Ovary cells obtained as described by Steimer et al., Science, 254:105-108 (1991). Another antigen, a synthetic cyclic peptide, N=CH-(CH₂)₃CO[SISGPGRAFYTG]NCH₂CO-Cys-NH₂ (SEQ ID NO 48) prepared as described by Satterthwait et al., Bulletin of the World Health Organization, 68: Suppl., 17-25 (1990) corresponding to the central most conserved part of the V3 loop of gp120 was coupled to maleimide-activated BSA. The library was panned using 1, 2 or 4 ELISA wells coated with 1 µg of protein antigen or 10 µg BSA-peptide per well. Four rounds of panning were carried out for each antigen as described above. Eluted phage from the final round were used to infect XL1-Blue cells. Four rounds of panning against the four antigens produced an amplification in eluted phage of between 100 and 1000 fold. The panned phage surface libraries were then converted into ones expressing soluble Fab fragments for further screening by ELISA as described below.

### 6) Preparation of Soluble Heterodimers and Characterization of Binding Specificity to HIV-1 Antigens

In order to further characterize the specificity of the mutagenized heterodimers expressed on the surface of phage as described above, soluble Fab heterodimers from acid eluted phage were prepared and analyzed in ELISA assays on HIV-1 derived antigen-coated plates and by competitive ELISA.

To prepare soluble heterodimers, phagemid DNA from the 20 gp120 positive clones and the 5 gp41 positive clones prepared above was isolated and digested with Spe I and Nhe I. Digestion with these enzymes produced compatible cohesive ends. The 4.7 kb DNA fragment lacking the gene III portion was gel-purified (0.6% agarose) and self-ligated. Transformation of E. coli XL1-Blue afforded the isolation of recombinants lacking the cpIII fragment. Clones were examined for removal of the cpIII fragment by Xho I - Xba I digestion, which should yield an 1.6-kb fragment. Clones were grown in 100 ml SB containing 50 µg/ml carbenicillin and 20 mM MgCl₂ at 37°C until an OD₆₀₀ of 0.2 was achieved. IPTG (1 mM) was added and the culture grown overnight at 30°C (growth at 37°C provides only a light reduction in heterodimer yield). Cells were pelleted by centrifugation at 4000 rpm for 15 minutes in a JA10 rotor at 4°C. Cells were resuspended in 4 ml PBS containing 34 µg/ml phenylmethylsulfonyl fluoride (PMSF) and lysed by sonication on ice (2-4 minutes at 50% duty). Debris was pelleted by centrifugation at 14,000 rpm in a JA20 rotor at 4°C for 15 minutes. The supernatant was used directly for ELISA analysis as described below and was stored at -20°C. For the study of a large number of clones, 10 ml cultures provided sufficient heterodimer for analysis. In this case, sonications were performed in 2 ml of buffer.

Assays as described above were also performed for the gp41-specific clones.

### a) Screening by ELISA

The soluble heterodimers prepared above were assayed by ELISA. For this assay, gp120 and gp41 were separately admixed to individual wells of a microtiter plate as described above for the panning procedure and maintained at 4°C overnight to allow the protein solution to adhere to the walls of the well. After the maintenance period, the wells were washed five times with water and thereafter maintained for one hour at 37°C with 100 µl solution of 1% BSA diluted in PBS to block nonspecific sites on the wells. Afterwards, the plates were inverted and shaken to remove the BSA solution. Twenty-five µl of soluble heterodimers prepared above reactive with the specific glycoprotein substrate were then admixed to each well and maintained at 37°C for one hour to form immunoreaction products. Following the maintenance period, the wells were washed ten times with water to remove unbound soluble antibody and then maintained with a 25 µl of a 1:1000 dilution of secondary goat anti-human IgG F(ab')₂ conjugated to alkaline phosphatase diluted in PBS containing 1% BSA. The wells were maintained at 37°C for one hour after which the wells were washed ten times with water followed by development with 50 µl of p-nitrophenyl phosphate (PNPP). Color development was monitored at 405 nm. Positive clones gave A405 values of >1 (mostly >1.5) after 10 minutes, whereas negative clones gave values of 0.1 to 0.2.

Approximate concentrations of gp120-reactive Fab were determined by ELISA using a sandwich ELISA as described by Zebedee et al., Proc. Natl. Acad. Sci., USA, 89:3175-3179 (1992) and are presented in the first column of Figure 6. In addition, since Fabs are expressed in E. coli and the fraction of correctly assemble protein can vary, the amount of Fab reacting with gp120 was also assessed by ELISA titration. That data is also presented in Figure 6 in the second column.

For the clones panned against the HIV-1 derived antigens, after conversion of the panned phage surface libraries to ones expressing soluble Fab fragments, 30-40 colonies were used to transform XL1-Blue cells and the supernates screened in ELISA assays against the antigen used in panning. Generally greater than 80% of the supernates tested positive. A representative number of positives were then selected from each antigen panning for further analysis.

### (b) Competitive ELISA with Soluble gp120 and CD4

Immunoreactive heterodimers as determined in the above ELISA were then analyzed by competition ELISA to determine the affinity of the selected heterodimers. The ELISA was performed as described above on microtiter wells separately coated with 5 µg/ml of gp120 or soluble CD4 (American Biotechnologies) in 0.1 M bicarbonate buffer at pH 8.6. Increasing concentrations of soluble or free gp120 ranging in concentration from 10⁻¹¹ M up to 10⁻⁷ M diluted in 0.5% BSA/0.025% Tween 20/PBS were admixed with soluble heterodimers, the dilutions of which were determined in titration experiments that resulted in substantial reduction of OD values after a 2-fold dilution. For the CD4 competition assays, increasing concentrations of soluble or free CD4 ranging in concentration from 10⁻¹¹ M up to 10⁻⁶ M diluted in 0.5% BSA/0.025% Tween 20/PBS were admixed with soluble heterodimers. The plates were maintained for 90-120 minutes at 37°C and carefully washed ten times with 0.05% Tween 20/PBS before admixture of alkaline phosphatase-labelled goat anti-human IgG F(ab')2 at a dilution of 1:500 followed by maintenance for 1 hour at 37°C. Development was performed as described for ELISA.

To establish the relationship between neutralizing ability as described in Example 3 below could be related to antigen binding affinity of HIV-1-specific Fabs, competition ELISAs were carried out where soluble gp120 was competed with gp120 coated on ELISA plates for Fab binding. Figure 7 shows that all Fabs were competed from binding to gp120 with a IC₅₀ of approximately 10⁻⁹ M free gp120. In addition as shown in Example 3, there is no correlation between antigen affinity and neutralization. The Fabs tested included Fabs 4, 12, 21 and 7 that are members of the same groups as determined by sequence analysis and comparison as described in Example 4. Fabs 13, 27, 6, 29, 2 and 3 are all members of the different groups as determined by sequence analysis and comparison as described in Example 4. Loop 2 is an Fab fragment selected from the same library as the other Fabs but which recognizes the V3 loop. Only with the V3 loop peptide was competition carried out with gp120 from the SF2 strain.

To investigate whether neutralization could be associated with blocking of the gp120-CD4 interaction, competition ELISAs were carried out with soluble CD4 competing with Fabs for binding to gp120-coated ELISA wells. The results are shown in Figure 8. P4D10 and loop 2 are controls not expected to be competed by CD4. P4D10 is a mouse monoclonal antibody reacting with the V3 loop of gp120 (IIIB). Loop 2 Fab competition was carried out using gp120 (SF2). As shown in Figure 8 the binding of all Fabs with the exception of the controls was inhibited with an IC₅₀ of approximately 10⁻⁸ M of soluble CD4. In addition, no difference was detected between the neutralizing and non-neutralizing Fabs to gp120 inhibited by CD4. This implies that blocking of the CD4-gp120 interaction is unlikely to be an important factor in Fab neutralization of the HIV-1 virus.

Similar competition assays were performed with the Fabs panned against the four HIV-1 derived antigens. The 19 Fabs derived from panning against gp120 (IIIB) showed apparent affinities (1/concentration at 50% inhibition) for gp120 (IIIB) in the range 10⁷ - 10⁻⁹ M with most being 1-3 X 10⁻⁸ M. The panning procedure tends to select strongly for tight binders so a grouping into a relatively narrow band of affinities was expected. Of 16 Fabs derived from panning against gp160 (IIIB), 6 were also reactive with gp120 (IIIB) and competition ELISAs showed they had similar apparent affinities as the gp120-panned Fabs. The non-gp120 reactive clones from the gp160 panning showed a lower ELISA reactivity with gp160 and could not be satisfactorily competed with gp160. They may be directed against gp41 but were not pursued here. Eight Fabs derived from panning against gp120 (SF2) also showed strong ELISA reactivity with gp120 (IIIB) and gave similar apparent binding affinities. Four Fabs were derived from panning against the V3 loop peptide. Of these Fabs, 2 reacted in ELISA with gp120 (SF2) but none with gp120 (IIIB). The apparent binding affinity of these loop binders to gp120 (SF2) was 10⁻⁸ M.

To complete the survey in terms of strain cross-reactivity of Fabs, those derived from the gp120 and gp160 (IIIB) pannings were examined for ELISA reactivity with gp120 (SF2). All were reactive. Therefore, all the Fabs examined, with the exception of those selected by panning against the V3 loop peptide, bound to gp1 from IIIB and SF2 strains.

The Fabs were screened for CD4 inhibition of their binding to gp120 (IIIB) immobilized on ELISA wells. All, again with the exception of the V3 loop binders, showed sensitivity to CD4 inhibition. The inhibition constants were in the range 10⁻⁷ to 10⁻⁹ M.

Also contemplated is a competition ELISA assay where the binding of HIV-1 recombinant Fabs of this invention is performed in the presence of excess Fabs of this invention as well as those HIV-1 antibodies, polyclonal or monoclonal, present in patient sera, either asymptomatic or symptomatic, or obtained by other means such as EBV transformation and the like. The ability of an exogenously admixed antibody to compete for the binding of a characterized Fab of this invention will allow for the determination of equivalent antibodies in addition to unique epitopes and binding specificities.

### 3. Neutralizing Activity of Recombinant Human Fab Fragments Against HIV-1 In Vitro

Binding of antibodies to viruses can result in loss of infectivity or neutralization and, although not the only defense mechanism against viruses, it is widely accepted that antibodies have an important role to play. However, understanding of the molecular principles underlying antibody neutralization is limited and lags behind that of the other effector functions of antibody. Such understanding is required for the rational design of vaccines and for the most effective use of passive antibody for prophylaxis or therapy. This is particularly urgent for the human immunodeficiency viruses.

A number of studies have led to the general conclusion that viruses are neutralized by more than one mechanism and the one employed will depend on factors such as the nature of the virus, the epitope recognized, the isotype of the antibody, the cell receptor used for viral entry and the virus:antibody ratio. The principle mechanisms of neutralization can be considered as aggregation of virions, inhibition of attachment of virus to cell receptor and inhibition of events following attachment such as fusion of viral and cellular membranes and secondary uncoating of the virion. One of the important features of the third mechanism is that it may require far less than the approximately stoichiometric amounts of antibody expected for the first two mechanisms since occupation of a small number of critical sites on the virion may be sufficient for neutralization. For instance it has been shown that neutralization of the influenza A virion obeys single hit kinetics as described by Outlaw et al., Epidemiol. Infect., 106:205-220 (1992).

Intensive studies have been carried out on antibody neutralization of HIV-1. For review, see Nara et al., FASEB J., 5:2437-2455 (1991). Most have focussed on a single linear epitope in the third hypervariable domain of the viral envelope glycoprotein gp120 known as the V3 loop. Antibodies to this loop are suggested to neutralize by inhibiting fusion of viral and cell membranes. Binding to the loop resulting in neutralization can occur prior to virus-cell interaction or following gp120 binding to CD4. See, Nara, In Retroviruses of Human Aids and Related Animal Diseases, eds. Girard et al., pp. 138-150 (1988); Linsely et al., J. Virol., 62:3695-3702 (1988); and Skinner et al., J. Virol., 67:4195-4200 (1988). Features of the V3 loop are sequence variability within the loop [Goudsmit et al., FASEB J., 5:2427-2436 (1991) and Albert et al., AIDS, 4:107-112 (1990)] and sensitivity of neutralizing antibodies against the loop to sequence variations outside the loop [Nara et al., FASEB J., 5:2437-2455 (1991); Albert et al., supra; McKeating et al., AIDS, 3:777-784 (1989); and Wahlberg et al., AIDS Res. Hum. Retroviruses, 7:983-990 (1991). Hence anti-V3 loop antibodies are often strain specific and mutations in the loop in vivo may provide a mechanism for viral escape from antibody neutralization.

Recently considerable interest has focused on antibodies capable of blocking CD4 binding to gp120. A number of groups have described the features of these antibodies as (a) reacting with conformational i.e., non-linear epitopes, (b) reacting with a wide range of virus isolates and (c) being the predominant neutralizing antibodies in humans after longer periods of infection. See, Berkower, et al., J. Virol., 65:5983-5990 (1991); Steimer et al., Science, 254:105-108 (1991); Ho et al., J. Virol., 65:489-493 (1991); Kang et al., Proc. Natl. Acad. Sci., USA, 88:6171-6175 (1991); Posner et al., J. Immunol., 146:4325-4332 (1991); and Tilley et al., Res. Virol., 142:247-259 (1991). Neutralizing antibodies of this type would appear to present a promising target for potential therapeutics. The mechanism(s) of neutralization of these antibodies is unknown although there is some indication that this may not be blocking of virus attachment since a number of mouse monoclonal antibodies inhibiting CD4 binding to gp120 are either non-neutralizing or only weakly neutralizing.

The generation of human monoclonal antibodies against the envelope of HIV-1 as described by Burton et al., Proc. Natl. Acad. Sci., USA, 88:10134-10137 (1991) using combinatorial libraries allows a novel approach to the problem of neutralization. Given the lack of a three-dimensional structure for gp120 and the complexity of the virus, the approach seeks to explore neutralization at the molecular level through the behavior of related antibodies. This is possible for the following reasons: (1) the combinatorial approach allows the rapid generation of large numbers of human antibodies; (2) the antibodies (Fab fragments) are expressed in E.coli and can readily be sequenced; and (3) antibodies have similar sequences and common structural motifs allowing functional differences to be meaningfully correlated with primary structure.

Neutralization studies were performed as described herein on the human recombinant Fab fragments from 20 clones against gp120 prepared as described in Examples 1 and 2, all of which are strain cross-reactive and inhibited by CD4 from binding to gp120. The results presented herein show that neutralization was not effected by virus aggregation or cross-linking of gp120 molecules on the virion surface and was not correlated with blocking of the interaction between soluble CD4 and recombinant gp120.

Two different assays, a p24 ELISA assay and a syncytium assay, were perfomed to measure neutralization ability of the recombinant human HIV-1 immunoreactive Fabs.

For some of these assays, the recombinant Fabs were first purified. One liter cultures of SB containing 50 µg/ml carbenicillin and 20 mM MgCl₂ were inoculated with appropriate clones and induced 7 hours later with 2 mM IPTG and grown overnight at 30°C. The cell pellets were sonicated and the resultant supernatant were concentrated to a 50 ml volume. The filtered supernatants were loaded on a 25 ml protein G-anti-Fab column, washed with 120 ml buffer at a rate of 3 ml/minute and eluted with citric acid at pH 2.3. The neutralized fractions were then concentrated and exchanged into 50 mM MES at pH 6.0 and loaded onto a 2 ml Mono-S column at a rate of 1 ml/minute. A gradient of 0-500 mM NaCl was run at 1 ml/minute with the Fab eluting in the range of 200-250 mM NaCl. After concentrating, the Fabs were positive when titered on ELISA against gp120 and gave a single band at 50 kD by 10-15% SDS-PAGE. Concentration was determined by absorbance measurement at 280nm using an extinction coefficient (1 mg/ml) of 1.4.

### a. Neutralization as Measured by the p24 ELISA Assay

For this assay, diluted tissue culture supernatants of HIV-1 IIIB or MN-infected peripheral blood mononuclear cells (PBMC) (50TCID₅₀ (50% tissue culture infectious dose), 100 µl) were maintained for 2 hours at 37°C with serial dilutions (1:2), beginning at a dilution of 1:20, of recombinant Fab supernates prepared in Example 2b6). Control Fab supernates were also provided that included human neutralizing sera, a known human neutralizing monoclonal antibody 2F5 and the Fab fragment derived from that antibody by papain digestion, and a known mouse neutralizing monoclonal antibody and its F(ab')₂ fragment as described by Broliden et al., J. Virol., 64:936-940 (1990). PBMC (1 x 10⁵ cells ) were admixed to the virus/antibody admixture and maintained for 1 hour at 37°C. Thereafter, the cells were washed and maintained in RPMI 1640 medium (GIBCO) supplemented with 10% fetal calf serum, 1% glutamine, antibiotics and IL-2. The culture medium was changed at days 1 and 4. At 7 days post-infection, supernates were collected and analyzed by HIV-1 p24 antigen capture ELISA as described by Sundqvist et al., J. Med. Virol., 29:170-175 (1989) the disclosure of which is hereby incorporated by reference. Neutralization was defined as positive if an 80% or greater reduction of optical density at 490nm in the culture supernatant occurred as compared to negative Fab or negative human serum. Tests with all Fabs, mAbs and sera were repeated on at least two occasions.

### b. Quantitative Infectivity Assay Based on Syncytial Formation

A quantitative neutralization assay with the MN strain of HIV-1 was performed as described by Nara et al., AIDS Res. Human Retroviruses, 3:283-302 (1987), the disclosure of which is hereby incorporated by reference. Monolayers of CEM-SS target cells were cultured with virus, in the presence or absence of antibody, and the number of syncytia forming units determined 3-5 days later. An equivalent amount of virus was used in the assays to allow direct comparison of the various antibody concentrations tested. The assays were repeatable over a virus-surviving fraction range of 1 to 0.001 within a 2 to 4-fold difference in the concentration of antibody (P<0.001).

### c. Results of the Neutralization Assays for gp120

Assays were generally repeated at least twice with reproducible results. For the data reported in Figure 6, the gp120-specific Fab supernates were divided into two parts, one being used in the p24 assay and the other in the syncytia assay. A dash (-) indicates that there was no neutralization at 1:20 dilution in the p24 assay and 1:16 in the syncytial assay (with most clones showing no detectable neutralization at a 1:4 dilution). Neutralization titers are indicated in the figure. For the p24 assay, the titer corresponds to the greatest dilution producing >80% reduction in absorbance in ELISA. For the syncytia assay, Fabs 4 and 12 produced >95% neutralization at a 1:4 dilution of supernate and 80 and 70% reduction at 1:128 dilution respectively. These Fabs were effective neutralizers in both types of assays. They have also been shown to neutralize infection by IIIB and RF strains using a PCR-based assay of proviral integration. Fabs 6 and 7 showed no neutralization in the syncytia assay but other supernate preparations showed activity. Fab 13 was consistently effective in the p24 assay but not in the syncytia assay. A number of other clones show lower levels of neutralizing ability.

Fabs were purified from a selection of some of the clones as described above and used in both neutralization assays. As shown in Figure 9, Fabs 4 and 12 were again effective in both assays at similar levels with for example 50% inhibition of syncytial formation at an Fab concentration of approximately 20 nM (1 µg/ml). The results shown are derived from the syncytia assay using the MN strain. Fabs 7 and 21 were equally effective in the syncytial assay but somewhat less so in the p24 assay. The p24 assay indicated greater than 80% neutralization of HIV-1 MN strain for Fab 4 at 3, Fab 7 at 15, Fab 12 at 3, Fab 13 at 4 and Fab 21 at 7 µg/ml, respectively. Fab 13 however was ineffective in the syncytial assay at 25 µg/ml. For the IIIB strain, greater than 80% neutralization was observed for Fab 4 at 13, Fab 7 at 15, Fab 12 at 7 and Fab 21 at 14 µg/ml, respectively. Although Fab 11 was not effective in neutralization assays when unpurified as shown in Figure 6, following purification, Fab 11 was equally effective as Fab 12 in neutralizing HIV-1. For this reason, the Fab is being deposited with the ATCC as described in Example 7 along with Fab 12 and Fab 13.

There are a number of conclusions arising from the data shown in the Figures 6 and 9. It is apparent that HIV-1 can be neutralized without virion aggregation or cross-linking of gp120 molecules on the virion surface since monovalent Fab fragments are effective. To further confirm this finding, a Fab fragment was produced by papain digestion of a known neutralizing human monoclonal antibody. As shown in Figure 6, the Fab fragment was approximately equally effective as the whole IgG in neutralization of the MN strain of HIV-1. This is consistent with results on Fabs prepared from two mouse monoclonal antibodies to the V3 loop. An F(ab')₂ fragment of a mouse monoclonal antibody was somewhat less effective than the parent IgG in neutralization of the MN strain. Interestingly, the fragments from these control antibodies were relatively poor in neutralizing the IIIB strain of HIV-1. The results also show that there appears to be a difference between the two assays employed since Fab 13 was consistently effective in one assay but not the other. The principal variables were the incubation time of the virus and antibody prior to infection (2 hours for the p24 assay and 0.5 hours for the syncytial assay), the amount of virus used for infection, the cells used to propagate virus (human PBMCs for the former and H9 cells for the latter) and the cells infected (human PBMCs for the former and CEM.SS cells for the latter). Of these, there is a strong possibility that the MN virus used in the two assays, having been passaged through different cells, is critically different.

### 4. Nucleic Acid Sequence Analysis Comparison Between HTV-1 Specific Monoclonal Antibody Fabs and the Corresponding Derived Amino Acid Residue Sequence

To explore the relationship between neutralising and weakly or non-neutralizing Fabs, the variable domains of 32 clones expressing human anti-gp120 Fabs, prepared in Example 2 including the 20 listed in Figure 6 for which neutralizing activity was assessed, were sequenced.

Nucleic acid sequencing was performed on dourle-stranded DNA using Sequenase 1.0 (USB, Cleveland, OH) and the appropriate primers hybridizing to sequences in the Cg1 domain (SEQGb ; 5' GTCGTTGACCAGGCAGCCCAG 3' SEQ ID NO 49) or the Ck domain (SEQKb : 5' ATACAAGTTGTTCAGCAGGCA 3' SEQ ID NO 50). Alternatively sequencing employed single stranded DNA and the T3 primer (5' ATTAACCCTCACTAAAG 3', SEQ ID NO 51) or one hybridizing to a sequence in the Ck domain (KEF : 5' GAATTCTAAACTAGCTAGTTCG 3' SEQ ID NO 52).

The amino acid residue sequences of the variable heavy and light chains derived from the nucleic acid sequences of the 32 gp120-specific clones are shown respectively in Figures 10 and 11. Groupings are made on the basis of similarities in heavy chain sequences. Dots indicate identity with the first sequence in each section. The SEQ ID Nos are listed to the right of the corresponding derived heavy and light chain (V_{H} from SEQ ID NO 53-81 and V_{L} from SEQ ID NO 82-113) amino acid residue sequences in the Figures themselves.

Alignment of derived sequences with one another and with the Genbank database made use of the MacVector suite of programs. For analysis of heavy chain CDR3 sequences as described by Sanz, J. Immunol., 147:1720-1729 (1991), the most 5' nucleotide was considered to be the first nucleotide after codon 95 of the H chain variable region according to Kabat et al, sequences of Proteins of immunological Interest, US Dept. of Health and Human Services, Washington, DC (1991). The most 3' nucleotide was assigned to the last unidentified nucleotide before the sequence matched with the published germline JH genes. The CDR3 sequences were analyzed using the DNASTAR software. Sequence comparisons were performed with both the ALIGN and COMPARE programs in order to determine the germline D gene which provided the best homology throughout. In a second step, the SEQCOMP program was used to find sequence identity of at least six nucleotides with either the coding strand or the reverse complement of germline D genes.

### a. Organization of Antibodies into Groups According to Heavy Chain Sequence

V_{H} and V_{L} domains of 32 gp120 clones were sequenced and the V_{H} domains compared using KacVector software. This analysis immediately established that a number of the clones, including those selected by panning against different antigens, are closely related to one another. The exception to this is the Fabs selected by panning against the V3 loop peptide which are not related to the tabs selected by panning against the gp120/160 antigens. Figure 10 shows that the V_{H} sequences derived from gp120/160 panning can be organized into 7 groups. The broad features apparent from a comparison of amino acid sequences are discussed herein.

The relatedness of sequences within a group varies considerably. For instance, in the group beginning with clone number b8 the amino acid sequences are very similar. Six clones were identical and the remainder showed a maximum of 5 differences from the predominant sequence (the EQ difference due to the 5' primer excluded). Only one clone showed a single difference in the CDR3 region. The average discrepancy over all the sequences in this group from the predominant sequence is 1.1 amino acid residues/variable domain. This amount corresponds to the order of magnitude of discrepancies which could arise from the PCR. Sequencing of constant domains indicated a PCR error frequency of about 1 base change per domain.

In contrast, in the group headed by clone b3, no two clones were absolutely identical. The average difference from the consensus group sequence is 3.3 residues per sequence and determination for the CDR3 alone is 1.3. Therefore, it seems likely that the heavy chains in this group are somatic variants of one another.

The group headed by clone 1 presents a third pattern. Clones b1 and b14 are identical as are clones b2 and B2. However, 23 amino acid differences exist between the two sets of clones. Clones b24 and B30 are approximately equally well differentiated (13-25 differences) from either of these two sets of clones or one another. Still the CDR3 regions are very similar. A number of explanations can be suggested for this pattern: 1) all clones in this group originate from the same germline gene which has undergone extensive somatic mutation, 2) cross-over events have occurred to essentially recombine different germline genes with the same DJ combination, 3) a "convergent evolution" process has led to the selection of different germline genes associated with the same DJ combination.

### b. Sequences of the V_{L} Domains from the gp120 Binders

The V_{L} sequences of the Fabs were organized into the groups defined in Figure 10 are shown in Figure 11. Immediately apparent was the extensive chain promiscuity as evidenced by the pairing of different light chains with the same or a very similar heavy chain with retention of antigen binding capability and indeed, for the most part, antigen affinity as compared with Figure 10. This promiscuity can be explored further by reference to the groups considered above.

The clone b8 group, in which the heavy chain members were identical or very similar, also produced 4 light chains which are identical or very similar (less than 3 amino acid differences). Therefore a predominant heavy-light chain combination can be described for this group. One member (clone b8) had the same or very closely related V_{L} gene but appeared to use a different Jk gene. Two other members (clones B8 and b18) were more distantly related to the major sequence (7-12 differences). Two further clones (b13 and B26) used a Vk gene from a different family, Vk3 compared to Vk1, and therefore were unrelated to the major sequence.

The clone b3 group, suggested to contain somatic variants of a single heavy chain, showed considerable light chain diversity with no two members being closely related to one another. Vk3-Jk2 combinations predominated but Vk3-Jk3 and Vk1-Jk3 combinations also occurred.

On the other hand, in the clone b1 group evidence existed for the heavy chains being more choosy about their light chain partner. Thus, closely related heavy chains appeared to be paired with related light chains. The identical heavy chain pairs (b1 and b14; b2 and B2) had very similar light chains (2 and 4 amino acid differences respectively) whereas the distinct heavy chains (b24 and B30) had distinct light chains which were unrelated to one another or the other group members. The clone 4 group provides another example of this phenomenon in that 4 closely related heavy chains were paired with 3 closely related light chains (a predominant heavy-light chain combination), except for the clone b7 light chain that was distinct.

In summary, the heavy chain (V_{H}) sequences was organized into 7 groups where each member of a group has an identical or very similar CDR3 region with a limited number of differences elsewhere. When the light chains (V_{L}) were constrained into the groupings defined by their heavy chain partners, considerable light chain sequence variation was observed. This phenomenon of chain promiscuity has been observed previously and can be appreciated by reference to Figure 11. Marked neutralizing ability was confined to two groups of sequences. The first group consisted of Fabs 4, 7, 12 and 21 which have very similar heavy and light chains. The second group consisted of Fabs 13, 8, 18, 22 and 27. Only Fab 13 showed marked neutralizing ability, although the others showed some weaker activity. Interestingly in this group Fab 13 did have a light chain distinct from the other members of the group.

### 5. Shuffling of the Heavy and Light Chain of a Single Clone Against the Library

To further explore possible functional heavy-light chain combinations, the heavy chain of clone b12 (also referred to as Fab 12 for the corresponding soluble Fab preparation) shown in Figure 10 was recombined with the original light chain library prepared in Example 2 to construct a new library H12-LCn. In addition, the b12 light chain was recombined with the original heavy chain library to construct a library Hn-L12. These two libraries were taken through 3 rounds of panning against gp120 (IIIB) as described in Example 2b5). The Fabs expressed from the resultant immunoreactant clones were analyzed as described in Example 3 above. Clone b12 was chosen as this Fab neutralized HIV-1 in vitro as shown in Example 3.

To accomplish the preparation of a shuffled library from the Fd gene of clone b12 with the original light chain library, the b12 heavy chain was first subcloned into a tetanus toxoid binding clone expressed in pComb2-3. The light chain library was then cloned into this construction to give a library of 1 x 10⁷ members. The subcloning step was used to avoid contamination with and over-representation of the original light chain. A similar procedure was adopted for shuffling of heavy chains against the light chain from clone b12 to give a library of 3 x 10⁶ members. Cloning and panning procedures were carried out as described above for the original library.

Eleven light chains which recombined with the b12 heavy chain and bound gp120 by panning were randomly chosen for subsequent competition ELISA and sequence analysis. The apparent affinities of these shuffled combinations were similar with an IC₅₀ of approximately 10⁻⁸ to 10⁻⁹ M. The sequences were organized where a set of 3 were very similar to the original b12 light chain and the other 8 showing many differences from the original with some sub-grouping possible.

The sequences of the light chains which bound to the b12 heavy chain clone are shown in Figure 12. The sequences are compared to the sequence for the original light chain from clone b12. The light chains are identified by numbers which do not correspond to the original light chain clones; the assigned numbers of the newly selected clones having new light chains are thus arbitrary. The sequences of these light chains are also listed in the Sequence Listing from SEQ ID NO 114 to 122. Some light chain sequences are identical. In addition to immunoreactivity with gp120, the new Fabs isolated from these shuffled clones were tested in the syncytia assay for neutralization of HIV-1 infection as described in Example 3. Four shuffled monoclonal Fab antibodies, each having the heavy chain from clone b12, a known HIV-1 neutralizing clone, and new light chains designated L28, L25, L26 and L22, all exhibited approximately 60% neutralization in a syncytia assay with 0.4 µg/ml purified Fab. This effect was equivalent to that obtained with the original clone b12 heavy and light chain pair. Maximum neutralization of approximately 80% was obtained with the H12/L28 and H12/L25 Fabs at 0.7 µg/ml which was equivalent to that seen with the original clone b12 heavy and light pair. The neutralization resulting from the H12/L22 and H12/L26 Fabs plateaued at 60% with Fab concentrations of 0.4 µg/ml up to 1.0 µg/ml. Thus, in addition to the gp120 immunoreactive and HIV neutralizing Fabs obtained in the original library prepared as described in Example 2, by shuffling a known neutralizing heavy chain with a library of light chains, new HIV-1 neutralizing Fab monoclonal antibodies have been obtained.

Ten heavy chains which recombined with the b12 light chain were also randomly chosen. One was very similar to the original b12 heavy chain but the others have many differences. Nevertheless, the V-D and D-J junctions were essentially identical indicating the clones had probably arisen from the same rearranged B-cell clone by somatic modification. Competition ELISA failed to reveal any clear difference in affinity between the variants selected from those originally analyzed.

The sequences of the heavy chains which bound to the b12 light chain clone are shown in Figure 13. The sequences are compared to the sequence for the original heavy chain from clone b12. The heavy chains are identified by numbers which do not correspond to the original light chain clones; the assigned numbers of the newly selected clones having new heavy chains are thus arbitrary. The sequences of these light chains are also listed in the Sequence Listing from SEQ ID NO 123 to 132. Some light chain sequences are identical. In addition to immunoreactivity with gp120, the new clones were tested in the syncytia assay for neutralization of HIV-1 infection as described in Example 3. Two shuffled monoclonal Fab antibodies, each having the light chain from clone b12, a known HIV-1 neutralizing clone, and new heavy chains designated H2 and H14, exhibited approximately 40% neutralization in a syncytia assay with 1.0 and 0.5 µg/ml purified Fab, respectively. This effect was equivalent to that obtained with the original clone b12 heavy and light chain pair at a concentration of 2 µg/ml. Maximum neutralization of approximately 50% was obtained with the Fab having the new H14 chain at 1.0 µg/ml compared to 80% neutralization with 0.7 µg/ml with the original clone b12 heavy and light pair. Thus, in addition to the gp120 immunoreactive and HIV neutralizing Fabs obtained in the original library prepared as described in Example 2, by shuffling a known neutralizing light chain with a library of heavy chains, new HIV-1 neutralizing Fab monoclonal antibodies have been obtained.

Thus, this shuffling process revealed many more heavy and light chain partners that bound to gp120 that were equal in affinity to those obtained from the original library prepared in Example 2. With this approach, additional HIV-1 neutralizing antibodies can easily be obtained over those present in an original library. The complexity of the clones arising from the heavy chain shuffling also suggests that this approach may be used to map the course of somatic diversification.

Combinatorial libraries randomly recombine heavy and light chains so to what extent antibodies derived from such libraries represent those produced in a response in vivo can be determined. In principle, a heavy-light chain combination binding antigen could arise fortuitously, i.e., neither chain is involved in binding antigen in vivo but the combination does bind antigen in vitro.

The available data suggests, however, that heavy chains, from immune libraries, involved in binding antigen tightly in vitro arise from antigen-specific clones in vivo. First, studies have generally failed to identify high-affinity binders in non-immunized IgG libraries. See, Persson et al. Proc. Natl. Acad. Sci., USA, 88:2432-2436 (1991) and Marks et al. Eur. J. Immunol., 21:985-991 (1991).

Further, as described above, gp120 binders were not observed in panning a bone marrow IgG library from an HIV seronegative donor against gp120. Second, heavy chains associated with binders from immunized libraries were typically at relatively high frequency in the library indicating they were strongly represented in the mRNA isolated from immunized animals. See, Caton et al., Proc. Natl. Acad. Sci., USA, 87:6450-6454 (1990) and Persson et al., supra. Third, heavy chains from immunized libraries appeared to dictate specificity when recombined with various unrelated light chains as described in Example 6. Fourth, the isolation of intraclonal heavy chain variants as here indicated that an active antibody response was cloned. Thus, the shuffling of a known heavy chain with a light chain binder and vice versa is preferred for use in this invention as new neutralizing Fabs can be obtained beyond those generated in vivo.

Heavy chain promiscuity, i.e., the ability of a heavy chain to pair with different light chains with retention of antigen affinity, presents serious problems for identifying in vivo light chain partners. This applies not only to the strict definition of partners as having arisen from the same B-cell but also to one which would encompass somatic variants of either partner. The existence of predominant heavy-light chain combinations, particularly involving intraclonal light chain variants, suggests that the light chains concerned are well represented in the library and probably are associated with antigen binding in vivo. However, promiscuity means that, although some combinations probably do occur in vivo, one cannot be certain that one is not shuffling immune partner chains in the recombination. For instance, the occurrence of a virtually identical light chain (b6, B20) in 2 out of 33 clones suggests that it is probably over-represented in the library consistent with an in vivo involvement in antigen-stimulated clones. However, there is no way of knowing whether the in vivo partner of the light chain is the b6 or B20 heavy chain or indeed another heavy chain arising from a stimulated clone.

The light chains arising from the combinatorial library may not be those employed in vivo. Nevertheless it is interesting to note that some heavy chains appear relatively choosy about light chain partner whereas others appear almost indifferent.
This observation needs to be tempered by the finding that apparently choosy heavy chains from this analysis will accept diverse light chains with maintenance of antigen binding in a binary plasmid system where pairings are forced as shown below in Example 6 rather than selected in a competitive situation.

Two reports compare heavy-light chain combinations arising from combinatorial libraries and hybridomas in immunized mice. The library approach begins with mRNA and is therefore probably reflecting plasma cell populations. In contrast, hybridomas are thought to reflect activated but not terminally differentiated B cell populations and EBV transformation to reflect resting B cell populations.

Whatever the arguments about light chain authenticity, the heavy chains of Figure 10 present many features of interest. The most frequently used heavy chain is of the clone b8 type. It could be argued that this usage simply represents bias in PCR amplification. However, the occurrence of approximately equal numbers of clones in this group amplified by VH1a and VH3a primers argues against this notion. Furthermore, the existence of intraclonal variants in some groups indicates that one is at least sampling different genes from the initial library.

The antibodies cloned here do bear qualitative relationship with the polyclonal antibodies present in the serum of the asymptomatic donor. The titer of anti-gp120 (IIIB) antibodies was approximately 1:3000, with greater than 50% of the reactivity being inhibited by CD4 or a cocktail of Fabs from clones 12, 13 and 14. The titer of anti-gp120 (SF2) antibodies was approximately 1:800. Further, the titer of serum against the short constrained V3 loop peptide was 1:500 and against the full length MN V3 loop peptide was only 1:300. The importance of "anti-CD4 site antibodies" seems general in donors with longer term HIV infection in that the cocktail of Fabs 12, 13 and 14 was able to inhibit binding of a large fraction of serum antibody reactivity with gp120 (IIIB) in 26 of 28 donors tested.

The ability of Fabs to neutralize viruses has been a controversial area. One of the problems has been that Fabs are classically generated by papain digestion of IgG. If the Fab, as is often the case, shows reduced activity relative to the parent IgG then it may be difficult to rule out IgG contamination in the Fab preparation. Recombinant Fabs, however, as shown herein definitively neutralize virus.

The mechanism of neutralization of HIV-1 appears to neither require virion aggregation nor gp120 cross-linking. In addition, there is no correlation with blocking of the CD4-gp120 interaction to neutralization. The existence of the cloned neutralizing Fabs of this invention should allow the molecular features that confer neutralizing potential to be explored. For instance, in the case of the group of clones containing Fab 13, the unique character of the light chain of that neutralizing clone suggests that chain shuffling experiments in which the 13 light chain was recombined with the other heavy chains in that group, might be revealing. Heavy chains paired with two dissimilar light chains have been shown to retain antigen affinity but exhibit altered fine specificity as shown in Example 6.

The observation here of a large number of Fabs with only a limited number being strongly neutralizing may have important consequences. If the pattern is repeated for whole antibodies then it would seem that much of the gp120 structure may be in a sense a "decoy", i.e., the immune system may invest considerable effort in producing antibodies of high affinity but limited anti-viral function. To exacerbate the situation the ineffective antibodies may bind to gp120 and inhibit the binding of strongly neutralizing antibodies. This has obvious consequences for vaccination which should be primarily designed to elicit neutralizing antibodies of this invention.

### 6. Shuffling of Selected Heavy and Light Chain DNA Sequences of a Combinatorial Library in a Binary Plasmid System

A binary system of replicon-compatible plasmids has been developed to test the potential for promiscuous recombination of heavy and light chains within sets of human Fab fragments isolated from combinatorial antibody libraries. The efficiency of the system is demonstrated for the combinatorial library of this invention derived from the bone marrow library of an asymptomatic HIV donor.

### a. Construction of the Binary Plasmid system

The binary plasmids pTAC01H and pTC01 for use in this invention contain the pelB leader region and multiple cloning sites from Lambda Hc2 and Lambda Lc3, respectively, and the set of replicon-compatible expression vectors pFL281 and pFL261. Both pFL281 and pFL261 have been described by Larimer et al., Prot. Eng., 3:227-231 (1990), the disclosure of which is hereby incorporated by reference. The nucleotide sequences of pFL261 and pFL281 are in the EMBL, GenBank and DDBJ Nucleotide Sequence Databases under the accession numbers M29363 and M68946. The plasmid pFL281 is based on the plasmid pFL260 also described by Larimer et al., supra, and having the accession number M29362. The only distinction between the plasmids pFL260 and pF1281 is that pFL281 lacks a 60 bp sequence of pFL260 between the Eag I site and the Xma III site resulting in the loss of one of the two BamH I sites. This deletion is necessary to allow for cloning of the BamH I Hc2 fragment into the expression vector as described herein.

The replicon-compatible expression vectors share three common elements: (i) the f1 single-stranded DNA page intergenic IG regions; (ii) the tightly regulated tac promoter and lac operator; and (iii) an rbs-ATG region with specific cloning sites. The plasmid vectors differ in their antibiotic resistance markers and plasmid replicons: pFL261 carries a gene encoding chloramphenicol acetyltransferase (cat), conferring chloramphenicol resistance,a nd the p15A replicon; pFL281 carries a gene encoding beta-lactamase (bla), conferring ampicillin resistance, and the ColE1 replicon (ori) from pMB1. The p15A and ColE1 replicons permit the coincident maintenance of both plasmids in the same E. coli host.

The Hc2 and Lc2 vectors prepared in Examples 1a2) and 1a3), respectively, were converted into the plasmid form using standard methods familiar to one of ordinary skill in the art and as described by Sambrook et al., Molecular Cloning: A Laboratory Manual, 2nd ed., Cold Spring Harbor Laboratory Press, New York (1989) and subsequently digested with Xho I-Spe I (pHc2) and Sac I-Xba I for (pLc2). The synthetic linkers for insertion into the digested pHc2 and Lc2 plasmids were prepared by American Synthesis. The linkers were inserted to increase the distance between cloning sites so as to increase the effectiveness of the digestions. The 5' and 3' linkers for preparing the double-stranded linker insert into pHc2 were 5' TCGAGGGTCGGTCGGTCTCTAGACGGTCGGTCGGTCA 3' (SEQ ID NO 133) and 5' CTAGTGACCGACCGACCGTCTAGAGACCGACCGACCC 3' (SEQ ID NO 134), respectively. The 5' and 3' linkers for preparing the double-stranded linker insert into pLc2 were 5' CGGTCGGTCGGTCCTCGAGGGTCGGTCGGTCT 3' (SEQ ID NO 135) and 5' CTAGAGACCGACCGACCCTCGAGGACCGACCGACCGAGCT 3' (SEQ ID NO 136), respectively. The pairs of linker oligonucleotides were separately ligated to their respective digested, calf intestinal phosphatase-treated vectors.

Subsequently, the multiple cloning sites of pHc2 and pLc2 were transferred into the expression vectors, pFL281 and pFL261, respectively. To accomplish this process, the multiple cloning regions of both Lc2 and Hc2 were separately amplified by PCR as described by Gram et al., Proc. Natl. Acad. Sci., USA, 89:3576-3580 (1992) and as described in Example 2b using Vent Polymerase (New England Biolabs) according to the manufacturer's recommendations. The forward primer, 5' CAAGGAGACAGGATCCATGAAATAC 3' (SEQ ID NO 137) was designed to provide a flush fusion of the pelB leader sequence to the ribosome binding sites of the cloning vectors pFL261 and pFL281 via its internal BamH I site indicated by the underlined nucleotides. The reverse primer 5' AGGGCGAATTGGATCCCGGGCCCCC 3' (SEQ ID NO 138) was designed to anneal downstream of the region of interest in the parent vector of pHc2/pLc2 and create a second BamH I site. The resultant Hc2 and Lc2 PCR amplification products were then digested with BamH I to provide for BamH I overhangs for subsequent ligation into BamH I linearized pFL281 and pFL261 vectors, respectively. The resulting light chain vector containing the Lc2 insert, designated pTC01, was used in this form, whereas the heavy chain vector was further modified with a histidine tail to allow purification of Fab fragments by immobilized metal affinity chromatography as described by Skerra et al., Bio/Technology, 9:273-278 (1991). For this purpose, the synthetic linker oligonucleotides, respectively the 5' and 3' linkers, 5' CTAGTCATCATCATCATCATTAAGCTAGC 3' (SEQ ID NO 139) and 5' CTAGGCTAGCTTAATGATGATGATGATGA '3 (SEQ ID NO 140) was inserted into the Spe I site, in effect removing the decapeptide tag sequence to generate the heavy chain vector designated as pTAC01H. The expression of Fab fragment in all subsequent cloning experiments was suppressed by adding 1% (w/v) glucose to all media and plates.

### b. Construction of Expression Plasmids

For expression of the light chain variable domain, pTC01 prepared above was first digested with Sac I and Xba I; individual light chain inserts were then obtained by separately digesting 22 of the pComb2-3 plasmids prepared and screened as described in Example 2 and listed in Figure 7 that bind to gp120 with the same combination of enzymes and isolating the 0.7 kb fragment using low melting point agarose gel electrophoresis followed by b-agarose digestion. For the chain-shuffling experiments, the following representative members of each of the seven groups shown in Figure 7 were chosen: b11; b6; b4-b12-b7-b21; b3; s8; b1-b14-b24; b13-b22-B26-b8-b18-b27-B8-B35-s4; and one loop peptide-binding clone, p35. The different groups are indicated by semicolon separations while members of the same group are dashed. The resultant isolated light chains were separately ligated into PTC01 overnight at 16°C under standard conditions using a 5:1 molar insert-to-vector ratio to form 21 light chain pTC01 expression vectors.

For expression of the heavy chain variable domain, pTAC01H prepared above was first digested with Xho I and Spe I; heavy chain inserts were then obtained by separately PCR amplification reactions of the 20 pComb2-3 plasmids from which light chain inserts were obtained. PCR was used to isolate the heavy chain inserts instead of restriction digestion in order to obtain heavy chain without the cpIII gene anchor sequence in the vector. For the PCR reaction, the respective 5' and 3' primers, 5' CAGGTGCAGCTCGAGCAGTCTGGG 3' (VH1a) (SEQ ID NO 42) and 5' GCATGTACTAGTTTTGTCACAAGATTTGGG 3' (CGlZ) (SEQ ID NO 44) were used to amplify the region corresponding to the heavy chain as described in Examples 2a1) and 2a2). The resultant PCR products were purified by low-melting point electrophoresis, digested with Xho I and Spe I, re-purified, and separately ligated to the similarly prepared heavy chain pTAC01H vector using a 1:2 molar vector-to-insert ratio to form 21 heavy chain pTAC01H expression vectors.

### c. Co-transformation of Binary Plasmids

CaCl₂-competent XL1-Blue cells (Stratagene; recAl, endA1, gyrA96, thi, hsdR17, supE44, relA1, lac, {F' proAB, lacl^{q}, ZDM15, Tn10(tet^{R})}} were prepared and transformed with approximately 0.5 µg purified DNA of each plasmid in directed crosses of each of the 20 light chain vectors with each of the 20 heavy chain vectors. The presence of both plasmids and the episome was selected for by plating transformants on triple-antibiotic agar plates (100 µg/ml carbenicillin, 30 µg/ml chloramphenicol, 10 µg/ml tetracycline, 32 g/l LB agar) containing 1% glucose.

A binary plasmid system consisting of two replicon-compatible plasmids was constructed as shown in 14. The pTAC01H heavy chain vector schematic is shown in Figure 14A and the pTCO1 light chain vector schematic is shown in Figure 14B. Both expression vectors feature similar cloning sites including pel B leader sequences fused to the ribosome binding sites and the tac promoters via BamH I sites as shown in Figure 15. The nucleotide sequences of the multiple cloning sites along with the tac promoter, ribosome binding sites (rbs) and the underlined relevant restriction sites for the light chain vector, pTCO1, and heavy chain vector, pTACO1H are respectively shown in Figure 15A and Figure 15B. The sequences are also listed in the Sequence Listing as described in the Brief Description of the Drawings. The heavy chain vector pTAC01H also contains a (His)₅-tail to allow purification of the recombinant Fab fragments by immobilized metal affinity chromatography. The presence of both plasmids in the same bacterial cell is selected for by the presence of both antibiotics in the media. Expression is partially suppressed during growth by addition of glucose and induced by the addition of IPTG at room temperature. Under these conditions, both plasmids are stable within the cell and support expression of the Fab fragment as assayed by ELISA using goat anti-human kappa and goat anti-human IgG1 antibodies.

### d. Preparation of Recombinant Fab Fragments

Bacterial cultures for determination of antigen-binding activity were grown in 96 well-tissue culture plates (Costar #3596). 250 µl Superbroth [SB had the following ingredients per liter: 10 g 3-(N-morpholino) propanesulfonic acid, 30 g tryptone, 20 g yeast extract at pH 7.0 at 25°C) containing 30 µg/ml chloramphenicol, 100 µg/ml carbenicillin, and 1% (w/v)] glucose were admixed per well and inoculated with a single double-transformant prepared in Example 6c above. The inoculated plates were then maintained with moderate shaking (200 rpm) on a horizontal shaker for 7-9 hours at 37°C, until the A₅₅₀ was approximately 1-1.5. The cells were collected by centrifugation of the microtiter plate (1,500 X g for 30 minutes at 4°C), the supernatants were discarded, and the cells were resuspended and induced overnight at room temperature in fresh media containing 1 mM IPTG, but no glucose. Cells were harvested by centrifugation, resuspended in 175 µl PBS (10 mM sodium phosphate, 160 mM NaCl at pH 7.4 at 25°C) containing 34 µg/ml phenylmethylsulfonyl fluoride (PMSF) and 1.5% (w/v) streptomycin sulfate, and lysed by 3 freeze-thaw cycles between -80°C and 37°C. The resultant crude extracts were partially cleared by centrifugation as above before analysis by antigen-binding ELISA.

### e. Assay and Determination of Relative Affinities

Relative affinities were determined as described in Example 2b6) after coating wells with 0.1 µg of antigen. The selected antigens included tetanus toxoid and recombinant gp120 (strain IIIB) and gp120 (strain SF2). For each antigen, a negative control extract of XL1-Blue cells co-transformed with pTC01 and pTAC01H was tested to determine whether other components in E. coli had any affinity for the antigens in the assay. Each extract was assayed for BSA-binding activity and BSA-positive clones were considered negative. All possible single-transformants expressing one chain only were prepared as described for the double-transformants and were found to have no affinity for any of the antigens used. Because of the nature of the assay, whether this was due to a lack of binding by the individual chains itself or due to a lack of expression or folding could not be determined.

### f. Results of Direct Crosses of Heavy and Light Chains within a Set of gp120/gp160 Binding Antibodies

The Fab fragments derived from the bone marrow of the same asymptomatic HIV donor but panned against gp120 (IIIB), gp160 (IIIB), and gp120 (SF2), were assigned to one of seven groups based on the amino acid sequences of the CDR3 of their heavy chains as described in Example 4. From the same library, antibodies to the constrained hypervariable v3-loop-like peptide JSISIGPGRAFYTGZC (SEQ ID NO 141) were isolated. For the chain-shuffling experiments, the following representative members of each of the seven groups shown in Figure 7 were chosen: b11; b6; b4-b12-b7-b21; b3; s8; b1-b14-b24; b13-b22-B26-b8-bl8-b27-B8-B35-s4; and one loop peptide-binding clone, p35. Clones b4, b7, b12, and b21 showed neutralization activity against HIV when monitoring inhibition of infection by syncytia formation and clones b13, b12, and b4 when monitoring p24 production as shown in Example 3. Light and heavy chains were cloned from the original constructs and cotransformed in all possible binary combinations into XL1-Blue cells as described above.

The results of the complete cross are shown in Figure 16. As is to be expected, identical chains derived from different Fab fragments had similar binding properties e.g., b18HC, b27HC, B8HC, B35HC, s4HC. The crosses of the original heavy chains with the original light chains in each case clearly recapitulated binding activity. Minor differences existed between some heavy chains with identical variable domain sequences, e.g., b4 and b12 (constant domains were not sequenced for any of the constructs). The exception is b8HC, which was identical in its variable domain to b18HC, b27HC, B8HC, B35HC, s4HC, yet shows more cross reactivity. Presumably, this is due to differences in expression levels in the cell or differences in the constant domain sequences. Clear differences existed between heavy chains in their tendency to accept different light chains and still bind antigen, but even the least promiscuous heavy chain in the set panned against gp120 (IIIB), b1HC, still did so in 43% of its crosses. On the other side of the spectrum, 5 heavy chains, b11HC, b6HC, b12HC, b7HC, and b8HC, crossed productively with all light chains in this set. For the heavy chain crosses examined in detail (all of s4HC, B35HC, B26HC; most of b12HC, b12HC), no significant differences in apparent binding affinity were found between Fab fragments using the same heavy chain but different light chains as shown in Figure 17 where the IC₅₀ from competition with soluble gp120 (IIIB) was approximately 10⁻⁸ M.

Within the original seven groups that were established according to the sequence of the CDR3 of the heavy chains and that are indicated by horizontal and vertical lines in Figure 16, complete promiscuity was present, i.e., heavy and light chains within these CDR3-determined groups were completely promiscuous with each other. However, there was a lack of promiscuity between other groups, e.g., between b1HC-b24HC and b13LC-s4LC. In the analysis of these sequence-based groups, the protein antigen against which the phage display library was panned was not a critical factor. The exception to this case was the cross of p35HC with all light chains; the only cross that bound either to gp120 (SF2 strain) or the original antigen, the loop peptide, was the cross containing the original heavy and light chains.

Unlike the heavy chains, no light chains crossed productively with all heavy chains nor were any distinguishable from the other light chains by unusually low promiscuity.

In the neutralization assays performed as described in Example 3, the directed cross resulting from the pairing of the heavy chain from clone b12 with the light chain from clone b21, was effective at neutralizing HIV-1.

### g. Interantigenic Crosses of Heavy and Light Chains

To determine whether conclusions derived from the crosses between high affinity Fab fragments originating from the same library can be extended to unrelated libraries, a non-related gamma1k-Fab fragment (P3-13) specific for tetanus toxoid from a different donor was chosen for a new set of crosses (clone 3 in Persson et al., Proc. Natl. Acad. Sci., USA, 88:2432-2436 (1991)]. Extracts were probed with tetanus toxoid or with gp120 (IIIB). The data confirm the results from the gp120 cross experiment in that the binding activity towards the antigen was determined by the heavy chain. The heavy chain of clone P3-13 paired with the light chains b4, b12, b21, and b14 to yield an Fab fragment with an affinity towards tetanus toxoid; the light chain of P3-13 paired with the heavy chains of b3, b6, b11, and b14 to yield an Fab fragment with an affinity towards gp120 (IIIB). None of the light chains originating from the gp120 binders was able to confer gp120 specificity in combination with the P3-13 heavy chain.

Similarly, the P3-13 light chain was unable to generate tetanus toxoid specificity in combination with any of the heavy chains originating from the gp120 binders, confirming the dominance of the heavy chain in the antibody-antigen interaction. Interestingly, all three light chains that showed a strong signal against tetanus toxoid (b4, b12, b21) were members of the same group when sorted by the CDR3's of their original heavy chains. As might be expected from crosses between unrelated libraries, not only was there a lower degree of promiscuity, i.e., chains paired productively with far fewer complementary chains, but the range of apparent affinity constants determined by competition ELISA was much broader (6.3 X 10⁶ - 6.3 X 10⁻⁸ M). The replacement of the original P3-13 light chain in the P3-13 Fab fragment with another light chain lowered the affinity of the Fab towards tetanus toxoid 10 to 100-fold (from 6.3 X 10⁻⁸ M to 6.3 X 10⁻⁶ M). In the crosses of the light chain of P3-13 with all the heavy chains of the HIV pannings, the productive crosses had similar affinities to gp120 (IIIB) (2.5 X 10⁷ - 6.3 X 10⁻⁷ M), with the exception of b14HC/P3-13LC, whose signal was too weak for a definite determination of the apparent binding constant. These affinities were approximately five-fold lower than those of the gp120-heavy chains with their original light chains.

Thus, the results show that chain shuffling is yet another maneuver allowed in vitro but not in vivo which can be expected to help extend antibody diversity beyond that of Nature. The overriding feature of the binary system of this invention is its ability to create large numbers (several hundred) of directed crosses between characterized light and heavy chains without the need for recloning individual chains for each cross after the initial vector construction. When used in combination with the phage-display method and biological assays, it allows the rapid analysis of the most interesting subset of the pool of antigen-binding clones by chain shuffling, with the aim of finding biologically or chemically active antibodies. For the set of antigens studied here, most heavy chains recombined with a number of light chains to yield an antigen-binding Fab fragment.

These results have important implications for the diversity of combinatorial antibody libraries. While it is not possible to predict reliably the original in vivo combinations of light and heavy chains due to the surprising promiscuity of individual chains, recombinant antibody libraries take advantage of the fact that even distantly related Fabs against the same antigen can recombine in vitro to give chain combinations not found in vivo. In fact, after the identification of a certain number of antibodies that have been shown to possess some biological or chemical activity, it may be better to shuffle their individual chains in a directed fashion than to continue sampling randomly from the same pool of binders. By extension, the promiscuity observed in this system indicates that in libraries constructed using degenerate, chemically synthesized oligonucleotides, there should be considerable flexibility in which separate synthetic heavy chains can pair with separate synthetic light chains to generate separate antigen-binding Fab fragments. The diversity of combinatorial libraries coupled with chain-shuffling should allow wide exploration of three dimensional space thereby solving the problem of how to approximate molecules in the ternary complex of antibody, substrate and cofactor.

### 7. Deposit of Materials

The following cell lines have been deposited on September 30, 1992, with the American Type Culture Collection (ATCC), 1301 Parklawn Drive, Rockville, MD, USA:

| Cell Line | ATCC Accession No. |
|---|---|
| E. coli MT11 | ATCC 69078 |
| E. coli MT12 | ATCC 69079 |
| E. coli MT13 | ATCC 69080 |

The deposits listed above, MT11, MT12 and MT13 are bacterial cells (E. coli) containing the expression vector pComb2-3 for the respective expression of the Fabs designated b11 (clone b11), b12 (clone b12), and b13 (clone b13) prepared in Example 2b. The sequences of the heavy and light chain variable domains are listed in Figure 10 and 11, respectively. This deposit was made with the ATCC under the provisions of the Budapest Treaty on the International Recognition of the Deposit of Microorganisms for the Purpose of Patent Procedure and the Regulations thereunder (Budapest Treaty). This assures maintenance of a viable culture for 30 years from the date of deposit. The organisms will be made available by ATCC under the terms of the Budapest Treaty which assures permanent and unrestricted availability of the progeny of the culture to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. §122 and the Commissioner's rules pursuant thereto (including 37 CFR §1.14 with particular reference to 886 OG 638). The assignee of the present application has agreed that if the culture deposit should die or be lost or destroyed when cultivated under suitable conditions, it will be promptly replaced on notification with a viable specimen of the same culture. Availability of the deposited strain is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the cell lines deposited, since the deposited embodiment is intended as a single illustration of one aspect of the invention and any cell lines that are functionally equivalent are within the scope of this invention. The deposit of material does not constitute an admission that the written description herein contained is inadequate to enable the practice of any aspect of the invention, including the best mode thereof, nor is it to be construed as limiting the scope of the claims to the specific illustration that it represents. Indeed, various modifications of the invention in addition to those shown and described herein will become apparent to those skilled in the art from the foregoing description and fall within the scope of the appended claims.

## Claims

1. A human monoclonal antibody capable of immunoreacting with human immunodeficiency virus (HIV) glycoprotein gp120 and neutralizing HIV, wherein the monoclonal antibody has the binding specificity of a monoclonal antibody comprising a heavy chain immunoglobulin variable region amino acid residue sequence selected from the group consisting of SEQ ID NOs 66, 67, 68, 70, 72, 73, 74, 75, 78 and 79, and conservative substitutions thereof.

2. The human monoclonal antibody of claim 1 wherein the monoclonal antibody has the binding specificity of a monoclonal antibody having heavy and light chain imnunoglobulin variable region amino acid residue sequences in pairs selected from the group consisting of SEQ ID NOs 66:95, 67:96, 72:102, 66:97, 73:107, 74:103, 70:101, 68:98, 75'104 72:105, 78:110, 66:118, 66:122, 66:121, 79:124, 79:132 and 66:98, and conservative substitutions thereof.

3. The human monoclonal antibody of claim 1, wherein the monoclonal antibody has the binding specificity of a monoclonal antibody produced by ATCC 69078, ATCC 69079 or ATCC 69080.

4. The human monoclonal antibody of claim 3, wherein the monoclonal antibody is the monoclonal antibody produced by ATCC 69078, ATCC 69079 or ATCC 69080.

5. A human monoclonal antibody capable of immunoreacting with human immunodeficiency virus (HIV) glycoprotein gp120 and neutralizing HIV, wherein the monoclonal antibody has the binding specificity of a monoclonal antibody comprising a light chain immunoglobulin variable region amino acid residue sequence selected from the group consisting of SEQ ID NOs 95, 96, 97, 98, 101, 102, 103, 104, 105, 107, 110, 115, 118, 121, 122, 124 and 132, and conservative substitutions thereof.

6. A polynucleotide sequence encoding a heavy chain immunoglobulin variable region amino acid residue sequence portion of a human monoclonal antibody capable of innunoreacting with human immunodeficiency virus (HIV) glycoprotein gp120 and neutralizing HIV, wherein the monoclonal antibody has the binding specificity of a monoclonal antibody comprising said heavy chain immunoglobulin variable region amino acid residue sequence selected from the group consisting of SEQ ID NOs 66, 67, 68, 70, 72, 73, 74, 75, 78 and 79, and conservative substitutions of the amino acid residue sequence, and polynucleotide sequences complementary thereto.

7. The polynucleotide sequence of claim 6, wherein the polynucleotide is DNA.

8. A polynucleotide sequence encoding a light chain immunoglobulin variable region amino acid residue sequence portion of a human monoclonal antibody capable of immunoreacting with human immunodeficiency virus (HIV) glycoprotein gp120 and neutralizing HIV, wherein the monoclonal antibody has the binding specificity of a monoclonal antibody comprising said light chain immunoglobulin variable region amino acid residue sequence selected from the group consisting of SEQ ID NOs 95, 96, 97, 98, 101, 102, 103, 104, 105, 107, 110, 115, 118, 121, 122, 124 and 132, and conservative substitutions of the amino acid residue sequence, and polynucleotide sequences complementary thereto.

9. A host cell comprising the polynucleotide sequence of claim 6 or claim 8.

10. A DNA expression vector comprising the polynucleotide sequence of claim 6 or claim 8.

11. A method of detecting human immunodeficiency virus (HIV) in vitro comprising contacting a sample suspected of containing HIV with a diagnostically effective. amount of the monoclonal antibody of claim 1 or claim 5, and determining whether the monoclonal antibody immunoreacts with the sample.

12. The method of claim 11, wherein the monoclonal antibody is detectably labelled with a label'selected from the group consisting of a radioisotope, a fluorescent compound, a colloidal metal, a chemiluminescent compound, a bioluminescent compound, and an enzyme.

13. The method of claim 11, wherein the monoclonal antibody is bound to a solid phase.

14. The use of a monoclonal antibody according to claim 1 or claim 5 in the manufacture of a medicament for providing passive immunotherapy to human immunodeficiency virus (HIV) disease in a human.

15. The use according to claim 14, wherein the passive immunotherapy is provided prophylactically.

16. The use according to claim 14, wherein said immunotherapy comprises administering said monoclonal antibody parenterally.

17. The use according to claim 16, wherein the parenteral administration is by subcutaneous, intramuscular, intraperitoneal, intracavity, transdermal, or intravenous injection.

18. The use according to claim 16, wherein the parenteral administration is by gradual perfusion.

19. The use according to claim 18, wherein the gradual perfusion is by intravenous or peristaltic means.

20. The use according to claim 16, wherein said immunotherapy comprises parenteral administration of from about 0.1 mg/kg to about 300 mg/kg.

21. The use of an anti-idiotype antibody to the monoclonal antibody of claim 1 or claim 5, in the manufacture of a medicament for inducing active immunotherapy to human immunodeficiency virus (HIV) disease in a human.

22. A pharmaceutical composition comprising at least one dose of an immunotherapeutically effective amount of the monoclonal antibody of claim 1 or claim 5 in a pharmacological carrier.

23. The pharmaceutical composition of claim 22 wherein said composition contains two or more different monoclonal antibodies.

24. A kit useful for the detection of human immunodeficiency virus (HIV) in a source suspected of containing HIV, the kit comprising carrier means being compartmentalized to receive in close confinement therein one or more containers comprising a container containing the monoclonal antibody of claim 1 or claim 5.

## Patentansprüche

1. Humaner monoklonaler Antikörper, der fähig ist mit humanem Immundefekt Virus (HIV) Glykoprotein gp120 eine Immunreaktion einzugehen und HIV zu neutralisieren, wobei der monoklonale Antikörper die Bindungsspezifität eines monoklonalen Antikörpers besitzt, der eine Sequenz von Aminosäurenresten aus der variablen Region der schweren Kette von Immunglobulin umfasst, ausgewählt aus der Gruppe bestehend aus SEQ ID NO's: 66, 67, 68, 70 ,72, 73, 74, 75, 78 und 19, sowie konservativen Substitutionen davon.

2. Der humane monoklonale Antikörper gemäß Anspruch 1, wobei der monoklonale Antikörper die Bindungsspezifität eines monoklonalen Antikörpers besitzt, der Sequenzpaare von Aminosäurenresten aus der variablen Region der leichten und der schweren Kette von Immunglobulin umfasst, ausgewählt aus der Gruppe bestehend aus SEQ ID NO's: 66:95, 67:96, 72:102, 66:97, 73:107, 74:103, 70:101, 68:98, 75:104, 72:105, 78:110, 68:118, 66:122, 66:121, 66:115, 19:124, 19:132 und 66:98, sowie konservativen Substitutionen davon.

3. Der humane monoklonale Antikörper gemäß Anspruch 1, wobei der monoklonale Antikörper die Bindungspezifität eines monoklonalen Antikörpers besitzt, der von ATCC 69078, ATCC 69079 oder ATCC 69080 produziert wurde.

4. Humaner monoklonaler Antikörper gemäß Anspruch 3, wobei der Antikörper der Antikörper ist, der von ATCC 69078, ATCC 69079 oder ATCC 69080 produziert wurde.

5. Humaner monoklonaler Antikörper, der fähig ist mit humanem Immundefekt Virus (HIV) Glykoprotein gp120 eine Immunreaktion einzugehen und HIV zu neutralisieren, wobei der monoklonale Antikörper die Bindungsspezifität eines monoklonalen Antikörpers besitzt, der eine Sequenz von Aminosäurenresten aus der variablen Region der leichten Kette von Immunglobulin umfasst, ausgewählt aus der Gruppe bestehend aus SEQ ID NO's; 95, 96, 97, 98, 101, 102, 103, 104, 105, 107, 110, 115, 118, 121, 122, 124 und 132, sowie konservativen Substitutionen davon.

6. Polynukleotidsequenz, kodierend einen Teil eines humanen monoklonalen Antikörpers mit einer Sequenz von Aminosäureresten aus der schweren Kette der variablen Region von Immunglobulin, wobei der Antikörper fähig ist mit humanem Immundefekt Virus (HIV) Glykoprotein gp120 eine Immunreaktion einzugehen und HIV zu neutralisieren, wobei der monoklonale Antikörper die Bindungsspezifität eines monoklonalen Antikörpers besitzt, der die genannte Sequenz aus Aminosäurenresten der variablen Region der schweren Kette von Immunglobulin umfasst, ausgewählt aus der Gruppe bestehend aus SEQ ID NO's: 66, 67, 68, 70, 72, 73, 74, 75, 78 und 19, sowie konservativen Substitutionen dieser Sequenz aus Aminosäurenresten, und dazu komplementäre Polynukleotidsequenzen.

7. Die Polynukleotidsequenz gemäß Anspruch 6, wobei das Polynukleotid DNA ist.

8. Polynukleotidsequenz, kodierend einen Teil eines humanen monoklonalen Antikörpers mit einer Sequenz von Aminosäureresten aus der leichten Kette der variablen Region von Immunglobulin, wobei der Antikörperfähig ist mit humanem Immundefekt Virus (HIV) Glykoprotein gp120 eine Immunreaktion einzugehen und HIV zu neutralisieren, wobei der monoklonale Antikörper die Bindungsspezifität eines monoklonalen Antikörpers besitzt, der die genannte Sequenz aus Aminosäurenresten der variablen Region der leichten Kette von Immunglobulin umfasst, ausgewählt aus der Gruppe bestehend aus SEQ ID NO's: 95, 96, 97, 98, 101, 102, 103, 104, 105, 107, 110, 115, 118, 121, 122, 124 und 132, sowie konservativen Substitutionen dieser Sequenz aus Aminosäurenresten, und dazu komplementäre Polynukleotidsequenzen.

9. Wirtszelle, umfassend die Polynukleotidsequenz gemäß Anspruch 6 oder Anspruch 8.

10. DNA Expressionsvektor, umfassend die Polynukleotidsequenz gemäß Anspruch 6 oder Anspruch 8.

11. Verfahren zur Detektion von humanem lmmundefekt Virus (HIV) *in vitro,* umfassend das Kontaktieren einer Probe, die vermutlich HIV enthält, mit einer diagnostisch effektiven Menge des monoklonalen Antikörpers gemäß Anspruch 1 oder Anspruch 5, und Bestimmung, ob der monoklonale Antikörper mit der Probe eine Immunreaktion eingeht.

12. Das Verfahren gemäß Anspruch 11, wobei der monoklonale Antikörper detektierbar mit einem Marker markiert wird, ausgewählt aus der Gruppe bestehend aus einem Radioisotop, einer fluoreszierenden Verbindung, einem kolloidalen Metall, einer chemiluminiszierenden Verbindung, einer bioluminiszierenden Verbindung, und einem Enzym.

13. Das Verfahren gemäß Anspruch 11, wobei der monoklonale Antikörper an eine feste Phase gebunden ist.

14. Verwendung eines monokolonalen Antikörpers gemäß Anspruch 1 oder Anspruch 5 zur Herstellung eines Medikamentes für die Bereitstellung einer passiven Immuntherapie gegen Humane Immundefekt Virus (HIV) Erkrankung im Menschen.

15. Die Verwendung gemäß Anspruch 14, wobei die passive Immuntherapie vorbeugend bereitgestellt wird.

16. Die Verwendung gemäß Anspruch 14, wobei die genannte passive Immuntherapie die parenterale Verabreichung des genannten Antikörpers umfasst.

17. Die Verwendung gemäß Anspruch 16, wobei die parenterale Verabreichung durch subkutane, intramuskuläre, intraperitoneale, intrakavitäre, transdermale, oder intravenöse Injektion erfolgt.

18. Die Verwendung gemäß Anspruch 16, wobei die parenterale Verabreichung durch graduelle Perfusion erfolgt.

19. Die Verwendung gemäß Anspruch 18, wobei die graduelle Perfusion auf intravenösem oder peristaltischem Weg erfolgt.

20. Die Verwendung gemäß Anspruch 16, wobei genannte Immuntherapie parenterale Verabreichung von etwa 0.1 mg/kg bis etwa 300 mg/kg umfasst.

21. Verwendung eines anti-idiotypischen Antikörpers gegen den monoklonalen Antikörper gemäß Anspruch 1 oder Anspruch 5, zur Herstellung eines Medikamentes zur Induktion aktiver Immuntherapie gegen humane Immundefekt Virus (HIV) Erkrankung im Menschen.

22. Pharmazeutische Zusammensetzung, umfassend mindestens eine Dosis einer immuntherapeutisch effektiven Menge des monoklonalen Antikörpers gemäß Anspruch 1 oder Anspruch 5 in einem pharmakologischen Träger.

23. Die pharmazeutische Zusammensetzung gemäß Anspruch 22, wobei genannte Zusammensetzung zwei oder mehrere unterschiedliche monklonale Antikörper enthält.

24. Kit, verwendbar zur Detektion von humanem Immundefekt Virus (HIV) in einer Probe, die vermutlich HIV enthält, wobei der Kit Trägermittel enthält, die so eingeteilt sind, dass in enger Begrenzung zueinander ein oder mehrere Container vorhanden sind, umfassend einen Container enthaltend den monoklonalen Antikörper gemäß Anspruch 1 oder 5.

## Revendications

1. Anticorps monoclonal humain capable d'immunoréagir avec la glycoprotéine gp120 du virus de l'immunodéficience humaine (VIH) et de neutraliser VIH, où l'anticorps monoclonal a la spécificité de liaison d'un anticorps monoclonal comprenant une séquence de résidus d'acides aminés de région variable d'immunoglobuline chaîne lourde sélectionnée dans le groupe consistant en SEQ ID Nos 66, 67, 68, 70, 72, 73, 74, 75, 78 et 79, et leurs substitutions conservatives.

2. Anticorps monoclonal humain de la revendication 1 où l'anticorps monoclonal a la spécificité de liaison d'un anticorps monoclonal ayant des séquences de résidus d'acides aminés des régions variables d'immunoglobuline chaîne lourde et légère par paires sélectionnées dans le groupe consistant en SEQ ID NOs 66:95, 67:96, 72:102, 66:97, 73:107, 74:103, 70:101, 68:98, 75:104, 72:105, 78:110, 66:118, 66:122, 66:121, 66:115, 19:124, 19:132 et 66:98, et leurs substitutions conservatives.

3. Anticorps monoclonal humain de la revendication 1, où l'anticorps monoclonal a la spécificité de liaison d'un anticorps monoclonal produit par ATCC 69078, ATCC 69079 ou ATCC 69080.

4. Anticorps monoclonal humain de la revendication 3 où l'anticorps monoclonal est l'anticorps monoclonal produit par ATCC 69078, ATCC 69079 ou ATCC 69080.

5. Anticorps monoclonal humain capable d'immunoréagir avec la glycoprotéine gp120 du virus de l'immunodéficience humaine (VIH) et de neutraliser VIH, où l'anticorps monoclonal a la spécificité de liaison d'un anticorps monoclonal comprenant une séquence de résidus d'acides aminés de région variable d'immunoglobuline chaîne légère sélectionnée dans le groupe consistant en SEQ ID Nos 95, 96, 97, 98, 101, 102, 103, 104, 105, 107, 110, 115, 118, 121, 122, 124 et 132, et leurs substitutions conservatives.

6. Séquence de polynucléotide codant pour une portion de séquence de résidus d'acides aminés de région variable d'immunoglobuline chaîne lourde d'un anticorps monoclonal humain capable d'immunoréagir avec la glycoprotéine gp120 du virus de l'immunodéficience humaine (VIH) et de neutraliser VIH, où l'anticorps monoclonal a la spécificité de liaison d'un anticorps monoclonal comprenant ladite séquence de résidus d'acides aminés de région variable d'immunoglobuline chaîne lourde sélectionnée dans le groupe consistant en SEQ ID NOs 66, 67, 68, 70, 72, 73, 74, 75, 78 et 79, et substitutions conservatives de la séquence de résidus d'acides aminés et séquences de polynucléotides complémentaires.

7. Séquence de polynucléotide de la revendication 6, où le polynucléotide est l'ADN.

8. Séquence de polynucléotide codant pour une portion de séquence de résidus d'acides aminés d'une région variable d'immunoglobuline chaîne légère d'un anticorps monoclonal humain capable d'immunoréagir avec la glycoprotéine gp120 du virus de l'immunodéficience humaine (VIH) et de neutraliser VIH, où l'anticorps monoclonal a la spécificité de liaison d'un anticorps monoclonal comprenant ladite séquence de résidus d'acides aminés de la région variable de l'immunoglobuline chaîne légère sélectionnée dans le groupe consistant en SEQ ID NOs 95, 96, 97, 98, 101, 102, 103, 104, 105, 107, 110, 115, 118, 121, 122, 124 et 132, et les substitutions conservatives de la séquence de résidus d'acides aminés et les séquences de polynucléotide complémentaires.

9. Cellule hôte comprenant la séquence de polynucléotide de la revendication 6 ou de la revendication 8.

10. Vecteur d'expression d'ADN comprenant la séquence de polynucléotide de la revendication 6 ou de la revendication 8.

11. Méthode de détection du virus de l'immunodéficience humaine (VIH) in vitro consistait à mettre en contact un échantillon suspecté de contenir VIH avec une quantité diagnostiquement efficace de l'anticorps monoclonal de la revendication 1 ou de la revendication 5 et à déterminer si l'anticorps monoclonal immunoréagit avec l'échantillon.

12. Méthode de la revendication 11, où l'anticorps monoclonal est marqué de façon détectable par un marqueur sélectionné dans le groupe consistant en un radioisotope, un composé fluorescent, un métal colloïdal, un composé chimioluminescent, un composé bioluminescent et une enzyme.

13. Méthode de la revendication 11, où l'anticorps monoclonal est lié à une phase solide.

14. Utilisation d'un anticorps monoclonal selon la revendication 1 ou la revendication 5 dans la fabrication d'un médicament pour appliquer une immunothérapie passive à une maladie du virus de l'immunodéficience humaine (VIH) chez un humain.

15. Utilisation selon la revendication 14, où l'immunothérapie passive est appliquée prophylactiquement.

16. Utilisation selon la revendication 15, où ladite immunothérapie comprend l'administration dudit anticorps monoclonal par voie parentérale.

17. Utilisation selon la revendication 16, où l'administration parentérale est par injection sous cutanée, intramusculaire, intrapéritonéale, intra-cavité, transcutanée ou intraveineuse.

18. Utilisation selon la revendication 16, où l'administration parentérale est par perfusion graduelle.

19. Utilisation selon la revendication 18, où la perfusion graduelle est par un moyen intraveineux ou péristaltique.

20. Utilisation selon la revendication 16, où ladite immunothérapie comprend l'administration parentérale d'environ 0,1 mg/kg à environ 300 mg/kg.

21. Utilisation d'un anticorps anti-idiotype à l'anticorps monoclonal de la revendication 1 ou de la revendication 5 dans la fabrication d'un médicament pour induire une immunothérapie active à une maladie du virus de l'immunodéficience humaine (VIH) chez un humain.

22. Composition pharmaceutique comprenant au moins une dose d'une quantité immunothérapeutiquement efficace de l'anticorps monoclonal de la revendication 1 ou de la revendication 5 dans un support pharmacologique.

23. Composition pharmaceutique de la revendication 22 où ladite composition contient deux anticorps monoclonaux différents ou plus.

24. Kit utile pour la détection du virus de l'immunodéfience humaine (VIH) dans une source suspectée de contenir VIH, le kit comprenant un moyen de support qui est compartimenté pour recevoir en proche confinement un ou plusieurs conteneurs comprenant un conteneur contenant l'anticorps monoclonal de la revendication 1 ou de la revendication 5.
